# EUROPEAN PATENT APPLICATION

(11) **EP 4 310 195 A1**
(43) Date of publication of application: **24.01.2024**
(21) Application number: 22382684.3
(22) Date of filing: 18.07.2022
(51) Int. Cl.: C12Q 1/6825, C12Q 1/6811

(54) **METHOD FOR NUCLEIC ACID DETECTION USING SIGNAL-MEDIATED AMPLIFICATION OF RNA TECHNOLOGY AND RNA APTAMERS**

(71) Applicant: Moirai Biodesign, S.L., 08028 Barcelona (ES)
(72) Inventor: DOTU RODRÍGUEZ, Ivan Javier, 08028 Barcelona (ES); MINUESA DINARÈS, Gerard, 08028 Barcelona (ES); ALSINA VERDÚ, Cristina, 08028 Barcelona (ES)
(74) Representative: Hoffmann Eitle

(57) **Abstract**

The present invention relates to a method for detecting a target nucleic acid in a sample using an optimized SMART method that comprises the use of at least one fluorogenic aptamer for the direct detection of the RNA transcripts. Preferably, the method is used after an amplification step, for example after a NASBA assay.

## Description

### TECHNICAL FIELD

The present invention relates to the field of biotechnology. Particularly, the present invention relates to the field of nucleic acid amplification and detection of target nucleic acids in a sample.

### BACKGROUND ART

Signal-mediated amplification of RNA technology (SMART) is an isothermal nucleic acid amplification assay that relies on signal amplification, being rapid and simple to perform without requiring thermal cycling steps. The assay has further advantages in that it can be used for either DNA or RNA targets. Further, the signal generated is highly target-dependent (i.e., a signal is only produced when a specific target is present), and discrimination of base changes within the target sequence is possible.

The SMART assay consists of two single-stranded oligonucleotide probes (extension and template); each probe includes one region (called herein foot region) that can hybridize to the target and another region (called herein arm region) that hybridizes to the other probe. The two probes are designed so that they can only anneal to each other in the presence of the specific target, forming a structure called a three-way junction.

Following three-way junction formation, a DNA polymerase extends the short (extension) arm of the probe and transforms a single-stranded promoter sequence into a functional double-stranded RNA polymerase promoter. The assay relies on the fact that only when the double-stranded RNA polymerase promoter is functional, the RNA polymerase can generate multiple copies of an RNA, which are the molecules that will subsequently be detected.

Several detection means have been described to sense and quantify the amplified RNA. Among them, enzyme-linked oligosorbent assay (ELOSA) is based on the detection by measuring color change in a standard plate reader where the amplified RNA molecules are attached and hybridized to a further probe comprising detection means, such as, an alkaline phosphatase (AP)-linked probe. Other detection means rely in the use of molecular beacons, which are oligonucleotide molecules that can report the presence of the amplified RNA, due to their hairpin-shaped structure with an internally quenched fluorophore, whose fluorescence is restored when they bind to their target.

It is important to note that the SMART process differs from other amplification and detection methods in that it is based on the amplification of a detection signal, not the amplification of the target nucleic acid. That is, the molecule that is detected is an amplified RNA that is only produced when the target nucleic acid has bound to the two probes forming the three-way junction.

SMART assay is currently being developed and used for the detection of specific nucleic acid sequences from clinical samples. However, it also has some drawbacks that leave room for improvement. One of them relates to the fact that, when the RNA molecules are generated, the method requires the addition of a probe that provides the means for detecting said RNA molecules. In the case of the ELOSA, said probe is an alkaline phosphatase (AP)-linked probe. If beacon molecules are used, they need to be added to the reaction so they can hybridize to the RNA molecules in order for them to be detected. As it usually occurs in amplification methods, the addition of further steps usually comes with the sacrifice of specificity and efficiency. Additionally, SMART assays including several steps hampers the implementation of said methods in a point of care (POC) setting, which is crucial when analyzing clinical samples in a time- and cost-effective and efficient way.

The present invention aims at solving the above problems and describes an optimized SMART method, called herein Fluorescent-SMART method, that provides detection means without the need of adding a further probe to detect the amplified RNA, simplifying the SMART method and increasing its sensibility.

### SUMMARY OF INVENTION

In a **first aspect,** the present invention relates to a method for detecting the presence of a target nucleic acid in a sample, the method comprising the steps of:
a) adding a first and a second nucleic acid probes to a sample comprising the target nucleic acid so as to form a three-way junction structure by hybridization between the target nucleic acid molecule, the first and the second nucleic acid probe, wherein:
   i) the first probe comprises a foot region located at the 5' region of the probe which is complementary to a first portion of the target nucleic acid and hybridizes thereto, and an arm region located at the 3' region of the probe;
   ii) the second probe comprises:
      (1) a foot region located at the 3' region of the probe which is complementary and hybridizes thereto to a second portion of the target nucleic acid, and
      (2) an arm region located at the 5' region of the probe, comprising, in the 5' to 3' direction:
         - a full length reverse complement sequence of at least one fluorogenic aptamer,
         - a full length reverse complement sequence of an DNA-directed RNA polymerase promoter, wherein the reverse complement sequence of the at least one fluorogenic aptamer is operably linked to said DNA-directed RNA polymerase promoter,
         - a region of between 5-9 nucleotides that is complementary and hybridizes thereto to the arm region of the first probe, optionally wherein said region of between 5-9 nucleotides is fully or partially included in the full length reverse complement sequence of the DNA-directed RNA polymerase promoter, and
   iii) the target nucleic acid comprises a first portion located at the 3' region of the target nucleic acid which is complementary to the foot region of the first probe, and a second portion located at the 5' region of the target nucleic acid which is complementary to the foot region of the second probe, wherein the first portion and the second portion are adjacent or substantially adjacent, preferably wherein the first portion and the second portions are separated by 0 to 6 nucleotides, and wherein the three-way junction structure is formed between the first probe, the second probe and the target nucleic acid when the target nucleic acid is present in the sample, and
b) adding a DNA-directed DNA polymerase which extends the arm of the first probe until the end of the arm of the second probe, creating a double-stranded structure comprising a functional RNA polymerase promoter and at least one fluorogenic aptamer, wherein one strand of the double-stranded structure is provided by the extended arm of the first probe, and the other strand of the double-stranded structure is provided by the arm of second probe;
c) adding a DNA-directed RNA polymerase which recognises the double-stranded promoter formed in step b), so as to cause the *de novo* synthesis of a single-stranded nucleic acid comprising the at least one fluorogenic aptamer; and
d) adding at least one fluorophore ligand of said at least one fluorogenic aptamer thereby directly detecting the *de novo* synthezised nucleic acid comprising the at least one fluorogenic aptamer, wherein the detection of said nucleic acid comprising the at least one fluorogenic aptamer indicates the presence of the target nucleic acid in the sample,
wherein, optionally, the method further comprises an amplification step previous to step a), wherein the target nucleic acid is amplified.

Preferably, the double-stranded RNA promoter formed in step b) is a T7 RNA polymerase promoter, and wherein the DNA-directed RNA polymerase added in step c) is a T7 RNA polymerase.

Preferably, the DNA-directed DNA polymerase added in step b) is the *Bacillus stearothermophilus* DNA Polymerase I.

Preferably, the foot region of the first probe and/or of the second probe is at least 15 nucleotides in length.

Preferably, the at least one fluorescent aptamer is a Mango aptamer or a Broccoli aptamer.

Preferably, the fluorophore ligand added in step d) is TO1 biotin fluorogen if the at least one fluorescent aptamer comprised in the arm of the second probe is a Mango aptamer or wherein the fluorophore ligand added in step d) is DFHBI or DFHBI-1T fluorogen if the at least one fluorogenic aptamer comprised in the arm of the second probe is a Broccoli aptamer.

Preferably, the method further comprises an amplification step previous to step a) comprising amplifying the target nucleic acid to provide a plurality of molecules identical to the target nucleic acid or the reverse complement thereof, wherein said plurality of molecules represent the target nucleic acid in the subsequent steps a) to d), and wherein said amplification step is optionally followed by a step of degrading residual amplification primers and dephosphorylating excess dNTPs after amplification.

Preferably, the method comprises an amplification step, wherein said amplification step is carried out by nucleic acid sequence-based amplification (NASBA), wherein said amplification step is followed by a step of degrading residual amplification primers and dephosphorylating excess dNTPs after amplification. Preferably, the step of degrading residual amplification primers and dephosphorylating excess dNTPs after amplification step is carried out by an exonuclease digestion followed by a phosphatase reaction.

Preferably, the target nucleic acid is an RNA molecule, preferably derived from the genome of an infectious agent such as a bacterium or a virus. More preferably, the target nucleic acid is a bacterial 16S or 23S rRNA. Also more preferably, the target nucleic acid is viral genomic RNA, preferably selected from SARS-CoV-2 virus or Influenza virus genomes.

Another aspect of the present invention relates to a **computer-implemented method** for designing at least a pair of probes suitable for implementing the method as defined in the first aspect, the method comprising the steps of:
i) reading a target nucleic acid,
ii) obtaining or generating the sequence of at least a pair of probes, wherein each of the probes comprise a foot region and an arm region, wherein:
   the foot region of the first probe is located at the 5' region of said probe and is complementary to a first portion of the target nucleic acid and hybridizes thereto, and the arm region of the first probe is located at the 3' region of said probe and is non-complementary to the target nucleic acid, wherein
   the foot region of the second probe is located at the 3' region of said probe and is complementary and hybridizes thereto to a second portion of the target nucleic acid, and the arm region of the second probe is located at the 5' region of said probe and comprises, in the 5' to 3' direction:
      - a full length reverse complement sequence of at least one fluorogenic aptamer,
      - a full length reverse complement sequence of an DNA-directed RNA polymerase promoter, wherein the reverse complement sequence of the at least one fluorogenic aptamer is operably linked to said DNA-directed RNA polymerase promoter,
      - a region of between 5-9 nucleotides that is complementary and hybridizes thereto to the arm region of the first probe, optionally wherein said region of between 5-9 nucleotides is fully or partially included in the full length reverse complement sequence of the DNA-directed RNA polymerase promoter,
   wherein the first portion and the second portion of the target nucleic acid are separated by 0 to 6 nucleotides,
   wherein the first and the second probes are capable of forming a three-way junction structure by hybridization with the target nucleic acid molecule, and
iii) optionally, providing the sequences obtained or generated in step ii) as output.

Preferably, step ii) comprises the steps of:
a) obtaining the foot region of the first probe by selecting a first portion of nucleotides comprised in the target nucleic acid and providing its complementary sequence,
b) obtaining the foot region of the second probe by selecting a second portion of nucleotides comprised in the target nucleic acid and providing its complementary sequence, wherein the first portion and the second portion are separated by 0 to 6 nucleotides,
c) obtaining the arm region of the second probe by selecting a nucleotide sequence comprising the full length sequence of an DNA-directed RNA polymerase promoter and the full length reverse complement sequence of at least one fluorogenic aptamer, wherein the reverse complement sequence of at least one fluorogenic aptamer is operably linked to said DNA-directed RNA polymerase promoter,
d) obtaining the arm region of the first probe by selecting between 5-9 nucleotides that are complementary to a region located at the 3' region of the arm region of the second probe obtained in step c), and
e) optionally, synthesizing the designed first and a second probe.

Another aspect of the present invention relates to a **kit of parts** for detecting the presence of a target nucleic acid in a sample, comprising the following elements:
a) at least one DNA-directed DNA polymerase, preferably Bst polymerase,
b) at least one DNA-directed RNA polymerase, preferably T7 RNA polymerase,
c) at least a suitable fluorophore ligand of a fluorogenic aptamer,
d) spermidine,
e) deoxynucleoside triphosphates,
f) suitable buffers,
g) optionally, a retrotranscriptase, an RNAse, at least an exonuclease and a phosphatase,
wherein elements a) to g) are comprised in different containers or grouped in one or more containers.

Another aspect relates to a **method** for detecting the presence of a target nucleic acid in a sample as defined in the first aspect, wherein the method uses the kit of part as defined in the previous aspect.

Another aspect relates to a **system** for detecting the presence of a target nucleic acid in a sample comprising a first probe and a second probe, wherein the first probe comprises a foot region located at the 5' region of the probe which is complementary to a first portion of the target nucleic acid and hybridizes thereto, and an arm region located at the 3' region of the probe; and wherein the second probe comprises:
(1) a foot region located at the 3' region of the probe which is complementary and hybridizes thereto to a second portion of the target nucleic acid, and
(2) an arm region comprising, in the 5' to 3' direction:
   i. a full length reverse complement sequence of at least one fluorogenic aptamer,
   ii. a full length reverse complement sequence of an DNA-directed RNA polymerase promoter, wherein the reverse complement sequence of the at least one fluorogenic aptamer is operably linked to said RNA polymerase promoter,
   iii. a region comprising between 5-9 nucleotides that is complementary and hybridizes thereto to the arm region of the first probe, wherein said region between 5-9 nucleotides may be a region included in the full length reverse complement sequence of an DNA-directed RNA polymerase promoter.

### BRIEF DESCRIPTION OF DRAWINGS

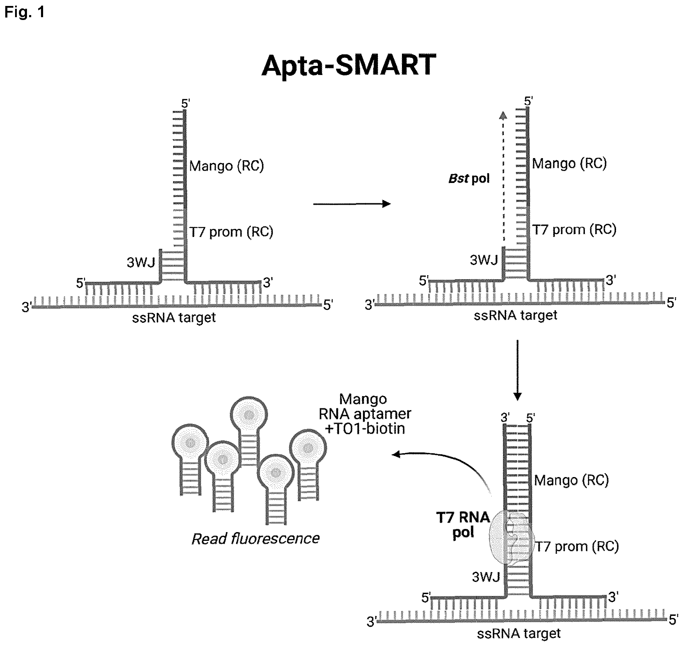

**Figure 1****. Schematic representation of Aptamer (apta)-SMART detection method.** Apta-SMART (Mango) detection (performed at fixed 41°C for 1 h) scheme showing the first (left side) and second DNA probes (right side) hybridization with a ssRNA target (in the example) or DNA. The second DNA probe contains the hybridization region with ssRNA (or DNA) and 3WJ forming region (hybridization with first probe), the T7 promoter region (in reverse complement, RC) and the Mango aptamer sequence (in RC). During SMART, the activity of *Bst* polymerase (*Bst* pol) extends the first probe from 3' to 5' and T7 RNA polymerase (T7 RNA pol) activity makes RNA copies of the Mango aptamer sequence (sense strand) that, in the presence of the fluorogen TO1-biotin, emits fluorescence at 535 nm after excitation at 510 nm wavelength, an indirect and proportional measurement of the presence of the ssRNA target.

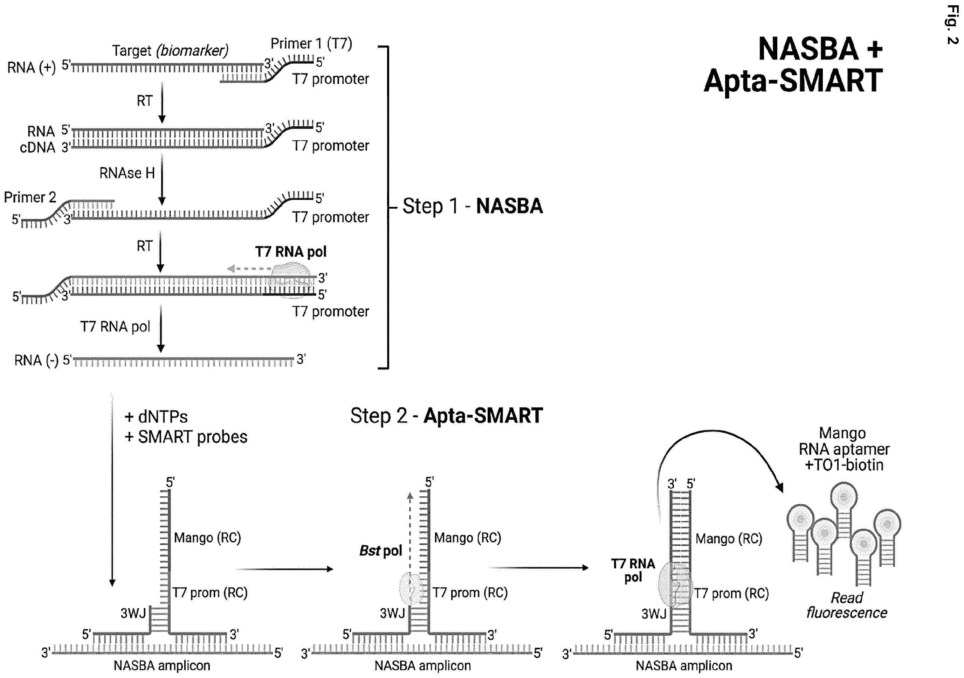

**Figure 2****. Schematic representation of NASBA isothermal amplification followed by apta-SMART protocol.** (*Step 1*) NASBA isothermal amplification (Kao *et al. Anal Letters,* 2010) is performed at 37°C during 1.5 h and starts with Primer 1 (containing a T7) annealing the 3' of a target (RNA(+)) and by retrotranscriptase (RT) activity is converted to a hybrid RNA/cDNA. This hybrid will be processed by RNAse H eliminating the RNA strand. Then Primer 2 anneals to the 3' end of the cDNA and again RT activity makes a copy of the cDNA to obtain a dsDNA with a T7 promoter. Then, T7 RNA polymerase makes a copy of RNA. The result of the isothermal amplification of the initial RNA(+) is its reverse complement (RC): RNA(-). This NASBA amplicon is the starting point for *Step 2.* (*Step 2*) Apta-SMART (Mango) detection (fixed at 41°C) starts by adding SMART probes, enzymes, NTPs and dNTPs (the latter being optional as remaining dNTPs from *Step 1* are enough for SMART to work optimally). The scheme shows the first (left side) and second DNA probes (right side) hybridization with a ssRNA target (in the example) or a DNA target. The second DNA probe contains the hybridization region with ssRNA (or DNA) and 3WJ forming region (hybridization between first and second probe), the T7 promoter region (in reverse complement, RC) and the Mango aptamer sequence (in RC). During SMART, the activity of *Bst* polymerase (*Bst* pol) extends the first probe from 3' to 5' and T7 RNA polymerase (T7 RNA pol) activity makes RNA copies of the Mango aptamer sequence (sense strand) that, in the presence of the fluorogen T01-biotin, emits fluorescence at 535 nm after excitation at 510 nm wavelength, an indirect and proportional measurement of the presence of the ssRNA target.

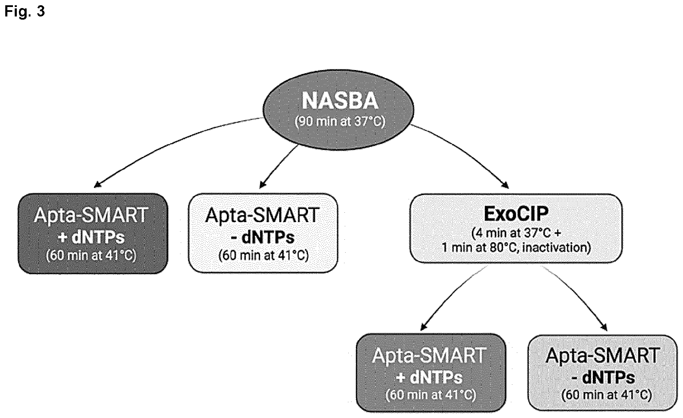

**Figure 3****. Diagram of NASBA, Exo-CIP treatment and Apta-SMART protocol combinations.** Apta-SMART reaction (1 h at 41°C) can be directly performed after NASBA isothermal amplification (1.5 h at 37°C) by adding dNTPs (+ dNTPs, far left *square*) to the mixture (besides SMART probes, enzymes and NTPs, always needed) or without adding them (- dNTPs, left *square*) (on the left side of diagram) or perform Exo-CIP treatment (right square) (to clean-up DNA primers and dephosphorylate remaining dNTPs) for 4 min at 37°C and 1 min at 80°C (to inactivate the enzymes) and, afterwards, proceed with Apta-SMART (41°C) either by adding (+dNTPs, bottom left square) or not dNTPs (-dNTPs, bottom right square).

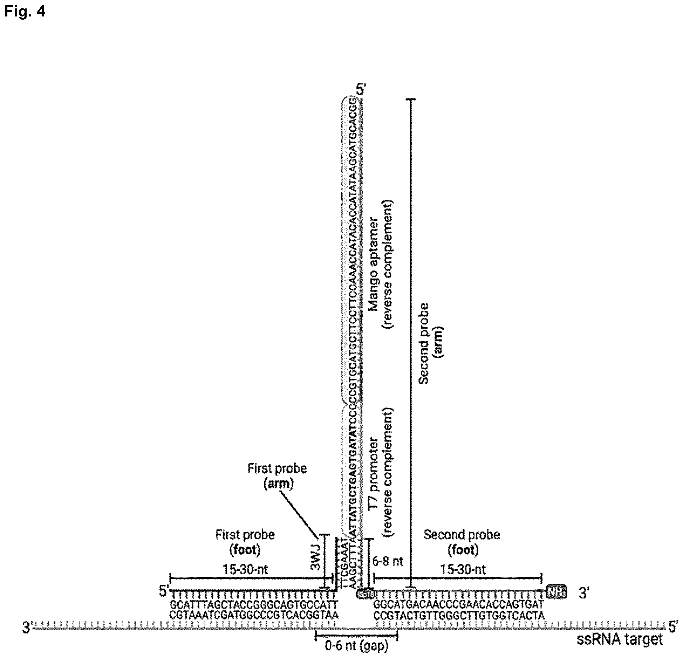

**Figure 4****. Scheme of SMART DNA probes with Mango aptamer detailing the sequences used for *E.coli* 23S rRNA detection.** The fragments of each probe (**seq id 1** and **2**) are detailed. These include the regions for ssRNA target binding (foot, in bold for the first probe **-SEQ ID NO: 1-** and for the second probe - **SEQ ID NO: 2**-) and hybridization to form the three-way junction structure (*3WJ* in the scheme), the reverse complement of the T7 promoter (sequence in bold) and the reverse complement of the Mango aptamer (*grey square*). Also, the 18-atom hexa-ethyleneglycol spacer (*iSp18*) is indicated in the second probe between the ssRNA target binding region and the 3WJ start as well as the terminal 3'-amino group (to prevent elongation on that 3' end) (NH₃, dark grey *box*). The lengths (in nucleotides, nt) of the space between the two annealing probes (0 - 6 nt -gap-) and of the 3WJ (6-8 nt) as well as the minimal length of first and second probes foot region (15-30-nt) are also indicated. The first and second probe regions of hybridization with the ssRNA target, 3WJ forming section and T7 promoter are identical to the ones reported in Wharam *et al. Nucl Acids Res,* 2001.

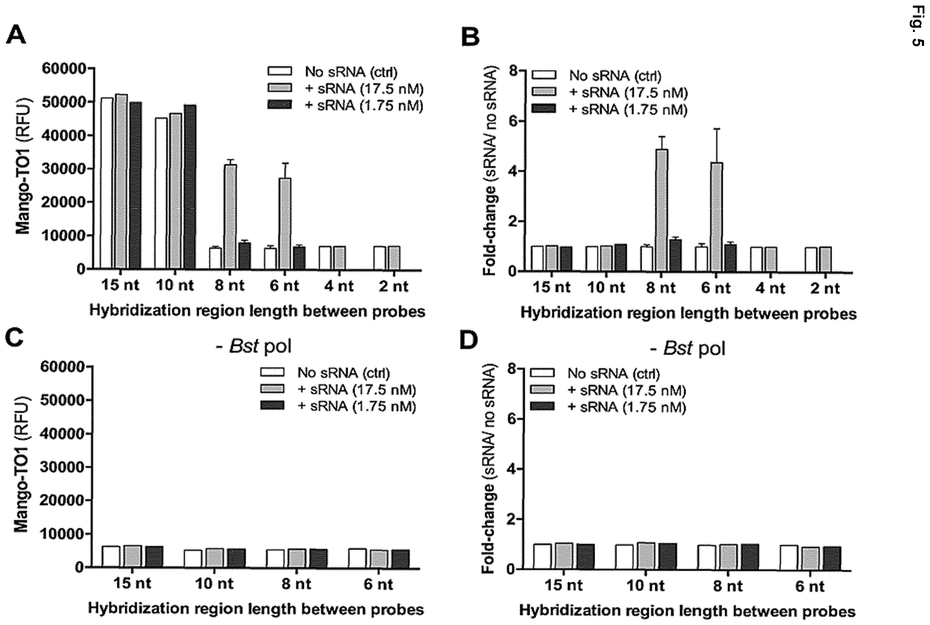

**Figure 5****. Apta-SMART (Mango aptamer) detection of *E.coli* 16S synthetic** RNA **(sRNA) at 1.75 and 17.5 nM using different lengths of the 3WJ hybridization region between probes.** Panel (**A**) shows the Raw Fluorescence Units (RFU) of Mango-T01 complexes after hybridization of *E.coli* 16S rRNA first probes with a hybridization between probes of 2-, 4-, 6-, 8-, 10- and 15-nt (**SEQ ID NO: 10, 11, 12, 7**, **14** and **15**) and a common second probe (**SEQ ID NO: 8**) to sRNA target (**SEQ ID NO: 13**). Panel (**B**) shows the fold-change of sRNA *versus* no sRNA control of the sample samples tested. The results of panel **A** and **B** represent the mean of fluorescence measurements of 3 independent experiments (for 8- and 6-nt) ± standard deviation of the mean or the results of a single experiment (for 15-, 10-, 4- and 2-nt, this latter only using 17.5 nM sRNA concentration). Panel (**C**) shows the Raw Fluorescence Units (RFU) of Mango-T01 complexes after hybridization of *E.coli* 16S rRNA first probes with a 3WJ of 6-, 8-, 10- and 15-nt (**SEQ ID NO: 12, 7, 14** and **15**) and a common second probe (**SEQ ID NO: 8**) to sRNA target (**SEQ ID NO: 13**) without using *Bst* polymerase in the SMART reaction (only adding T7 RNA polymerase). Panel (**D**) shows the fold-change of sRNA *versus* no RNA control of the same samples as in panel (**C**). The results of panel **C** and **D** represent the fluorescence measurements of a single experiment.

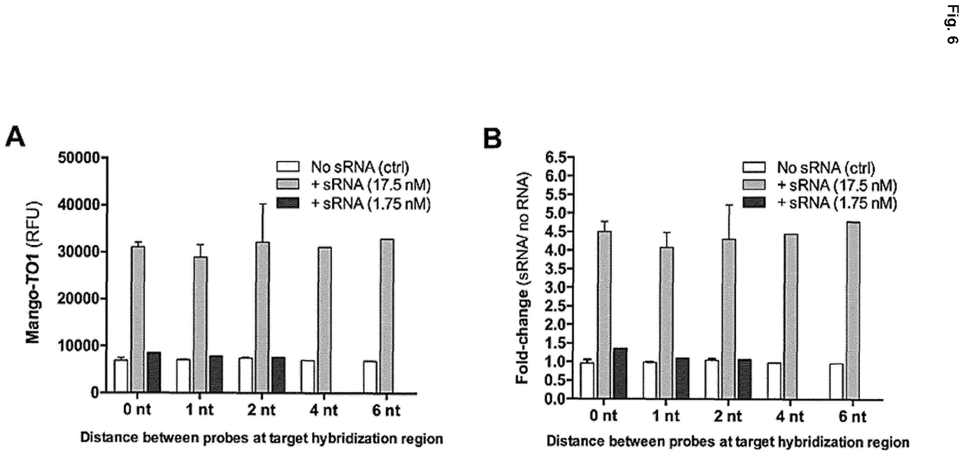

**Figure 6****. Apta-SMART (Mango aptamer) detection of synthetic RNA (sRNA) at 1.75 and 17.5 nM using different distances between *E.coli* 16S rRNA probes and the RNA target.** Panel (**A**) shows the Raw Fluorescence Units (RFU) of Mango-T01 complexes after hybridization of *E.coli* 16S rRNA first probes distancing 0, 1, 2, 4 and 6-nt (**SEQ ID NO: 7, 16, 17, 18** and **19**) one from each other once bound to the sRNA target (**SEQ ID NO: 13**), using a common second probe (**SEQ ID NO: 8**). Panel (**B**) shows the fold-change of sRNA *versus* no RNA control of the sample samples tested. The results represent the mean ± standard deviation of the mean of 2 independent experiments (for 0, 1 and 2-nt) or the results of a single experiment (for 4- and 6-nt).

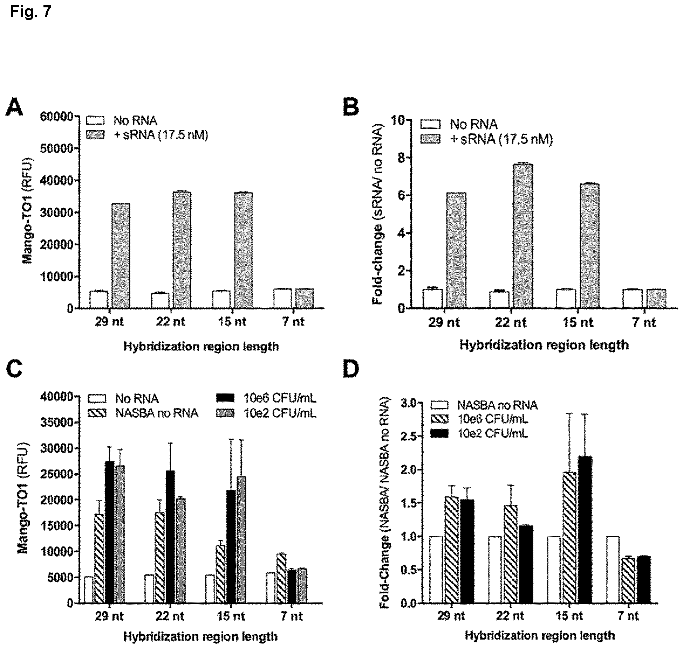

**Figure 7****. Apta-SMART (Mango aptamer) detection of *E.coli* 16S synthetic RNA (sRNA) at 17.5 nM using different lengths of hybridization region between probes and target.** Panel (**A**) shows the Raw Fluorescence Units of Mango-T01 (510 nm - exc- and 535 nm -emi- wavelengths) complexes after SMART reaction of *E.coli* 16S first probes with a 29-, 22-, 15- or 7-nt hybridization region (**SEQ ID NO: 7, 20, 21** and **22**) and same length hybridization region for second probe (**SEQ ID NO: 8, 23, 24** and **25**) in the presence (+sRNA) and absence (no RNA) of *E.coli* 16S synthetic RNA (75-nt, **SEQ ID NO: 13**) at 17.5 nM. Panel (**B**) shows the fold-change of sRNA *versus* no RNA control of the same samples tested. Panel (**C**) shows the Raw Fluorescence Units of Mango-T01 complexes after SMART reaction of *E.coli* 16S first probes with a 29-, 22-, 15- or 7-nt hybridization region (**SEQ ID NO: 7, 20, 21** and **22**) and same length hybridization region for second probe (**SEQ ID NO: 8, 23, 24** and **25**) in the presence of NASBA product from an *E.coli* culture of 10⁶ CFU/mL (10e6 CFU/mL) or 10² CFU/mL (10e2 CFU/mL). Panel (**D**) shows the fold-change of the NASBA product *versus* NASBA no RNA (control) of the samples tested. The results for all panels represent the mean of fluorescence measurements of an independent experiment ± standard error of the mean of data points per duplicate.

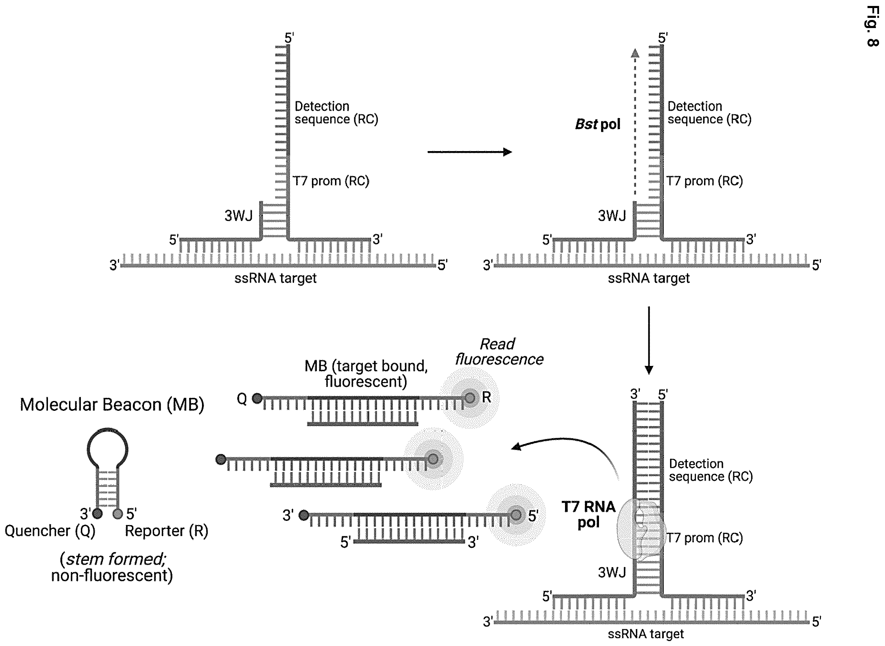

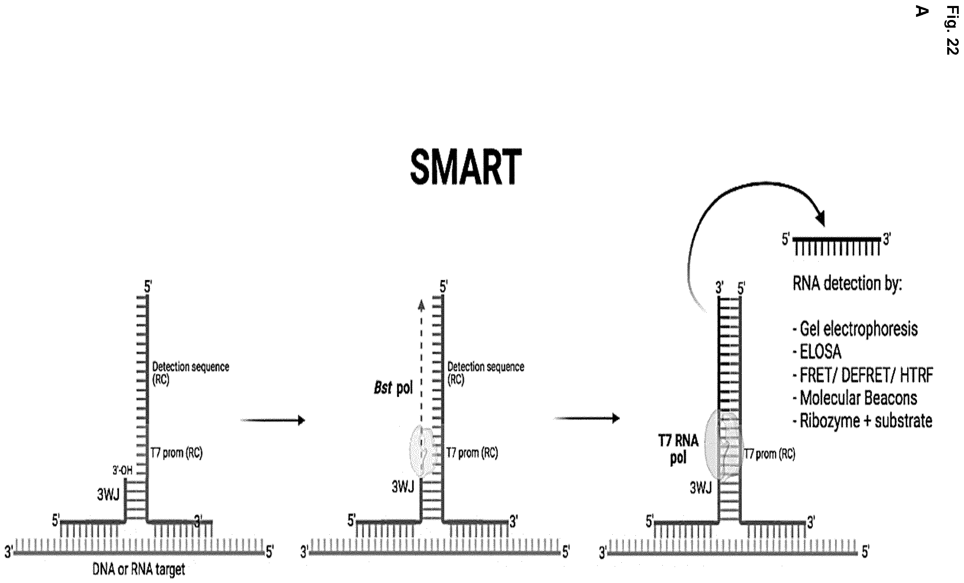

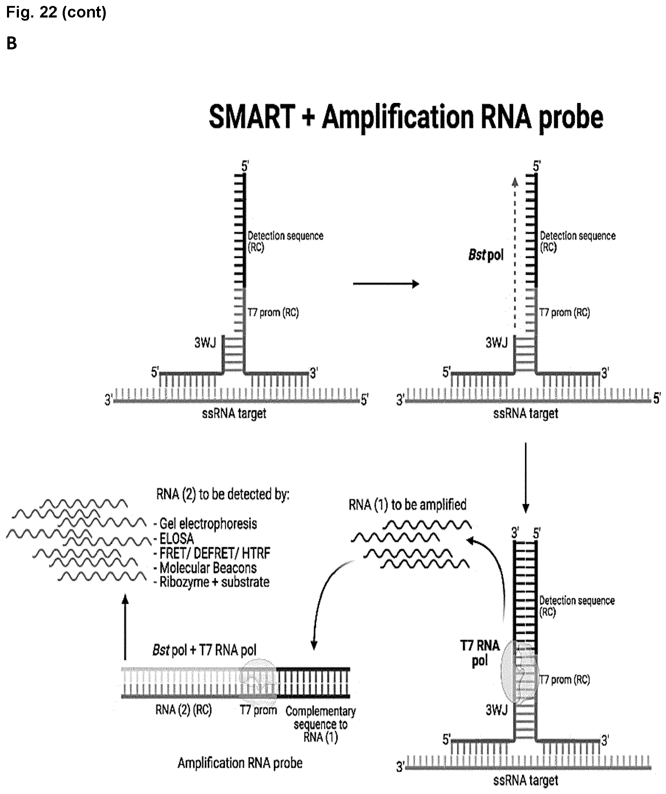

**Figure 8****. SMART reaction and detection by Molecular Beacon.** SMART reaction is performed at 41°C during 1 h as previously described (please refer to Figure 22) and here the detection is done by a Molecular Beacon (MB) interaction with the RNA detection sequence (in reverse complement, RC). Once bound, the hairpin shaped MB (containing a quencher -Q- and a reporter -R- fluorophore at 3' and 5' sides, respectively, that do not emit fluorescence while stem is formed), changes conformation, opening up to bind the detection sequence, quencher and reporter separate apart and the reporter emits fluorescence (different wavelengths depending on the fluorophore used). In the *Example 4,* a 5'-6-FAM is used as the reporter (R) and a 3'-Dabcyl is used as the quencher (Q) and the fluorescence signal from MB/target hybrids was read at 495 nm (excitation -exc-) and 520 nm (emission -emi-).

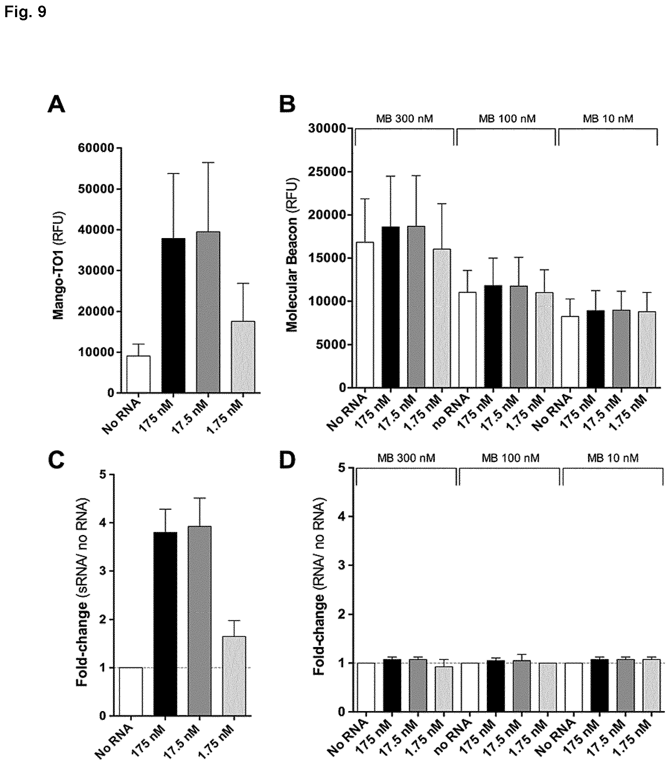

**Figure 9****. Apta-SMART (Mango aptamer) and Molecular Beacon (MB) detection of SARS-CoV-2 using a synthetic RNA (sRNA) fragment of S *'spike'* ssRNA at increasing concentrations** (**1.75, 17.5 and 175 nM**)**.** Panel (**A**) shows Raw Fluorescence Units (RFU) of Mango-T01 using the first and second probes (**SEQ ID NO: 26** and **27**) of SARS-CoV-2 sRNA (**SEQ ID NO: 28**)**.** Panel (**B**) shows the RFUs of MB detection using the first probe (**SEQ ID NO: 26**), a modified second probe (**SEQ ID NO: 30**) and the MB (**SEQ ID NO: 31**) by adding SARS-CoV-2 sRNA (**SEQ ID NO: 28**)**.** Panel (**C**) shows the fold-change of sRNA over no RNA control for Mango-T01 and panel (**D**) the calculated fold-change for MB detection. In panels (**B**) and (**D**) MB was tested at 300, 100 and 10 nM concentrations. Mango aptamer fluorescence (510 nm - exc- and 535 nm -emi-) was measured in the presence of 480 nM T01-biotin fluorogen substrate. Fluorescence signal from the hybridized MB/target was read at 495 nm (exc) and 520 nm (emi). The results represent the mean ± standard deviation of 4 independent experiments.

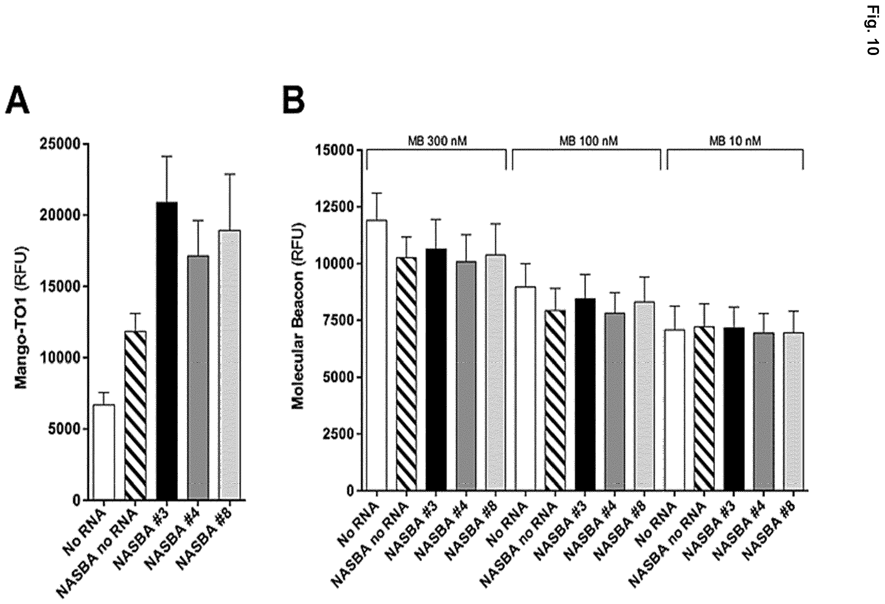

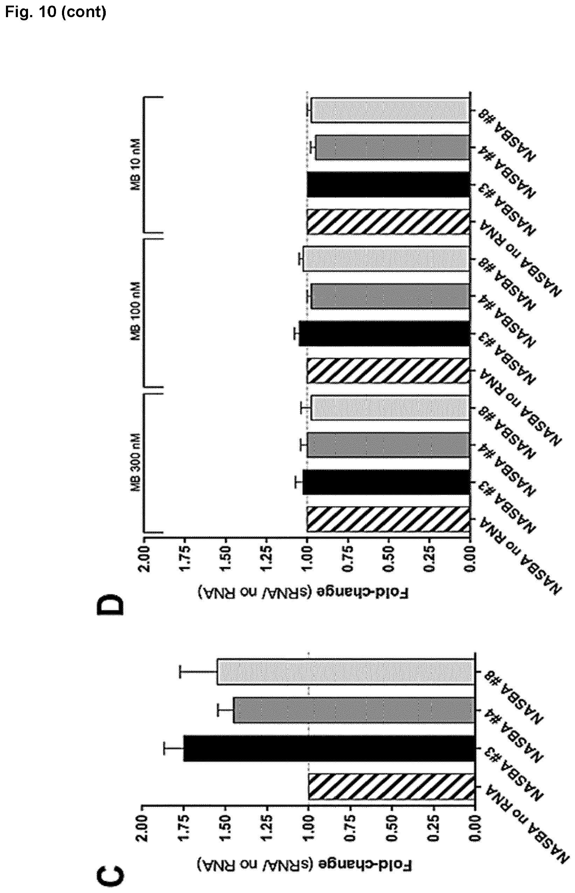

**Figure 10****. Apta-SMART (Mango aptamer) *versus* Molecular Beacon (MB) detection of SARS-CoV-2 after NASBA amplification of a fragment of S *'spike'* RNA region.** Panel (**A**) shows the Raw Fluorescence Units (RFU) from Mango-SMART detection of a SARS-CoV-2 S *'spike'* RNA region after NASBA amplification (**SEQ ID NO: 29**) using first and second probes (**SEQ ID NO: 26** and **27**) of three SARS-CoV-2 RNA samples (#3-black; #4, dark grey; and #8, light grey bars), as well as a NASBA no RNA control (black lined bar) and a no RNA control (white bar). Panel (**B**) shows the same samples' detection by a MB (**SEQ ID NO: 31**) (using first and second probes, **SEQ ID NO: 26** and **30,** respectively) tested at three different concentrations (300, 100 and 10 nM). Panels (**C**, **D**) represent the panel (**A**) and panel (**B**) data as fold-change of NASBA samples normalized over NASBA no RNA control. The content of the three SARS-CoV-2 RNA samples were previously analyzed by standard qPCR method and positivity confirmed with Ct amplification values of 13.23 (NASBA #3), 20.80 (NASBA#4) and 22.66 (NASBA#8). Mango aptamer fluorescence (510 nm -exc- and 535 nm -emi-) was measured in the presence of 480 nM T01-biotin fluorogen substrate. Fluorescence signal from the hybridized MB/target was read at 495 nm (exc) and 520 nm (emi). The results represent the mean ± standard deviation of 4 independent experiments. No RNA control is omitted in the fold-change panels as it was only used as background RFU value.

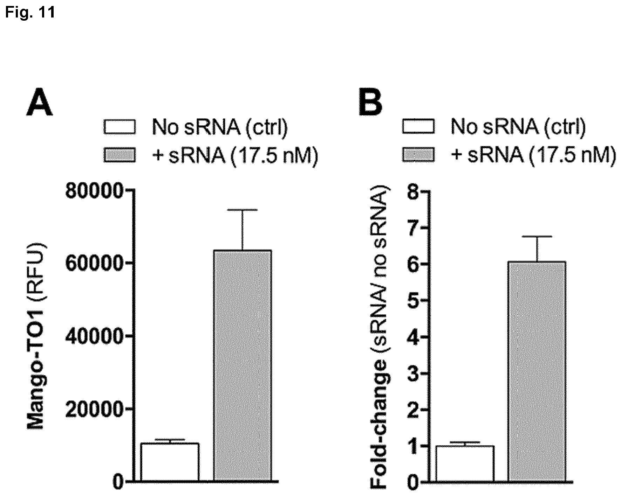

**Figure 11****. Apta-SMART detection of *E.coli* 16S rRNA by SMART probes and a synthetic RNA (sRNA) fragment of 75-nt.** The aptamer and substrate used are the Mango and T01-biotin. Panel (**A**) shows the Raw Fluorescence Units (RFU) from Mango-TO1 detection (510 nm -exc- and 535 nm -emi- wavelengths) using first and second probes (**SEQ ID NO: 7** and **8**) using the *E.coli* 16S sRNA (**SEQ ID NO: 13**); panel (**B**) shows the fold-change of the fluorescence detection in the presence of sRNA *versus* no RNA present (ctrl = control). Data represents the mean ± the standard deviation of fluorescence reads at 1 h after SMART detection of 9 independent experiments.

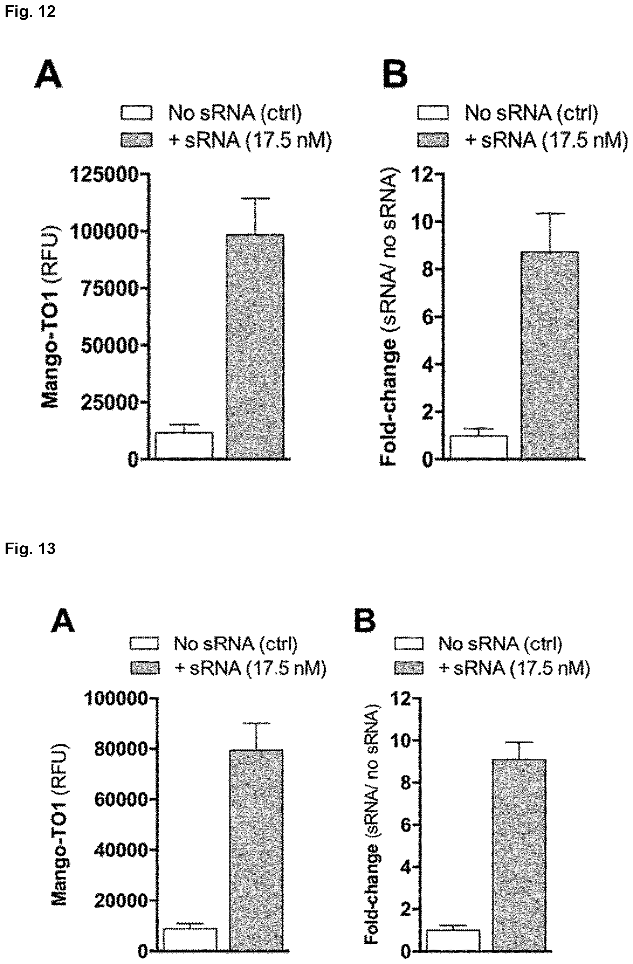

**Figure 12****. Apta-SMART detection of *E.coli* 23S rRNA by 23S SMART probes and a synthetic RNA (sRNA) fragment of 79-nt of *E.coli* 23S rRNA.** Panel (**A**) shows the Raw Fluorescence Units (RFU) from Mango-T01 detection (510 nm -exc- and 535 nm -emi- wavelengths) using first and second probes (**SEQ ID NO: 35** and **36**) and the sRNA (**SEQ ID NO: 39**). Panel (**B**) shows the fold-change of the fluorescence detection in the presence of sRNA *versus* no RNA present (ctrl = control). Data represents the mean ± the standard deviation of fluorescence reads at 1 h after SMART detection of 6 independent experiments.
**Figure 13****. Apta-SMART detection of *mecA S.aureus* by mecA SMART probes and a synthetic RNA fragment of 98-nt.** Panel (**A**) shows the Raw Fluorescence Units (RFU) from Mango-T01 detection (510 nm -exc- and 535 nm -emi- wavelengths) using first and second probes (**SEQ ID NO: 40** and **41**) and sRNA (**SEQ ID NO: 44**). Panel (**B**) shows the fold-change of the fluorescence detection in the presence of sRNA *versus* no RNA present (ctrl = control). Data represents the mean ± the standard deviation of fluorescence reads at 1 h after SMART detection of 6 independent experiments.

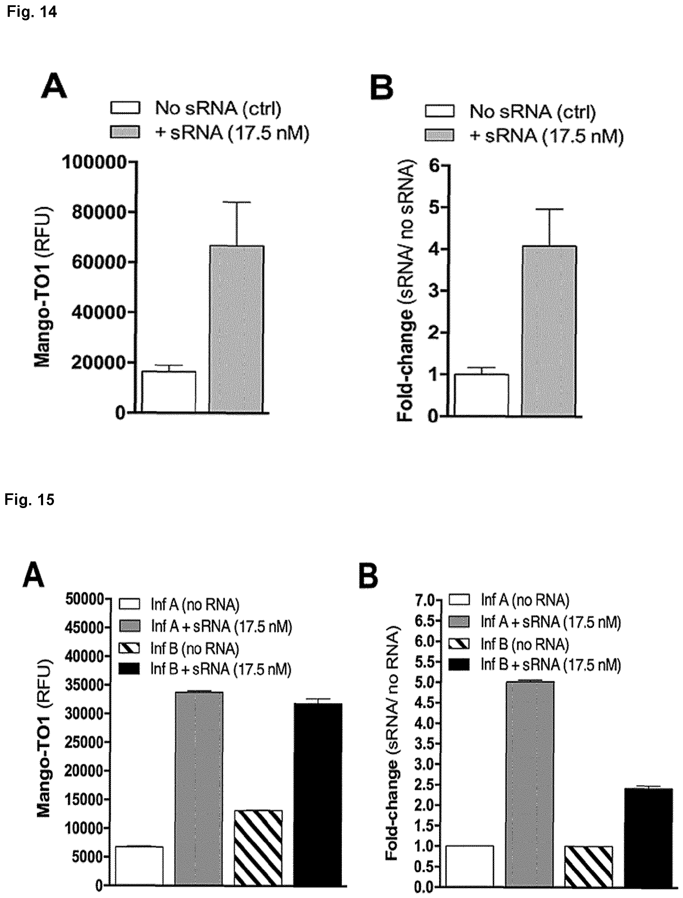

**Figure 14****. Apta-SMART detection of SARS-CoV-2 *'spike' S* RNA 30-nucleotide synthetic RNA (sRNA) region using SARS-CoV-2 probes.** Panel (**A**) shows the Raw Fluorescence Units (RFU) from Mango-T01 detection (510 nm -exc- and 535 nm -emi-wavelengths) using first and second probes (**SEQ ID NO: 26** and **27**) and sRNA (**SEQ ID NO: 28**)**.** Panel (**B**) shows the fold-change of the fluorescence detection in the presence of sRNA *versus* no RNA present (ctrl = control). Data represents the mean ± the standard deviation of fluorescence reads at 1 h after SMART detection of 4 independent experiments.
**Figure 15****. Mango-SMART detection of Influenza A and B synthetic RNA (sRNA) regions using Influenza A and B specific probes.** Panel (**A**) shows the Raw Fluorescence Units (RFU) from Mango-T01 detection (510 nm -exc- and 535 nm -emi-wavelengths) using specific first and second probes for Influenza A (**SEQ ID NO: 45** and **47**) and B (**SEQ ID NO: 46** and **48**) and Influenza A or B sRNAs (**SEQ ID NO: 49** and **50,** respectively) at 17.5 nM. Panel (**B**) shows the fold-change of the fluorescence detection of sRNA *versus* no RNA data points (ctrl = control). Data represents the mean ± the standard deviation of fluorescence reads at 1 h after SMART detection of an independent experiment performed in duplicate.

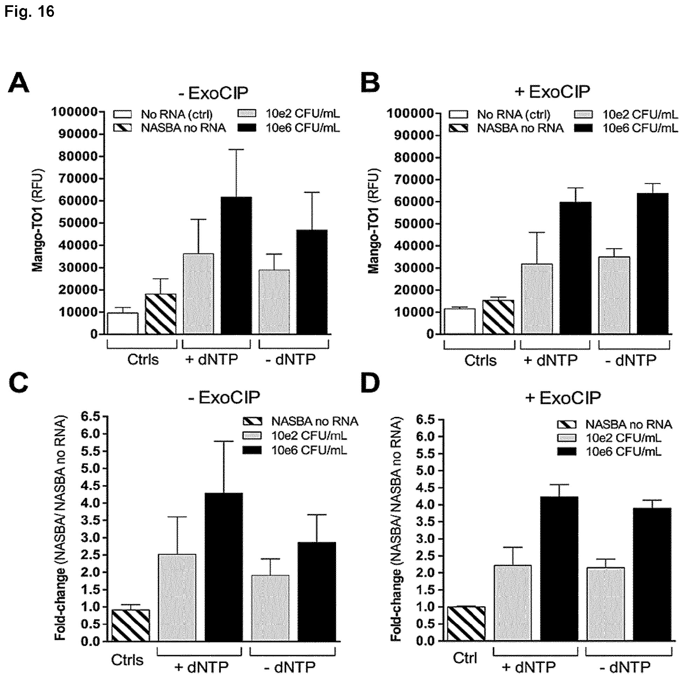

**Figure 16****. Apta-SMART detection by SMART probes of an *E.coli* 16S rRNA region after NASBA amplification.** Panel (**A**) shows the Raw Fluorescence Units (RFU) from Mango-TO1 detection (510 nm -exc- and 535 nm -emi- wavelengths) using first and second probes (**SEQ ID NO: 7** and **8**) of no RNA (ctrl = control), NASBA no RNA and NASBA product (**SEQ ID NO: 9**) from 10² and 10⁶ CFU *E.coli* inoculated in 1 mL of LB after adding (+ dNTP) and not adding (- dNTPs) in the SMART reaction mix. Panel (**B**) shows the RFU from Mango-T01 complex using first and second probes (**SEQ ID NO: 7** and **8**) of same samples after adding (+ dNTP) and not adding (- dNTPs) in the SMART reaction mix using ExoCIP treatment after NASBA (refer to scheme in **Figure 3**). Panel (**C**) shows the fold-change of the fluorescence detection for non-ExoCIP treated samples from panel A, normalizing the signal of 10² and 10⁶ CFU/mL NASBA reactions over NASBA no RNA control. Panel (**D**) shows the fold-change of the fluorescence detection (normalized over NASBA no RNA) of ExoCIP-treated samples from panel B. No RNA (ctrl) is omitted for normalization as it was only used as background RFU. Data represents the mean ± the standard deviation of fluorescence reads at 1 h after SMART detection of at least 3 independent experiments.

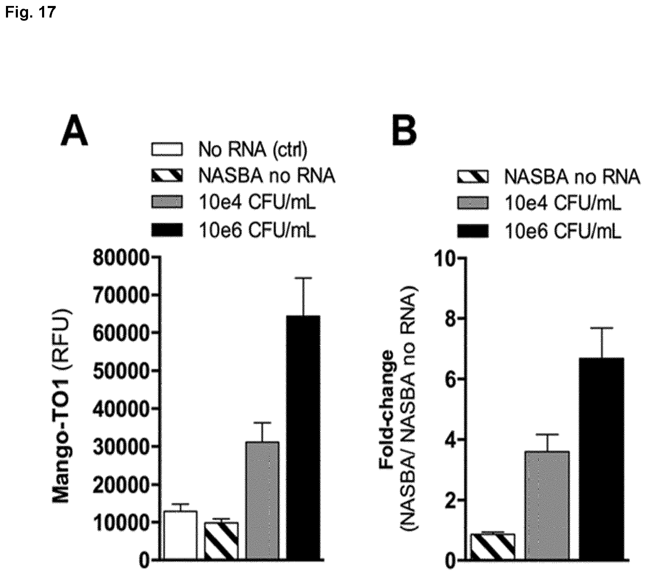

**Figure 17****. Apta-SMART detection by 23S SMART probes of an *E.coli* 23S rRNA region after NASBA amplification.** Panel (**A**) shows the Raw Fluorescence Units (RFU) from Mango-T01 detection (510 nm -exc- and 535 nm -emi- wavelengths) using first and second probes (**SEQ ID NO: 35** and **36**) of no RNA ctrl (control), NASBA no RNA and 10⁴ and 10⁶ CFU *E.coli* inoculated in 1 mL of LB. Panel (**B**) shows the fold-change of the fluorescence detection (normalizing the signal of 10⁴ and 10⁶ NASBA amplicon over NASBA no RNA control). No RNA (ctrl) is not used for normalization (only used as background RFU). Data represents the mean ± the standard deviation of fluorescence reads at 1 h after SMART detection of at least 3 independent experiments.

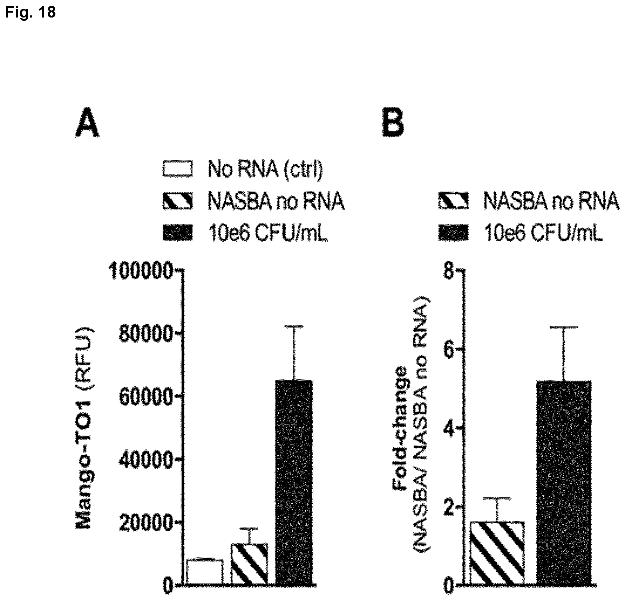

**Figure 18****. Apta-SMART detection of a reverse complement RNA region (NASBA product) of *mecA S.aureus* by mecA SMART probes.** Panel (**A**) panel shows the Raw Fluorescence Units (RFU) from Mango-T01 detection (510 nm -exc- and 535 nm -emi- wavelengths) using first and second probes **(SEQ ID NO: 40** and **41).** Panel **(B)** shows the fold-change of the fluorescence detection in the presence of the NASBA product **(SEQ ID NO: 42)** over NASBA no RNA control. Data represents the mean ± the standard deviation of fluorescence reads at 1 h after SMART detection of 6 independent experiments.

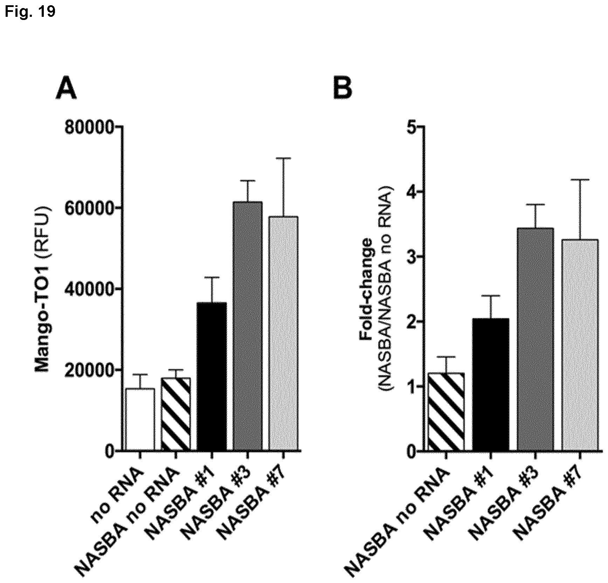

**Figure 19****. Apta-SMART detection of a SARS-CoV-2 S *'spike'* RNA region by SARS-CoV-2 SMART probes after NASBA amplification.** Panel **(A)** shows the raw fluorescence units (RFU) from Mango-TO1 detection (510 nm -exc- and 535 nm -emi-wavelengths) using first and second probes **(SEQ ID NO: 26** and **27)** of three SARS-CoV-2 RNA samples after NASBA amplification (#1, black; #3, dark grey; and #7, light grey bars) as well as a NASBA no RNA control (black lined bar) and a no RNA control (white bar). Panel **(B)** shows the fold-change of the fluorescence detection in the presence of a NASBA amplicon *versus* NASBA no RNA control. The RNA content of the three SARS-CoV-2 RNA samples were previously analyzed by standard qPCR method and positivity confirmed with Ct amplification values of 18 (NASBA #1), 7.5 (NASBA#3) and 24 (NASBA#7). Data represents the mean of fluorescence reads ± the standard deviation at 1 h after SMART detection of 6 independent experiments.

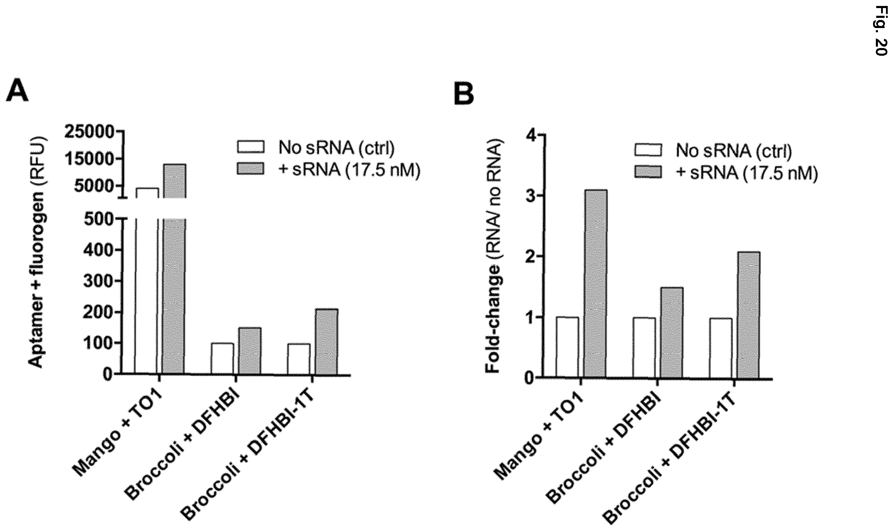

**Figure 20****. Apta-SMART detection of *E.coli* 16S rRNA by Broccoli-SMART probes and a synthetic RNA (sRNA) fragment of 75-nt.** The aptamer and substrate used are Mango and T01-biotin or Broccoli and DFHBI or DFHBI-1T. Panel (A) shows the raw fluorescence units (RFU) from RNA aptamer-substrate detection (510 nm -exc- and 535 nm -emi- wavelengths for Mango and 472 nm -exc- and 507 nm -emi-wavelengths for Broccoli) using a common first **(SEQ ID NO: 7)** and second probes **(SEQ ID NO: 8** for Mango, and **SEQ ID NO: 58** for Broccoli aptamer) and sRNA **(SEQ ID NO: 13).** Panel **(B)** shows the fold-change of the fluorescence detection in the presence of sRNA *versus* no RNA present (ctrl = control). Data represents the fluorescence reads at 1 h after SMART detection of a single experiment.

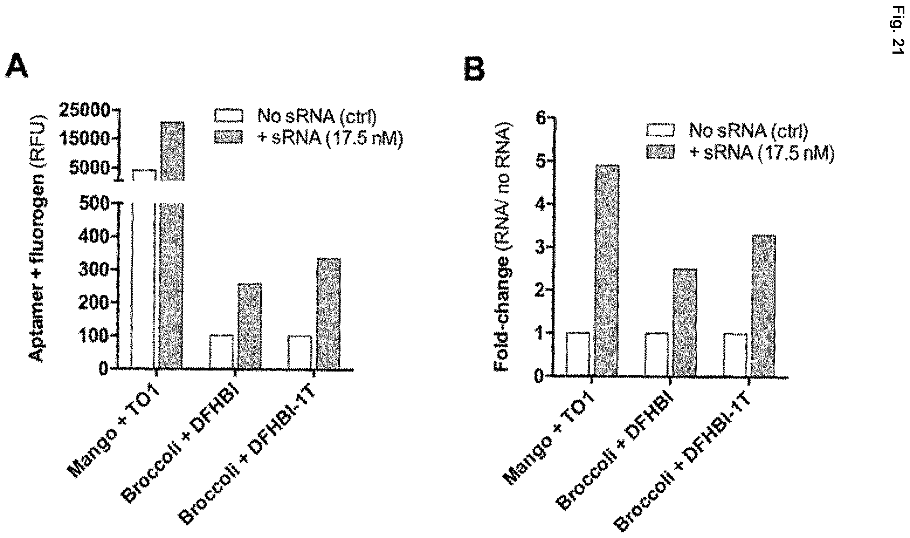

**Figure 21****. Apta-SMART detection of SARS-CoV-2 S *'spike'* 30-nucleotide RNA region using SARS-CoV-2 first and second probes.** The aptamer and substrate used are Mango and TO1-biotin or Broccoli and DFHBI or DFHBI-1T. Panel **(A)** shows the raw fluorescence units (RFU) from RNA aptamer-substrate complex (510 nm -exc- and 535 nm -emi- wavelengths for Mango and 472 nm -exc- and 507 nm -emi-wavelengths for Broccoli) using a common first **(SEQ ID NO: 26)** and second probes **(SEQ ID NO: 27** for Mango, and **SEQ ID NO: 59** for Broccoli aptamer) and sRNA **(SEQ ID NO: 28).** Panel **(B)** shows the fold-change of the fluorescence detection in the presence of synthetic RNA *versus* no RNA present (ctrl = control). Data represents the mean of fluorescence reads at 1 h after SMART detection of a single experiment.
**Figure 22****. SMART and SMART plus amplification RNA probe detection schemes. (A)** SMART detection method (isothermally performed during 1h at 41°C) showing the first and second DNA probes hybridization with a DNA or ssRNA target (in the example). The second DNA probe contains the hybridization region with DNA or ssRNA and 3WJ forming region, the T7 promoter region (in reverse complement, RC) and the detection sequence. During SMART, the activity of Bst polymerase (Bst pol) extends the first probe from 3' to 5' and T7 RNA polymerase (T7 RNA pol) activity makes RNA copies of the detection sequence (that could be detected by different methods, as detailed). **(B)** SMART detection method as shown in **(A)** including an amplification RNA probe that contains a complementary sequence of the RNA (1) (product of T7 RNA pol) and a T7 promoter. Once the RNA (1) binds its complementary sequence again the combined activity of Bst pol and T7 RNA pol will make several copies of the so-called RNA (2) that will be detected by any RNA detection method detailed. Scheme and procedure adapted from Wharam et al. Nucl Acids Res, 2001.

### GENERAL DEFINITIONS

It must be noted that, as used herein, the singular forms "a", "an", and "the", include plural references unless the context clearly indicates otherwise. Further, unless otherwise indicated, the term "at least" preceding a series of elements is to be understood to refer to every element in the series. Those skilled in the art will recognize or be able to ascertain using no more than routine experimentation, many equivalents to the specific embodiments of the invention described herein. Such equivalents are intended to be encompassed by the present invention.

The term "about" when referred to a given amount or quantity is meant to include deviations of plus or minus ten, preferably five, percent.

As used herein, the conjunctive term "and/or" between multiple recited elements is understood as encompassing both individual and combined options. For instance, where two elements are conjoined by "and/or", a first option refers to the applicability of the first element without the second. A second option refers to the applicability of the second element without the first. A third option refers to the applicability of the first and second elements together. Any one of these options is understood to fall within the meaning, and therefore satisfy the requirement of the term "and/or" as used herein. Concurrent applicability of more than one of the options is also understood to fall within the meaning, and therefore satisfy the requirement of the term "and/or."

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having". Any of the aforementioned terms (comprising, containing, including, having), whenever used herein in the context of an aspect or embodiment of the present invention may be substituted with the term "consisting of", though less preferred.

When used herein "consisting of" excludes any element, step, or ingredient not specified in the claim element. When used herein, "consisting essentially of does not exclude materials or steps that do not materially affect the basic and novel characteristics of the claim.

In the context of the present invention, reference to "portions", "regions", "fragments" or "segments" of a nucleotide sequence is made. By "portions", "regions", "fragments" or "segments" is meant herein a specific nucleotide sequence optionally comprised in longer nucleotide sequences. Thus, "portions", "regions", "fragments" or "segments" should be considered synonymous and can be used interchangeably to refer to a specific nucleotide sequence.

The term "complementary" and "complementarity" are interchangeable and refer to the ability of polynucleotides to form base pairs with one another. Base pairs are typically formed by hydrogen bonds between nucleotide units in antiparallel polynucleotide strands or regions. Complementary polynucleotide strands or regions can base pair in the Watson-Crick manner (e.g., A to T, A to U, C to G). 100% (or total) complementary refers to the situation in which each nucleotide unit of one polynucleotide strand or region can hydrogen bond with each nucleotide unit of a second polynucleotide strand or region. Less than perfect (or partial) complementarity refers to the situation in which some, but not all, nucleotide units of two strands or two regions can hydrogen bond with each other and can be expressed as a percentage. Please note that the term "complementary" as used in the present invention also encompasses the term "substantially complementary". A region is "substantially complementary" to a target region when the percentage of complementarity between both regions is of at least 60%, at least 70%, at least 80%, at least 85%, at least 90%, at least 93%, at least 95%, at least 96%, at least 97%, at least 98%, at least 99% or at least 100%. A region is "substantially complementary" to another region if they hybridize under low stringency conditions, preferably medium stringency conditions, most preferably high stringency conditions. Similarly, when the term "non-complementary" is used herein, it should be understood that said term also encompasses the term "substantially non-complementary". A region is "substantially non-complementary" to another region when the complementarity between both regions is less than 40%, less than 30%, less than 20%, preferably less than 10%, most preferably less than 5%, or even most preferably 0% complementarity. A region is "substantially non-complementary" to another region if they do not hybridize under high stringency conditions, preferably medium stringency conditions, most preferably low stringency conditions.

The term "hybridization" is used to refer to the structure formed by 2 independent strands of RNA that form a double stranded structure via base pairings from one strand to the other. These base pairs are considered to be G-C, A-U/T and G-U. (A - Adenine, C -Cytosine, G- Guanine, U - Uracil, T - thymine). As in the case of the complementarity, the hybridization can be total or partial. In the context of the present invention, each uracil and thymine base can be optionally replaced, respectively, by a thymine or uracil base.

Because of the complementary nature of base-pairing between nucleic acid polymers, a double-stranded DNA molecule will be composed of two strands with sequences that are reverse complements of each other. To help molecular biologists specifically identify each strand individually, the two strands are usually differentiated as the "sense" strand and the "antisense" strand. An individual strand of DNA is referred to as positive-sense (also positive (+) or simply sense) if its nucleotide sequence corresponds directly to the sequence of an RNA transcript which is translated or translatable into a sequence of amino acids (provided that any thymine bases in the DNA sequence are replaced with uracil bases in the RNA sequence). The other strand of the double-stranded DNA molecule is referred to as negative-sense (also negative (-) or antisense), and is reverse complementary to both the positive-sense strand and the RNA transcript. It is actually the antisense strand that is used as the template from which RNA polymerases construct the RNA transcript, but the complementary base-pairing by which nucleic acid polymerization occurs means that the sequence of the RNA transcript will look identical to the sense strand, apart from the RNA transcript's use of uracil instead of thymine. Thus, by "reverse complement sequence" of a DNA/RNA element or region (i.e., a promoter) is meant herein the reverse, complement, or reverse-complement counterpart sequence of the positive sense. For example, the positive sense sequence of the T7 promoter is "TAATACGACTCACTATA", and its reverse complement sequence is thus "TATAGTGAGTCGTATTA"

By "functional RNA promoter" or "functional double-stranded RNA polymerase promoter" is meant herein a sequence recognised by an RNA polymerase and which causes the synthesis of RNA in the presence of a suitable polymerase and reagents. Included within the term of "functional RNA promoter" or "functional double-stranded RNA polymerase promoter" is the term "substantially functional", which may be defined for present purposes as a nucleic acid complex which possesses at least 20% or more (preferably at least 50%, more preferably at least 75%, and most preferably at least 90%) of the promoter activity of a fully double stranded, wild type promoter sequence, the relative amount of promoter activity being measured by quantitation of the amount of a given RNA transcript produced by the promoter in a given amount of time, under equivalent conditions (e. g. of temperature and ribonucleotide triphosphate concentration).

By "probe" is meant herein a nucleotide sequence of DNA or RNA, preferably a single-stranded, that is used to search and detect the target nucleic acid. The probes are placed into contact with the sample under conditions that allow the probe sequence to hybridize with the target nucleic acid.

By "functional RNA aptamers transcripts" is referred herein to as aptamer RNA sequences that are able to emit fluorescence after addition of their corresponding fluorophore ligand.

By "three-way junction structure" or "3WJ structure" is referred herein as a branched nucleic acid formed by the hybridization of at least three nucleotides that are partially complementary to each other and that form three double helical arms connected at the junction point, with or without a number of unpair bases in one or more of the three different strands. 3WJ structures are the simplest and most commonly occurring branched nucleic acids. For further definitions and examples of the 3WJ structure, see e.g., Wu, B., Girard, F., van Buuren, B., Schleucher, J., Tessari, M., & Wijmenga, S. (2004). Global structure of a DNA three-way junction by solution NMR: towards prediction of 3H fold. Nucleic acids research, 32(10), 3228-3239. https://doi.org/10.1093/nar/gkh645. The 3WJ structure can also be named 3H or HHH structure according to IUPAC nomenclature (see Lilley DM, Clegg RM, Diekmann S, Seeman NC, von Kitzing E, Hagerman P. Nomenclature Committee of the International Union of Biochemistry and Molecular Biology (NC-IUBMB). A nomenclature of junctions and branchpoints in nucleic acids. Recommendations 1994. Eur J Biochem. 1995 May 15;230(1):1-2. doi: 10.1111/j.1432-1033.1995.tb20526.x. PMID: 7601087.)

By "the 3WJ structure of the present invention" is meant herein the 3WJ structure formed by hybridization of a first and a second probe with a target nucleic acid or nucleic acid of interest as defined below in the first and second aspects of the invention or in any of their embodiments. The 3WJ structure of the present invention is characterized by comprising a first and a second probes, wherein each of the probes comprise a foot region and an arm region, wherein the foot region of the first probe is located at the 5' region of said first probe and is complementary to a first portion of the target nucleic acid and hybridizes thereto, and the arm region of the first probe is located at the 3' region of said first probe and is non-complementary to the target nucleic acid but it is complementary to the arm of the second probe; wherein the foot region of the second probe is located at the 3' region of said second probe and is complementary and hybridizes thereto to a second portion of the target nucleic acid, and the arm region of the second probe is located at the 5' region of said second probe, is complementary to the arm region of the first probe, and comprises, preferably in the 5' to 3' direction:
- a full length reverse complement sequence of at least one fluorogenic aptamer,
- a full length reverse complement sequence of an DNA-directed RNA polymerase promoter, wherein the reverse complement sequence of the at least one fluorogenic aptamer is operably linked to said DNA-directed RNA polymerase promoter,
- a region of between 5-9, preferably 6-8, nucleotides that is complementary and hybridizes thereto to the arm region of the first probe, optionally wherein said region of between 5-9 nucleotides is fully or partially included in the full length reverse complement sequence of the DNA-directed RNA polymerase promoter,
wherein the first portion and the second portion are adjacent or substantially adjacent, preferably wherein the first portion and the second portions are separated by 0 to 10, more preferably 0 to 6, nucleotides, so that when the first and the second probes hybridize with their foot regions to the target nucleic acid, and with their arm regions to each other, the 3WJ structure of the present invention is formed. A representation of the 3WJ structure of the invention with exemplary sequences and target nucleic acid is reproduced in Fig. 4.

### DESCRIPTION OF THE EMBODIMENTS

The SMART (Signal-Mediated Amplification of RNA Technology) assay has the advantage over PCR-based methods that it is an isothermal method and provides a simplification of the technology and protocols, allowing to move the molecular diagnosis from centralized labs with specialized equipment and professionals, to a point of care use.

Typically, the SMART assay comprises the use of a first and a second probe that are capable of forming the so-called three-way junction (3WJ) structure in the presence of target nucleic acid, as shown in Fig. 4. Further, the SMART method includes a detection step, which usually implies the addition of at least another probe (such as molecular beacons) in order to detect the RNA transcripts that are generated after the 3WJ structure is formed. However, the addition of further probes usually comes with the sacrifice of specificity and efficiency, as they need to hybridize efficiently to the target transcripts and then be detected. The authors of the present invention have found a way of improving the detection step of the SMART assay by directly measuring the RNA transcripts that are generated during the method, without needing to add more molecules that hybridize to them. This is achieved by including, in the arm of the second probe, at least an RNA aptamer sequence that, upon transcription, will generate functional RNA aptamers transcripts that will be directly detected after addition of their corresponding fluorophore ligand. This way, the detection is a direct detection, rather than a detection based on intermediate molecules, such as molecular beacons.

The method disclosed herein has been named the Apta-SMART method and, as shown in Example 5, Fig. 9 and 10, is able to detect more efficiently low concentration of target nucleic acid, in comparison to the use of molecular beacon probes. Additionally, it is also shown herein that this method can be implemented after isothermal amplification methods, such as the NASBA method. As shown in Fig. 16 and Example 11, Mango-SMART detection after NASBA assay was able to detect up to 10² CFU/mL of bacterial genome.

In view of this, a **first aspect** of the present invention provides a system, from hereinafter referred to as "a three-way junction structure" or "3WJ structure", formed by a hybridization reaction comprising three nucleic acid molecules. The "3WJ structure" is defined herein as a branched nucleic acid structure that comprises a target nucleic acid molecule and a first and a second nucleic acid probe molecules; wherein the first probe comprises a foot region which is complementary to a first portion of the target and is hybridized thereto, and an arm region which is non-complementary to the target nucleic acid; wherein the second probe comprises a foot region which is complementary to a second portion of the target, such that the foot region of the second probe is hybridized to the target adjacent or substantially adjacent to the foot region of the first probe, the second probe also comprising an arm region which is non-complementary to the target nucleic acid but which is complementary and hybridizes to the arm region of the first probe; and wherein formation of the 3WJ structure leads to the creation of a functional double-stranded RNA polymerase promoter and at least one fluorogenic aptamer in the presence of a DNA-directed DNA polymerase, one strand of the promoter being provided by the elongated arm of the first probe, and the other strand being provided the arm of the second probe. This system is also referred herein to as "the 3WJ structure of the present invention".

By "foot region" we refer herein to a region present in the first and the second probe, which is complementary to a first portion of the target nucleic acid. By "arm region" we refer herein to a region present in the first and second probe which is non-complementary to the target nucleic acid. For the sake of clarity, it is mentioned that the first and second probes can also be referred to as "extension" and "template" probes, respectively .

In a **second aspect,** the invention provides a method of detecting the presence of a target nucleic acid molecule in a sample, the method comprising the steps of contacting the sample comprising the target nucleic acid with the first and second probes, each probe comprising a foot region complementary to respective first and second portions of the target, which portions are adjacent or substantially so; wherein the first and second probes each further comprise an arm region non-complementary to the target, wherein at least part of the arm region of the first probe is complementary to at least part of the arm region of the second probe, such that respective foot regions of the first and second probes hybridize to the target, allowing hybridization of the complementary parts of the arm regions of the first and second probes; thereby creating the 3WJ structure of the present invention comprising, after the elongation of the arm of the first probe by a DNA-directed DNA polymerase, a functional double-stranded RNA polymerase promoter and at least one fluorogenic aptamer, one strand of the promoter and of the at least one fluorogenic aptamer being provided by the first probe, the other strand being provided by the second probe; thereby causing RNA synthesis of the at least one fluorogenic aptamer from the RNA promoter; and detecting said at least one fluorogenic aptamer so synthesised. From hereinafter, the method of the second aspect is also named herein "the method of the present invention".

The method of the second aspect is thus a method of detecting in a sample the presence of a nucleic acid sequence of interest; the method comprising the steps of: a hybridization step comprising contacting a first and second probe as defined herein, with the sample, so as to form the 3WJ structure of the first aspect of the invention (the 3WJ structure of the present invention), wherein the target nucleic acid is the sequence of interest or is formed as a result of the presence in the sample of the sequence of interest; an extension step comprising adding a DNA-directed DNA polymerase; a transcription step comprising a DNA-directed RNA polymerase under suitable conditions for them to be enzymatically active; and a detection step comprising detecting directly the RNA transcripts comprising at least one fluorogenic aptamer of the template portion of the first probe by adding the suitable fluorophore ligand.

In a preferred embodiment, the method of the second aspect of the invention comprises the steps of:
a) adding a first and a second nucleic acid probes to a sample comprising the target nucleic acid so as to form the three-way junction (3WJ) structure of the present invention by hybridization between the target nucleic acid molecule, the first and the second nucleic acid probe (hybridization step),
b) adding a DNA-directed DNA polymerase which extends the arm of the first probe until the end of the arm of the second probe, creating a double-stranded structure comprising a functional RNA polymerase promoter and a at least one fluorogenic aptamer, wherein one strand of the double-stranded structure is provided by the extended arm of the first probe, and the other strand of the double-stranded structure is provided by the arm of second probe (extension step);
c) adding an DNA-directed RNA polymerase which recognises the double-stranded promoter formed in step b), so as to cause the *de novo* synthesis of a single-stranded nucleic acid comprising the at least one fluorogenic aptamer (transcription step); and
d) adding at least one fluorophore ligand of said at least one fluorogenic aptamer thereby directly detecting the *de novo* synthesized single-stranded nucleic acid comprising the at least one fluorogenic aptamer, wherein the detection of said nucleic acid comprising the at least one fluorogenic aptamer indicates the presence of the target nucleic acid in the sample (detection step),
wherein, optionally, the method further comprises an amplification step previous to step a), wherein the target nucleic acid is amplified.

The method of the second aspect of the invention thus results in formation of the complex or 3WJ structure of the first aspect. Therefore, all the embodiments disclosed herein, especially the structural embodiments describing the first and second probes and the 3WJ structure, apply to both the first and the second aspects of the present invention.

It is essential that the first and second probes only hybridize in the presence of the target nucleic acid. The person skilled in the art will know how to select the appropriate conditions, materials and sequences for the probes, in order to ensure that the 3WJ structure occurs in a target dependent manner. Nonetheless, we describe below some preferred embodiments of the 3WJ structure and the method of the invention.

As stated above, the first probe comprises a foot region which is complementary (including substantially complementary) to a first portion of the target nucleic acid, and an arm region which is non-complementary (including substantially non-complementary) to the target nucleic acid. In an embodiment, the foot region is located at the 5' region of the first probe, being the arm region located at the 3' region of the first probe. By "at the 5' region" and "at the 3' region" is referred herein that the foot and arm regions of the first probe are located towards or in the vicinity of the 5' or 3' end, respectively, of the first probe. In an embodiment, the foot region of the first probe is located less than 50, 40, 30, 25, 20, 15, 10, 9, 8, 7, 6, 5, 4, 3, 2, or 1 nucleotides from the 5' end of the first probe. More preferably, the foot region of the first probe is located exactly at the 5' end of the first probe. In an embodiment, the arm region of the first probe is located less than 50, 40, 30, 25, 20, 15, 10, 9, 8, 7, 6, 5, 4, 3, 2, or 1 nucleotides from the 3' end of the first probe. More preferably, the arm region of the first probe is located exactly at the 3' end of the first probe. In an embodiment, the first probe comprises the elements as shown in Fig. 4, wherein the nucleotide sequence of the first probe is adapted/modified to hybridize to the target nucleic acid and to the second probe depending on each case.

The second probe comprises a foot region which is complementary (including substantially complementary) to a second portion of the target nucleic acid and an arm region which is non-complementary (including substantially non-complementary) to the target nucleic acid but which comprises a region that is complementary (including substantially complementary) to the arm region of the first probe. In an embodiment, the foot region is located at the 3' region of the second probe, being the arm region located at the 5' region of the second probe. By "at the 5' region" and "at the 3' region" is referred herein that the foot and arm regions of the second probe are located towards or in the vicinity of the 5' or 3' end, respectively, of the second probe. In an embodiment, the foot region of the second probe is located less than 50, 40, 30, 25, 20, 15, 10, 9, 8, 7, 6, 5, 4, 3, 2, or 1 nucleotides from the 3' end of the second probe. More preferably, the foot region of the second probe is located exactly at the 3' end of the second probe. In an embodiment, the arm region of the second probe is located less than 50, 40, 30, 25, 20, 15, 10, 9, 8, 7, 6, 5, 4, 3, 2, or 1 nucleotides from the 5' end of the second probe. More preferably, the arm region of the second probe is located exactly at the 5' end of the probe. In an embodiment, the foot region of the second probe hybridizes to the target in a region adjacent or substantially adjacent to the place of hybridization of the foot region of the first probe, as will be further explained below. In an embodiment, the second probe comprises the elements shown in Fig. 4, wherein the nucleotide sequence of said second probe is adapted/modified to hybridize to the target nucleic acid and to the second probe depending on each case.

The total number of nucleotides forming the arm region of the first and the second probes is not limited, provided that the arm regions will not become hybridized to each other in the absence of a target. In essence, the degree of complementarity between the arm regions of the first and second probes must be such that, in the conditions employed, they will not hybridize unless stabilized by hybridization of the respective foot regions of the first and second probes to the target, ensuring that the 3WJ structure of the first aspect and the method of the invention are completed only in the presence of the target nucleic acid. Therefore, the arm regions of the first and the second probe only hybridize to each other when the foot regions of the first and second probes have hybridized to the first and second portions, respectively, of the target nucleic acid. The 3WJ structure of the present invention is thus formed between the first probe, the second probe and the target nucleic acid only when the target nucleic acid is present in the sample. In an embodiment, the 3WJ structure is the one depicted in Fig. 4, wherein the specific nucleotide sequence of the first and second probe is adapted/modified to hybridize to the target nucleic acid that wants to be detected, depending on each case.

Therefore, the number of complementary bases between the arm regions of the first and second probes before the DNA-directed DNA polymerase extends the arm of the first probe, will normally be no more than 25, typically less than 15, and optimally between 5 and 13 bases, preferably between 5 and 10 bases, such that the arm regions will not (under the assay conditions employed) become hybridized to each other in the absence of target. In a preferred embodiment, the total length of the arm of the first probe before it is extended is less than 15, 14, 13, 12, 11, 10, 9, 8, or 7 nucleotides. Preferably, the length of the arm of the first probe before it is extended is of at least 5 or 6 nucleotides, preferably between 5-10, more preferably between 5-9, most preferably 6-8 nucleotides (see Figs. 4 and 5).

In an embodiment, the region in the arm of the second probe that is complementary to the arm region of the first probe before the extension step occurs is of at least 5 or 6 nucleotides, preferably between 5-9, more preferably between 6-8, nucleotides. Preferably, the region of the second probe that is complementary to the arm region of the first probe before the extension step occurs is of between 5-10 nucleotides in length, preferably of 6-8 nucleotides in length. In a preferred embodiment, the total length of the arm of the second probe is between 150-50, between 100-50, preferably between 75-50, 100-60, 100-70 nucleotides.

In an embodiment, the arm region of the first probe is shorter than the arm of the second probe, as the arm of the second probe comprises essential elements for the invention (a promoter and at least a fluorogenic aptamer), and its length may be, at least at some extent, limited to the size of said elements.

In an embodiment, the arm of the second probe comprises, preferably in the 5' to 3' direction, the following sequences:
- a full length reverse complement sequence of at least one fluorogenic aptamer,
- a full length reverse complement sequence of an DNA-directed RNA polymerase promoter, wherein the reverse complement sequence of the at least one fluorogenic aptamer is operably linked to said DNA-directed RNA polymerase promoter, and
- a region that is complementary to the arm region of the first probe, optionally wherein said region is fully or partially included in the full length reverse complement sequence of the DNA-directed RNA polymerase promoter.

Preferably, the arm of the second probe comprises in its 5' region a full length reverse complement sequence of at least one, preferably one, fluorogenic aptamer. By "aptamer" is meant herein a short, single-stranded DNA or RNA (ssDNA or ssRNA) molecule that can selectively bind to a specific target, including proteins, peptides, carbohydrates, small molecules, toxins, and even live cells. Although DNA or RNA aptamers are themselves not fluorescent, it is possible to design them so that they can bind to a fluorophore (a small fluorescent molecule) and enhance its fluorescence. In this case, said DNA or RNA aptamers are called fluorogenic aptamers and will emit light when the fluorophore is added. Thus, by "fluorogenic aptamer" is referred herein an aptamer that is able to bind to a fluorophore and form a complex with it enhancing its fluorescence. In some embodiments, more than one fluorogenic aptamer is included. Preferably, the full length reverse complement sequence of 1, 2, 3, 4, 5, or more than 5 fluorogenic aptamers is comprised in the arm of the second probe.

Fluorogenic aptamers are generally known in the art, such as the Spinach aptamer (DB entry 4kzd), the Corn aptamer (PDB entry 5bjp), the Mango I, Mango II, Mango III, Mango IV aptamers (see Autour, A., et al. Fluorogenic RNA Mango aptamers for imaging small non-coding RNAs in mammalian cells. Nat Commun 9, 656 (2018)), the red DIR2s aptamer (PDB entry 6db8), or the Broccoli aptamer (see Filonov, G. S., et al. Broccoli: rapid selection of an RNA mimic of green fluorescent protein by fluorescence-based selection and directed evolution. J. Am. Chem. Soc. 136, 16299-16308), etc. In a preferred embodiment, the aptamer is an RNA fluorogenic aptamer. Preferably, the RNA fluorogenic aptamer is a Mango aptamer or a Broccoli aptamer.

In a preferred embodiment, the RNA fluorogenic aptamer is a Mango aptamer and its sequence comprises or consists of SEQ ID NO: 3 or a sequence which is at least 75%, 80%, 85%, 89%, 90%, 91%, 92%, 95%, 97%, 98%, 99%, or 100% identical over the full length of SEQ ID NO: 3. More preferably, the RNA fluorogenic aptamer is a Mango aptamer and its sequence comprises, consists, or consists essentially of SEQ ID NO: 3. In a preferred embodiment, the RNA fluorogenic aptamer is a Mango aptamer and its reverse complement sequence comprises or consists of SEQ ID NO: 60 or a sequence which is at least 75%, 80%, 85%, 89%, 90%, 91%, 92%, 95%, 97%, 98%, 99%, or 100% identical over the full length of SEQ ID NO: 60. More preferably, the RNA fluorogenic aptamer is a Mango aptamer and its reverse complement sequence comprises, consists, or consists essentially of SEQ ID NO: 60.

In a preferred embodiment, the RNA fluorogenic aptamer is a Broccoli aptamer and its sequence comprises or consists of SEQ ID NO: 57 or a sequence which is at least 75%, 80%, 85%, 89%, 90%, 91%, 92%, 95%, 97%, 98%, 99%, or 100% identical over the full length of SEQ ID NO: 57. More preferably, the RNA fluorogenic aptamer is a Broccoli aptamer and its sequence comprises, consists, or consists essentially of SEQ ID NO: 57. In a preferred embodiment, the RNA fluorogenic aptamer is a Broccoli aptamer and its reverse complement sequence comprises or consists of SEQ ID NO: 61 or a sequence which is at least 75%, 80%, 85%, 89%, 90%, 91%, 92%, 95%, 97%, 98%, 99%, or 100% identical over the full length of SEQ ID NO: 61. More preferably, the RNA fluorogenic aptamer is a Broccoli aptamer and its reverse complement sequence comprises, consists, or consists essentially of SEQ ID NO: 61.

Preferably, the region in the arm of the second probe that is complementary and thus hybridizes to the arm region of the first probe is fully or partially included in the reverse complement sequence of the DNA-directed RNA polymerase promoter, so that both regions overlap. Therefore, in an embodiment, the arm of the second probe comprises or consists of, preferably in the 5' to 3' direction, a full length reverse complement sequence of at least one fluorogenic aptamer followed by and operably linked to a full length reverse complement sequence of an DNA-directed RNA polymerase promoter, wherein the nucleotides in the arm of the second probe that are complementary to the arm of the first probe are partially or are fully included in the full length reverse complement sequence of the DNA-directed RNA polymerase promoter. That is, in an embodiment, the arm of the first probe is complementary or substantially complementary to a region of the reverse complement sequence of the DNA-directed RNA polymerase promoter comprised in the arm of the second probe.

In an embodiment, the first and second probes are DNA and the 3' end of the first probe is extended by a DNA-directed DNA polymerase (also called herein extension step) creating a double-stranded structure comprising a functional double-stranded RNA polymerase promoter and at least one fluorogenic aptamer, wherein one strand of the double-stranded structure is provided by the extended arm of the first probe, and the other strand of the double-stranded structure is provided by the arm of second probe. In this case, once the extension step occurs, the arm of the first probe is thus fully complementary (or substantially complementary) to the arm of the second probe. As mentioned above, extension by the DNA-directed DNA polymerase only occurs when the 3WT structure is formed in the presence of the target nucleic acid. Therefore, the design of the first and second probes is such that, in the absence of target nucleic acid, substantially no *de novo* RNA is synthesized, as no substantially functional double-stranded RNA promoter is formed. In an embodiment, a situation where no target nucleic acid is present in the sample would be that in which the first and second probes, in the absence of target nucleic acid, are unable to provide at least 20%, 15%, more preferably 10%, or even more preferably 5% of the activity of the fully double stranded wild type RNA promoter.

Hence, one of the strands of the promoter is provided by the arm of the second probe in the form of the reverse complement sequence, which will be the "anti sense" (-) strand. The other strand of the promoter, which will be the "sense" (+) strand, is generated in the method of the present invention when the DNA-directed DNA polymerase extends the arm of the first probe until the end of the arm of the second probe, thereby creating a functional double-stranded RNA polymerase promoter. Typically, therefore, the second probe is longer than the first probe and may act as a template for the extension of the first probe, only in presence of the target nucleic acid (and the formation of the 3WJ structure) and upon addition of a DNA-directed DNA polymerase. Once the functional double-stranded RNA polymerase promoter is generated after extension step, the at least one fluorogenic aptamer sequence, which is now in the form of a double-stranded sequence, will be transcribed upon addition of a DNA-directed RNA polymerase (also called herein the transcription step). The extended first probe thus comprises a template portion of at least one fluorogenic aptamer, which is transcribed into multiple RNA copies upon formation of the functional double-stranded RNA polymerase promoter.

Accordingly, the first probe will generally comprise a stretch of "template" nucleic acid to be transcribed, wherein the template comprises the reverse complement sequence of at least one fluorogenic aptamer sequence, and at least one fluorogenic aptamer (positive strand) sequence is thus generated after the enzymatic activity of the DNA-directed DNA polymerase. Therefore, the sequences of the DNA-directed RNA polymerase promoter and the at least one fluorogenic aptamer included in the arm of the second probe are not the positive strands, but the reverse complement of said positive strands, so that once the extension of the arm of the first probe is finished, a functional double stranded DNA-directed RNA polymerase promoter and a double stranded fluorogenic aptamer are formed; where one of the strands (the extended first probe arm) comprises the positive sequence of the at least one fluorogenic aptamer that, upon transcription, the transcript of the at least one fluorogenic aptamer will be in the correct 5' - 3' orientation, and thus it will emit light in the presence of the corresponding fluorophore ligand. Thus, preferably the extended arm of the first probe is designed to be the sense strand.

It is thus also essential that the sequence of the at least one fluorogenic aptamer sequence is operably linked and under the control of the DNA-directed RNA polymerase promoter. A sequence and a promoter are said to be operably linked when they are linked in such a way as to place the transcription of the sequence under the influence or control of the promoter. In this case, the at least one fluorogenic aptamer sequence is operably linked to the promoter nucleotide sequence so that the transcription levels of the at least one fluorogenic aptamer sequence are regulated by the promoter.

In an embodiment, the DNA-directed RNA polymerase promoter is preferably a promoter recognised by bacteriophage polymerases, preferably those promoters recognised by one of T3, T7 or SP6 polymerase. These promoters will generally comprise a minimum of 15 or 16 bases. Preferably, the DNA-directed RNA polymerase promoter is a T7 promoter. Efficient RNA polymerases such as T7 polymerase may then produce large quantities of RNA (including the sequence of the at least one fluorogenic aptamer) from one of the strands of the double stranded nucleic acid formed by the extended first probe and the second probe. Thus in one embodiment, both probes are DNA and extension of the arm of the first probe results in the creation of newly-synthesized double-stranded DNA (dsDNA), said dsDNA comprising a DNA-directed RNA polymerase promoter and a transcription initiation site for transcription by an RNA polymerase, the dsDNA also comprising a double stranded sequence of at least one fluorogenic aptamer, which is operably linked and regulated by the promoter.

In a preferred embodiment, the DNA-directed RNA polymerase promoter is the T7 promoter and its reverse complement sequence comprises or consists of SEQ ID NO: 62 or a sequence which is at least 75%, 80%, 85%, 89%, 90%, 91%, 92%, 95%, 97%, 98%, 99%, or 100% identical over the full length of SEQ ID NO: 62. More preferably, DNA-directed RNA polymerase promoter is the T7 promoter and its reverse complement sequence comprises, consists, or consists essentially of SEQ ID NO: 62.

With regard to the foot regions, there is no upper limit on the size of the foot regions of the first and second probes. Preferably, the foot regions will comprise at least 7, or at least 10 bases, preferably at least 15 or 20 bases, and more preferably at least 25 or at least 30 bases. Preferably, the food region comprises between 8-20, 10-40, 15-35, preferably 15-30 or 15-25 bases. In practice, it is also preferred for the foot regions to comprise no more than about 75 bases nor less than 7 bases.

In an embodiment, the target nucleic acid comprises a first portion and a second portion, wherein the first portion is located at 3' of the second portion. In an embodiment, the first portion of the target nucleic acid is complementary (including substantially complementary) to the foot region of the first probe, and the second portion of the target nucleic acid is complementary (including substantially complementary) to the foot region of the second probe.

It is an essential feature of the invention that the first and second probes, when hybridized to the first and second portions, respectively, of the target nucleic acid sequence, are adjacent or substantially adjacent to each other. Use of the term "adjacent" is herein intended to mean that there are no nucleotides (i.e., there are 0 nucleotides) of the target sequence left without base-pairing between the first and second portion of the target nucleic acid sequence which are base paired to the complementary sequence of the probes. This proximity between the probes enables the complementary arm portions of the probes to anneal. As will readily be apparent to those skilled in the art, by designing the probes so as to allow for annealing to each other at greater separations from the target sequence, gaps between the first and second portions of the target sequence may be introduced between nucleotides to which the foot of the probes hybridize In this situation the probes are said to be "substantially adjacent", because there may be some nucleotides of the target sequence left without base-pairing between those portions of the target sequence which are base-paired to the probes. Preferably, the term "substantially adjacent" is herein intended to mean that there is a maximum of 15 nucleotides, preferably a maximum of 10, 9, 8, 7, 6, 5, 4, 3, 2 or 1 nucleotides of the target sequence left without base-pairing between the first and second portions of the target sequence which are base paired to the complementary sequence of the probes. Clearly, the number of intervening unpaired nucleotides of the target sequence can vary according to the design of the probes. Thus, whilst it is preferred that the first and second probes hybridize so as to be adjacent, that is, as to leave 0 nucleotides in between the first and second portion of the target sequence, the foot of each probes may be separated by a maximum of 10, 9, 8, 7, 6 nucleotides of target sequence, and the term "substantially adjacent" is intended in the present invention to refer to such latter situation.

In an embodiment, the first probe comprises, preferably in the 5' to 3' direction, the following sequences:
- a foot region which is complementary (including substantially complementary) to a first portion of the target nucleic acid and hybridizes thereto,
- an arm region which is non-complementary (including substantially non-complementary) to the target nucleic acid and which is complementary (including substantially complementary) to a part of the arm of the second probe.

In the presence of the target nucleic acid, when the 3WJ structure is formed, but before the DNA-directed DNA polymerase is added (i.e, before the extension step occurs), the arm region of the first probe is only complementary to the arm of the second probe in less than 9, 8, 7, 6, or 5 nucleotides. Preferably only between 6-8 nucleotides of the arm of the first probe are complementary to the arm of the second probe, before the arm of the first probe is extended, as previously explained.

However, in the presence of the target nucleic acid, when the 3WJ structure of the invention is formed and, after the activity of the DNA-directed DNA polymerase (i.e. after the extension step), the arm region of the first probe is extended and thus, the extended first probe comprises, preferably in the 5' to 3' direction:
- a foot region which is complementary (including substantially complementary) to a first portion of the target nucleic acid and is hybridized thereto,
- an arm region which is which is non-complementary (including substantially non-complementary) to the target nucleic acid and which is complementary (including substantially complementary) to the arm of the second probe in its entire length, wherein the arm region comprises, preferably in the 5' to 3' direction, the sense strand of a functional double-stranded RNA promoter and the sense strand of at least one fluorogenic aptamer, wherein the at least one fluorogenic aptamer sequence is operably linked and under the control of said RNA promoter.

In a preferred embodiment, the first probe comprises or consists of SEQ ID NO: 1, 7, 10, 11, 12, 14, 15, 16, 17, 18, 19, 20, 21, 22, 26, 35, 40, 45, 46, or a sequence which is at least 75%, 80%, 85%, 89%, 90%, 91%, 92%, 95%, 97%, 98%, 99%, or 100% identical over the full length of SEQ ID NO: 1, 7, 10, 11, 12, 14, 15, 16, 17, 18, 19, 20, 21, 22, 26, 35, 40, 45, or 46. More preferably, the first probe comprises, consists, or consists essentially of SEQ ID NO: 1, 7, 10, 11, 12, 14, 15, 16, 17, 18, 19, 20, 21, 22, 26, 35, 40, 45, or 46.

In an embodiment, the second probe comprises, preferably in the 5' to 3' direction, the following sequences:
- a full length reverse complement sequence of at least one fluorogenic aptamer, preferably Mango or Broccoli aptamers,
- a full length reverse complement sequence of an DNA-directed RNA polymerase promoter, preferably the T7 promoter, wherein the reverse complement sequence of the at least one fluorogenic aptamer is operably linked to said DNA-directed RNA polymerase promoter,
- a region that is complementary (including substantially complementary) and hybridizes thereto to the arm region of the first probe, wherein optionally said region is fully or partially included in the full length reverse complement sequence of the DNA-directed RNA polymerase promoter, and
- a foot region which is complementary (including substantially complementary) to a second portion of the target nucleic acid and is hybridized thereto.

In a preferred embodiment, the second probe comprises or consists of SEQ ID NO: 2, 8, 23, 24, 25, 27, 36, 41, 47, 48, 58, 59, 63-74 or a sequence which is at least 75%, 80%, 85%, 89%, 90%, 91%, 92%, 95%, 97%, 98%, 99%, or 100% identical over the full length of SEQ ID NO: 2, 8, 23, 24, 25, 27, 36, 41, 47, 48, 58, 59, 63-74. More preferably, the second probe comprises, consists, or consists essentially of SEQ ID NO: 8, 23, 24, 25, 27, 36, 41, 47, 48, 58, 59, 63-74.

In a preferred embodiment, the first probe comprises or consists of SEQ ID NO: 1 (*E.coli* 23S rRNA) and the second probe comprises or consists of SEQ ID NO: 2 or 63 (*E.coli* 23S rRNA), and the target sequence comprises or consists of SEQ ID NO: 37 or 39, or a sequence which is at least 75%, 80%, 85%, 89%, 90%, 91%, 92%, 95%, 97%, 98%, 99%, or 100% identical over the full length of SEQ ID NO: 1, 2, 63, 37 or 39, respectively.

In a preferred embodiment, the first probe comprises or consists of SEQ ID NO: 7, 16, 17, 18, 19, 20, 21 or 12 , the second probe comprises or consists of SEQ ID NO: 8, 23, 24, 58, 64, 65, 66 or 73, and the target sequence comprises or consists of SEQ ID NO: 9 or 13 (*E.coli* 16S rRNA), or a sequence which is at least 75%, 80%, 85%, 89%, 90%, 91%, 92%, 95%, 97%, 98%, 99%, or 100% identical over the full length of SEQ ID NO: 7, 16, 17, 18, 19, 20, 21, 12, 8, 23, 24, 58, 64, 65, 66, 73, 9 or 13, respectively.

In a preferred embodiment, the first probe comprises or consists of SEQ ID NO: 26, the second probe comprises or consists of SEQ ID NO: 27, 59, 68, or 73 and the target sequence comprises or consists of SEQ ID NO: 28 or 29 (SARS-CoV-2), or a sequence which is at least 75%, 80%, 85%, 89%, 90%, 91%, 92%, 95%, 97%, 98%, 99%, or 100% identical over the full length of SEQ ID NO: 26, 27, 59, 68, 73, 28, or 29, respectively.

In a preferred embodiment, the first probe comprises or consists of SEQ ID NO: 35, the second probe comprises or consists of SEQ ID NO: 36 or 69, and the target sequence comprises or consists of SEQ ID NO: 37 or 39 (*E*. *coli* 23S rRNA), or a sequence which is at least 75%, 80%, 85%, 89%, 90%, 91%, 92%, 95%, 97%, 98%, 99%, or 100% identical over the full length of SEQ ID NO: 35, 36, 69, 37 or 39, respectively.

In a preferred embodiment, the first probe comprises or consists of SEQ ID NO: 40 the second probe comprises or consists of SEQ ID NO: 41 or 70, and the target sequence comprises or consists of SEQ ID NO: 42 or 44 (*S.aureus mecA*), or a sequence which is at least 75%, 80%, 85%, 89%, 90%, 91%, 92%, 95%, 97%, 98%, 99%, or 100% identical over the full length of SEQ ID NO: 40, 41, 70, 42 or 44, respectively.

In a preferred embodiment, the first probe comprises or consists of SEQ ID NO: 45 the second probe comprises or consists of SEQ ID NO: 47 or 71, and the target sequence comprises or consists of SEQ ID NO: 49 (*Influenza A*), or a sequence which is at least 75%, 80%, 85%, 89%, 90%, 91%, 92%, 95%, 97%, 98%, 99%, or 100% identical over the full length of SEQ ID NO: 45, 47, 71 or 51 respectively.

In a preferred embodiment, the first probe comprises or consists of SEQ ID NO: 46 the second probe comprises or consists of SEQ ID NO: 48 or 72, and the target sequence comprises or consists of SEQ ID NO: 50 (*Influenza B*), or a sequence which is at least 75%, 80%, 85%, 89%, 90%, 91%, 92%, 95%, 97%, 98%, 99%, or 100% identical over the full length of SEQ ID NO: 46, 48, 72 or 50, respectively.

Any of the first or second probes may comprise DNA, peptide nucleic acid (PNA), locked nucleic acid (LNA), RNA, or any combination thereof. It will, however, generally be desirable that those portions of the probes which constitute the promoter comprise conventional DNA, so as to ensure recognition by the relevant polymerase. The terms "nucleic acid complex", "nucleic acid molecule" and "nucleic acid probe" should accordingly not be construed as being limited to complexes, molecules or probes (respectively) which consist solely of conventional nucleic acid, but also encompass complexes, molecules or probes which comprise non-conventional nucleic acid (such as PNA or LNA) or non-nucleic acid portions.

PNA is a synthetic nucleic acid analogue in which the sugar/phosphate backbone is replaced by a peptide-linked chain (typically of repeated N- (2-aminoethyl)-glycine units), to which the bases are joined by methylene carbonyl linkages. PNA/DNA hybrids have high Tm values compared to double stranded DNA molecules, since in DNA the highly negatively-charged phosphate backbone causes electrostatic repulsion between the respective strands, whilst the backbone of PNA is uncharged. Another characteristic of PNA is that a single base mis-match is, relatively speaking, more destabilizing than a single base mis-match in heteroduplex DNA. Accordingly, PNA is useful to include in probes for use in the present invention, as the resulting probes have greater specificity than probes consisting entirely of DNA. Synthesis and uses of PNA is known in the technical field of this invention.

LNA is a synthetic nucleic acid analogue, incorporating "internally bridged" nucleoside analogues. Synthesis of LNA, and properties thereof, have been described extensively in the art.

Additionally, a spacer molecule may be added between the foot and the arm regions of the first and/or the second probes. By "spacer molecule" is referred herein to any molecule or molecules (including nucleotide sequences, organic and inorganic compounds, etc.) that connect, on the one hand, the foot region of the probe and, on the other hand, the arm region of the probe. Preferably, the spacer is a non-nucleosidic spacer. Preferably, the spacer molecule is an organic compound comprising 20-15, preferably 18, atoms. Preferably, the spacer molecule is an organic compound comprising 18, 17, 16, 15, 14, 13, 12, 11, 10, or less than 10 carbon atoms. Preferably, the spacer molecule is an 18-atom hexa-ethyleneglycol spacer (referred here as iSp18 spacer, see Fig 4).

Further, the first and/or second probe may also be coupled at their 3' or 5' end to another compound or element. In an embodiment, the 3' end of the second probe is blocked, so that the RNA polymerase-mediated extension thereof is not possible from that end. In an embodiment, said blocking compound or element is coupled at the 3' end. Blocking of the 3' end is conveniently accomplished by providing a phosphate group, or a propyl group, instead of an-OH group, on the 3' terminal nucleotide. Other methods of blocking the 3'end are well known to those skilled in the art. In a preferred embodiment, the compound or element coupled to the molecule, preferably coupled to the second probe, is a 3'-amino group, preferably NH₃ (see Fig. 4). In an embodiment, the compound or element coupled to the molecule, preferably coupled to the second probe, is a 3' Amino Modifier (also referred herein as /3AmMO/).

The target nucleic acid is the sequence of interest that wants to be detected or is formed as a result of the presence in the sample of the sequence of interest. Thus, depending on the use to which the invention is applied, the "sequence of interest" or the "target nucleic acid" may be very long (for example, an entire gene) or may be rather short. Thus, the first and second probes defined previously may hybridize to the target sequence such that they are joined to just a small length of the sequence of interest or they may be joined such that some of the target sequence is beyond the sequence which is strictly of interest.

Said target may be DNA, RNA, and/or a hybrid DNA/RNA. The target sequence may be formed as a result of the presence in the sample of the sequence of interest (e.g. by PCR, or by any other amplification method). Preferably, the target nucleic acid is or is derived from an infectious disease agent. Preferably, the target nucleic acid is an RNA molecule derived from the genome of an infectious agent such as a bacterium or a virus. In an embodiment, the target nucleic acid is or is derived from a bacterial genome, preferably bacterial 16S or 23S rRNA.

In an embodiment, the target nucleic acid is or is derived from a viral genome, preferably viral RNA genome. Preferably, the target nucleic acid is or is derived from the genome of a positive-strand RNA virus, preferably a Coronavirus or an Influenza virus genome. Preferably, the Coronavirus is SARS-CoV-2 virus. Preferably, the Influenza virus is Influenza A or B virus.

With regard to the sample, in an embodiment, the sample is an isolated biological sample. Exemplary biological samples include tissue samples (such as tissue sections and needle biopsies of a tissue); cell samples (e.g., cytological smears (such as Pap or blood smears) or samples of cells obtained by microdissection); samples of whole organisms (such as samples of yeasts or bacteria); or cell fractions, fragments or organelles (such as obtained by lysing cells and separating the components thereof by centrifugation or otherwise). Other examples of biological samples include blood, serum, urine, semen, fecal matter, cerebrospinal fluid, interstitial fluid, mucous, tears, sweat, pus, biopsied tissue (e.g., obtained by a surgical biopsy or needle biopsy), nipple aspirates, milk, vaginal fluid, saliva, swabs (such as buccal swabs), or any material containing biomolecules that is derived from a said biological sample. In some embodiments, the biological sample can be a body fluid, which can be fluid isolated from the body of an individual. For example, "body fluid" may include blood, plasma, serum, mucus, bile, saliva, nasopharyngeal swabs, urine, tears, perspiration, or washings from bodily cavities.

The biological sample may be obtained from a subject in need of the analysis. A "subject" may be a human (i.e., male or female of any age group, for example, pediatric subject (e.g., infant, child, or adolescent) or adult subject (e.g., young adult, middle-aged adult, or senior adult). Alternatively, the subject may be a non-human animal. In certain embodiments, the non-human animal is a mammal (e.g., primate, for example, cynomolgus monkey or rhesus monkey), commercially relevant mammal (e.g., cattle, pig, horse, sheep, goat, cat, or dog), or bird (e.g., commercially relevant bird, such as chicken, duck, goose, or turkey). In other examples, the non-human animal is a fish, reptile, or amphibian. The non-human animal may be a transgenic animal or genetically engineered animal. In some examples, the subject may also be a plant.

Step a) of the method of the second aspect comprises adding the first and the second probes to a sample comprising the target nucleic acid as to form the 3WJ structure. It will be apparent to those skilled in the art that the order of addition of probes in the step a) of the method is not critical: the first probe may, for example, be mixed to the second first probe before the sample (potentially comprising the target nucleic acid to be detected) is added. Alternatively, all probes may be simultaneously mixed with the sample containing the target molecule.

Thus, this step is a hybridization step that results in the formation of the 3WJ of the first aspect if there is target nucleic acid in the sample. Hybridization refers to the ability of complementary single-stranded DNA or RNA to form a complex. The hybridization step can be performed under suitable hybridization conditions, which are within the knowledge of those skilled in the art. Hybridization conditions resulting in particular degrees of stringency will vary depending upon the nature of the hybridization method and the composition and length of the hybridizing nucleic acid sequences. Generally, the temperature of hybridization and the ionic strength of the hybridization buffer will determine the stringency of hybridization. Calculations regarding hybridization conditions for attaining particular degrees of stringency are routine and known in the art. The hybridization temperature can be determined based on various factors, for example, the length of the complementary regions between the probes and the target nucleic acid, the composition of the complementary regions (e.g., G/C content), and the stringency needed, which are within the knowledge of those skilled in the art.

In an embodiment, the first and the second probe are designed such that suitable hybridization of both probes and the target nucleic acid occurs at substantially the same temperature. In such cases, this step may be performed in a single step. By "suitable temperature" is meant herein a temperature under which the first and second probe form the 3WJ structure with the target nucleic acid with high specificity and form little or no complexes with other nucleic acids, even those that share sequence homology with the target nucleic acid of interest. Such a suitable hybridization temperature can be determined based on various factors as known to those skilled in the art, for example, melting temperatures of the capture and detection probes, ion strength, length of the target nucleic acid, or presence of homologous non-target nucleic acids in the same sample.

Step b) of the method of the second aspect, also called extension step, comprises adding or providing a DNA-directed DNA polymerase which extends the arm of the first probe until the end of the arm of the second probe, creating a double-stranded structure comprising a functional RNA polymerase promoter and at least one fluorogenic aptamer, wherein one strand of the double-stranded structure is provided by the extended arm of the first probe, and the other strand of the double-stranded structure is provided by the arm of second probe.

The DNA-directed DNA polymerase catalyses DNA synthesis by addition of deoxyribonucleotide units to a DNA chain using DNA as a template. In this case, the template is the arm of the second probe, and the addition of deoxyribonucleotide units occurs in the arm of the first probe, causing the extension of said arm and thus increasing the number complementary bases between the arms of the first and the second probe. As explained above, the extension of the arm of the first probe creates a functional double-stranded RNA promoter and the positive strand of the at least one fluorogenic aptamer.

In an embodiment, the DNA-directed DNA polymerase is a prokaryote polymerase enzyme, preferably of type I. Preferably the DNA-directed DNA polymerase is *Bacillus stearothermophilus* DNA Polymerase I (Bst).

The extension step is performed under suitable enzymatic conditions, which are within the knowledge of those skilled in the art and that will depend on the DNA-directed DNA polymerase chosen.

Step c) of the method, also called transcription step, comprises adding or providing a suitable DNA-directed RNA polymerase which recognises the double-stranded promoter formed in step b), so as to cause the *de novo* synthesis of a plurality of single-stranded nucleic acid copies comprising the at least one fluorogenic aptamer. In an embodiment, the DNA-directed RNA polymerase may be T7, T3, or SP6 polymerase. Addition of a suitable DNA-directed RNA polymerase thereby produces RNA copies of the sense strand, which in this case is the arm of the first probe, being said RNA copies functional fluorogenic aptamers.

The choice of the RNA polymerase will depend on which reverse complement sequence is placed in the arm of the second probe. Thus, if the arm of the second probe comprises the reverse complement sequence of the T7 promoter, then the DNA-directed RNA polymerase added in step c) of the method should be the T7 polymerase.

The transcription step can be performed under suitable enzymatic conditions, which are within the knowledge of those skilled in the art and that will depend on the RNA polymerase chosen.

Step d) of the method, also called the detection step, comprises adding or providing at least one fluorophore ligand of said at least one fluorogenic aptamer thereby directly detecting the *de novo* synthesized nucleic acid comprising the at least one fluorogenic aptamer, wherein the detection of said nucleic acid comprising the at least one fluorogenic aptamer indicates the presence of the target nucleic acid in the sample.

Therefore, step d) of the method implies the direct detection of the synthesized nucleic acid, making it unnecessary to further amplify the *de novo* synthesized nucleic acid or to add any other oligonucleotide for the detection of the sequence. Those skilled in the art will appreciate that both the second probe and the target sequence are required to hybridize, at least partially, to the first probe, in order to form the 3WJ structure and the functional double-stranded RNA promoter. Accordingly, RNA transcripts of the template portion of the first probe comprising at least one fluorogenic aptamer are indicative of the presence in a sample of the target sequence. Thus, the 3WJ structure of the invention provides the basis for an assay comprising the direct detection of a nucleic acid sequence of interest in a sample.

Similar as above, the conditions to perform the detection step are within the knowledge of those skilled in the art.

Therefore, the method of the present invention provides a one-step method to detect target nucleic acid. In preferred embodiments, the invention provides a method of generating a signal in a target-dependent manner (i.e. creation of the 3WJ structure and hence formation of the functional promoter) and causing amplification of this signal (generation of multiple RNA transcripts, comprising at least one fluorogenic aptamer, under the control of the promoter) in a system that only requires the use of two enzymes (DNA and RNA polymerases), and a fluorophore ligand, without the need for additional steps or reactive or enzymes to bring about the detection.

The fluorophore ligand to be added in the detection step depends on the fluorogenic aptamer or aptamers included in the arm of the second probe, as it needs to be suitable for said aptamer(s). For example, if a Mango aptamer is included, then the fluorophore ligand should be thiazole orange (TO1) derivatives, such as TO 1 biotin fluorogen. If a Broccoli aptamer is included in the sequence of the second probe, then the ligand to be added I step d) is 5Z)-5-[(3,5-Difluoro-4-hydroxyphenyl)methylene]-3,5-dihydro-2,3-dimethyl-4H-imidazol-4-one (DFHBI) or (5Z)-5-[(3,5-Difluoro-4-hydroxyphenyl)methylene]-3,5-dihydro-2-methyl-3-(2,2,2-trifluoroethyl)-4H-imidazol-4-one (DFHBI-1T).

When the interaction between the RNA transcript comprising the at least one fluorogenic aptamer with the at least one fluorophore ligand occurs, the fluorescence of the fluorophore significantly increases compared to the fluorescence of the fluorophore that does not form a complex with the at least one fluorogenic aptamer. Thus, in an optional step e) of the method, also called the quantification step, the fluorescence emitted by the at least one fluorophore-aptamer complex is measured and quantified by fluorescence reading. In an embodiment, step e) of the method includes the determination of the levels or concentration of the target nucleic acid present in the sample. Methods for measuring fluorescence are known in the art and may include the use of fluorescence plate readers.

To measure the levels (concentration) of a target nucleic acid in a sample, a calibration curve may be developed using samples containing known concentrations of the target nucleic acid molecule. The concentration of the target nucleic acid in a sample may be determined by comparison of a measured parameter to a calibration standard. In some cases, a calibration curve may be prepared, wherein the total measured signal is determined for a plurality of samples comprising the target nucleic acid at a known concentration using a substantially similar assay format. For example, the total intensity of the array may be compared to a calibration curve to determine a measure of the concentration of the target nucleic acid in the sample. The calibration curve may be produced by completing the method with a plurality of standardized samples of known concentration under similar conditions used to analyse test samples with unknown concentrations. A calibration curve may be used to relate the detected signal of the target nucleic acid (i.e., of the fluorophore-aptamer formed when the target nucleic acid is present) complex with a known concentration of the target nucleic acid. The assay may then be completed on a sample containing the target nucleic acid or fragment in an unknown concentration, and the signals detected may be compared to the calibration curve, (or a mathematical equation fitting the same) to determine a measure of the concentration of the target nucleic acid in the sample.

Given the high sensitivity of the method of the invention, a target nucleic acid may not need to be pre-amplified. However, in a preferred embodiment, the target nucleic acid is amplified before step a) of the method of the present invention. Thus, in an embodiment, the method of the present invention further comprises an amplification step previous to step a) comprising amplifying the target nucleic acid to provide a plurality of molecules identical to the target nucleic acid or the reverse complement thereof, wherein said plurality of molecules represent the target nucleic acid to be detected in the method steps a) to d), preferably a) to e), as defined above. Methods to amplify the target nucleic acid to provide a plurality of molecules identical to the target nucleic acid are known in the art. Preferably, the amplification method used is an isothermal amplification method, more preferably an isothermal amplification method selected from the group consisting of Loop-Mediated Isothermal Amplification (LAMP), Helicase-Dependent Amplification (HDA), Rolling Circle Amplification (RCA), Multiple Displacement Amplification (MDA), Recombinase Polymerase Amplification (RPA), Nucleic Acid Sequence-Based Amplification (NASBA). Preferably, the method of the present invention comprises an amplification step before step a), wherein said amplification step is carried out by nucleic acid sequence-based amplification (NASBA).

In an embodiment, said amplification step is optionally followed by a step of degrading residual amplification primers and dephosphorylating excess dNTPs after amplification. In an embodiment, the step of degrading residual amplification primers and dephosphorylating excess dNTPs is carried out by an exonuclease digestion followed by a phosphatase reaction.

Therefore, in a preferred embodiment, the method of the invention comprises the steps of:
a) amplifying the target nucleotide, preferably by using a isothermal amplification method, preferably NASBA method, and subsequently or simultaneously, preferably subsequently, adding to the sample a first and a second nucleic acid probes so as to form the three-way junction structure of the invention by hybridization between the target nucleic acid molecule, the first and the second nucleic acid probe, wherein:
   i) the first probe comprises a foot region located at the 5' region of the probe which is complementary to a first portion of the target nucleic acid and hybridizes thereto, and an arm region located at the 3' region of the probe;
   ii) the second probe comprises:
      (1) a foot region located at the 3' region of the probe which is complementary and hybridizes thereto to a second portion of the target nucleic acid, and
      (2) an arm region located at the 5' region of the probe, comprising, preferably in the 5' to 3' direction:
         - a full length reverse complement sequence of at least one fluorogenic aptamer,
         - a full length reverse complement sequence of an DNA-directed RNA polymerase promoter, wherein the reverse complement sequence of the at least one fluorogenic aptamer is operably linked to said DNA-directed RNA polymerase promoter,
         - a region comprising between 5-9, preferably 6-8, nucleotides that is complementary and hybridizes thereto to the arm region of the first probe, optionally wherein said region comprising between 5-9, preferably 6-9, nucleotides is fully or partially included in the full length reverse complement sequence of the DNA-directed RNA polymerase promoter, and
   iii) the target nucleic acid comprises a first portion located at the 3' region of the target nucleic acid which is complementary to the foot region of the first probe, and a second portion located at the 5' region of the target nucleic acid which is complementary to the foot region of the second probe, wherein the first portion and the second portion are adjacent or substantially adjacent, preferably wherein the first portion and the second portions are separated by 0 to 10, more preferably 0 to 6, nucleotides, and wherein the three-way junction structure is formed between the first probe, the second probe and the target nucleic acid when the target nucleic acid is present in the sample, and
b) adding a DNA-directed DNA polymerase which extends the arm of the first probe until the end of the arm of the second probe, creating a double-stranded structure comprising a functional RNA polymerase promoter and at least one fluorogenic aptamer, wherein one strand of the double-stranded structure is provided by the extended arm of the first probe, and the other strand of the double-stranded structure is provided by the arm of second probe;
c) adding a DNA-directed RNA polymerase which recognises the double-stranded promoter formed in step b), so as to cause the *de novo* synthesis of a single-stranded nucleic acid comprising the at least one fluorogenic aptamer; and
d) adding at least a fluorophore ligand of said at least one fluorogenic aptamer thereby directly detecting, and optionally quantifying, the *de novo* synthesised nucleic acid comprising the at least one fluorogenic aptamer, wherein the detection, optionally quantification, of said nucleic acid comprising the at least one fluorogenic aptamer indicates the presence of the target nucleic acid in the sample.

Optionally, further steps may be included in between, before, or after the steps described above. Such further steps may be, for instance, one or more washing steps. Further, preferably, some of the reagents and buffers used in the amplification step, for instance, the deoxynucleotide triphosphates, are also used in the hybridization step of the method, thereby simplifying the method without compromising the efficiency while saving reagents and time.

It will be apparent to those skilled in the art that the steps of the method of the second aspect or any of its embodiments may be performed in order, i.e., from steps a) to d), optionally from a) to e), or, alternatively, all steps may be simultaneously performed by adding the reagents defined in each of these steps at the same time to the sample containing the target molecule. Thus, in an embodiment, the method is performed following steps a) to d), optionally to e), in said order. In another embodiment, the method is performed carrying out all steps a) to d), optionally to e), simultaneously.

In an embodiment, the method of the invention is an isothermal method, so that all the reactions described herein can potentially be achieved at a single temperature, preferably at about between 30°C and 45°C, more preferably between 35°C and 43°C, most preferably between 37°C and 41°C. In a preferred embodiment where an amplification step is included previous the method of the present invention, said amplification step is performed at an single temperature, preferably of about 35°C-40°C, more preferably 37°C, and the subsequent method of the present invention is performed at a different temperature, preferably between 40°C-43°C, more preferably at 41°C. The skilled artisan would know what enzymes are suitable for the method of the present invention to be performed isothermally, and what temperatures to choose accordingly to obtain the optimum efficiency of the method.

The applications of the method of the present invention include detecting the presence and/or measuring the level of a target nucleic acid of interest in a suitable sample. In some embodiments, the sample may be a biological sample obtained from a subject and the results obtained from the assay methods described herein may be used for diagnostic and/or prognostic purposes. In other embodiments, the assay methods described herein can be used in research settings for detecting presence or measuring the level of a target nucleic acid in a sample. The method may be applied in a diagnostic/prognostic setting to detect the presence or measure the level of a nucleic acid biomarker that is associated with a target disease. For example, the methods may be used to detect/measure the presence of an infectious agent, which may be associated with a specific disease, e.g., covid or flu. The methods can be used in detecting such a nucleic acid biomarker in subjects that are absent of any symptom of the disease for early stage diagnosis. The assay methods can also be used to detect nucleic acids of microorganisms for determining whether a subject has been infected by such microorganisms, for example, viruses (e.g., HBV, HCV, HPV, HIV, Influenza, coronavirus).

The method described herein can be used for direct detection of nucleic acids in a sample in a very sensitive and efficient way. Further, the method described herein can also be used to detect several targets present in a sample or measure the levels of two or more targets (multiplex assay) of a sample. In this case, the two or more target nucleic acids may then be detected using different second probes comprising different fluorogenic aptamers, so that a different signal is associated with each target nucleic (i.e., each i.e., fluorophore-aptamer complex). For example, in a sample suspected of containing three different target nucleic acids, the detection step may comprise (i) measuring a first signal released from a first fluorophore-aptamer complex and determining the presence or a level of a first target nucleic acid in the sample based on the intensity of the first signal; (ii) measuring a second signal released from a second fluorophore-aptamer complex and determining the presence or a level of the second target nucleic acid in the sample based on the intensity of the second signal; and (iii) measuring a third signal released from a third fluorophore-aptamer complex and determining the presence or a level of the third target nucleic acid in the sample based on the intensity of the second signal.

In a **third aspect,** the present invention relates to a computer-implemented method for designing a pair of probes, preferably the first and the second probe as defined above, for use in the method of the present invention. In an embodiment, the method of the third aspect comprises the steps of:
i) reading the target nucleic acid,
ii) obtaining or generating the sequence of at least a pair of probes characterized in that one of them comprises or consists of the sequence of the first probe as defined above and the other one comprises or consists of the sequence of the second probe as defined above, and further characterized in that they are capable of forming the three-way junction structure of the invention by hybridization with the target nucleic acid molecule, and
iii) optionally, providing the sequences obtained or generated in step ii) as output.

In a preferred embodiment of the third aspect, the computer-implemented method comprises the steps of:
i) reading a target nucleic acid,
ii) obtaining or generating the sequence of at least a pair of probes, wherein each of the probes comprise a foot region and an arm region wherein:
   the foot region of the first probe is located at the 5' region of said probe and is complementary to a first portion of the target nucleic acid and hybridizes thereto, and the arm region of the first probe is located at the 3' region of said probe and is non-complementary to the target nucleic acid, and wherein
   the foot region of the second probe is located at the 3' region of said probe and is complementary and hybridizes thereto to a second portion of the target nucleic acid, and the arm region of the second probe is located at the 5' region of said probe and comprises, preferably in the 5' to 3' direction:
      - a full length reverse complement sequence of at least one fluorogenic aptamer,
      - a full length reverse complement sequence of an DNA-directed RNA polymerase promoter, wherein the reverse complement sequence of the at least one fluorogenic aptamer is operably linked to said DNA-directed RNA polymerase promoter,
      - a region of between 5-9 nucleotides that is complementary and hybridizes thereto to the arm region of the first probe, optionally wherein said region of between 5-9 nucleotides is fully or partially included in the full length reverse complement sequence of the DNA-directed RNA polymerase promoter,
   wherein the first portion and the second portion are adjacent or substantially adjacent, preferably wherein the first portion and the second portions are separated by 0 to 10, more preferably 0 to 6, nucleotides,
   wherein the first and the second probes are capable of forming the three-way junction structure of the invention by hybridization with the target nucleic acid molecule, and
iii) optionally, providing the sequences obtained or generated in step ii) as output.

In an embodiment, the target nucleic acid read in step i) is retrieved from a database. In another embodiment, the target nucleic acid read in step i) may be provided by the user.

In an embodiment, step ii) of method of the third aspect in turn comprises the steps of:
a) obtaining the foot region of the first probe by selecting a first portion of nucleotides comprised in the target nucleic acid and generating its complementary sequence,
b) obtaining the foot region of the second probe by selecting a second portion of nucleotides comprised in the target nucleic acid and generating its complementary sequence, wherein the first portion and the second portion are adjacent or substantially adjacent, preferably separated by 0 to 10, more preferably 0 to 6, nucleotides,
c) obtaining the arm region of the second probe by selecting a nucleotide sequence comprising the full length sequence of an DNA-directed RNA polymerase promoter and the full length reverse complement sequence of at least one fluorogenic aptamer, wherein the reverse complement sequence of at least one fluorogenic aptamer is operably linked to said DNA-directed RNA polymerase promoter, and
d) obtaining the arm region of the first probe by selecting between 5-9, preferably 6-8, nucleotides that are complementary to a region located at the 3' region of the arm region of the second probe obtained in step c).

In an embodiment, the output of step iii) is provided in a readable format, such as .txt or a html file.

Please note that the steps of the computer-implemented method may be performed in any order.

In a **fourth aspect,** the present invention further provides a kit of parts for detecting the presence of a target nucleic acid in a sample. In an embodiment, the kit may include one or more containers housing components for performing the method described herein and optionally instructions of uses. Specifically, such a kit may include one or more agents described herein (for example, a first and a second probe as described herein), optionally along with instructions describing the intended application and the proper use of these agents including instructions to design the first or the second probe, in case they are not provided as reagents in the kit. Preferably, the kit comprises one or multiple containers or vials comprising the first or second probes, preferably together with the necessary reagents to carry out the method of the second aspect, optionally also the reagents for performing a previous amplification step.

Thus, in an embodiment, the kit may comprise at least one of (i) a DNA-directed DNA polymerase, preferably *Bst* polymerase, (ii) at least one DNA-directed RNA polymerase, preferably T7 RNA polymerase, and/or (iii) at least a suitable fluorophore ligand of at least one fluorogenic aptamer to detect the presence of the target nucleotide. Preferably, the kit comprises all (i) to (iii). Preferably, the kit comprises a first and a second oligo suitable for carrying out the method of the second aspect and to form the structure of the first aspect, together with reagents (i) to (iii). Other elements that may be included in the kit are spermidine, dNTPs, and/or suitable buffers for the correct functioning of the method.

In an embodiment, the kit for detecting the presence of a target nucleic acid in a sample may comprise at least one, preferably all, of the following:
a) at least one DNA-directed DNA polymerase, preferably *Bst* polymerase,
b) at least one DNA-directed RNA polymerase, preferably T7 RNA polymerase,
c) at least a suitable fluorophore ligand of at least one fluorogenic aptamer,
d) spermidine,
e) deoxynucleoside triphosphates,
f) suitable buffers,
g) optionally, a retrotranscriptase, a RNAse and at least an exonuclease and a phosphatase (to perform the previous amplification step, preferably the NASBA assay)
wherein elements a) to g) are comprised in different containers or grouped in one or more containers.

Such kits may be designed to be suitable for diagnostic uses or for other purposes, for example, research uses. For example, the kit may contain apparatus for sample collection from a patient, and/or reagents for detecting diseases associated with nucleic acid molecules. Kits for research purposes may contain the components in appropriate concentrations or quantities for running various experiments.

In an embodiment, a computer-readable storage medium is provided, wherein said computer-readable storage contains instructions stored therein, in which when the instructions are executed by a computer, and are configured to enable a processor of the computer to perform the method of the third aspect for providing and/or designing the first and the second probes. In an embodiment, a device is provided, wherein the device is for providing and/or designing the first and second probes nucleic acid sequence data set, in which the device includes a computer processor and the computer-readable storage medium coupled to the computer processor. In an embodiment, a computer program is provided, wherein said computer program is stored in a computer-readable storage medium, in which when the program is executed by a computer, the program is configured to enable a processor of the computer to perform the method of the third aspect.

In a **fifth aspect,** the present invention further provides a method for detecting the presence of a target nucleic acid in a sample as defined in the second aspect of the present invention, wherein the method uses the kit of part as defined in the fourth aspect of the present invention.

In a **sixth aspect,** the present invention further provides a system for detecting the presence of a target nucleic acid in a sample, the system comprising a first probe and a second probe as defined in the first and/or second aspect of the present invention.

### SEQUENCE LISTING:

Sequence ID NO 1:
   *>* ***E.coli* 23S rRNA SMART-first probe** (DNA form from Wharam et al. Nucl Acids Res, 2001)
   Target-specific region underlined, complementary sequence to second probe in *italics* GCATTTAGCTACCGGGCAGTGCCATT*TTCGAAAT*
Sequence ID NO 2: *>* ***E.coli* 23S rRNA SMART-Mango second probe** (design based on Wharam *et al. Nucl Acids Res,* 2001)
   Mango Reverse Complement in lower-case letter, T7 promoter in **bold,** complementary sequence to first probe in *italics,* target-specific region underlined ggcacgtacgaatataccacataccaaaccttccttcgtacgtgccCCC**TATAGTGAGTCGTATTA**ATTT CGAA/iSp18/GGCATGACAACCCGAACACCAGTGAT/3AmMO/
Sequence ID NO 3:
   **> Mango-III (A10U) RNA aptamer** (45-nt, Trachman et al. Nat Chem Biol, 2019) Aptamer core in lower-case letter, nucleotides forming the stem structure underlined GGCACGUACGAAggaagguuuguaugugguauaUUCGUACGUGCC
Sequence ID NO 4:
   > **5' primer for *E.coli* 16S rRNA NASBA amplification reaction** (OV2, Kao et al. Anal Letters, 2010)
   GGAGGCAGCAGTGGGGAATA
Sequence ID NO 5:
   > 3' **primer for *E.coli* 16S rRNA NASBA amplification reaction** (OV3, Kao et al. Anal Letters, 2010)
   T7 promoter **in bold**
   AATTC**TAATACGACTCACTATA**GGGATTACCGCGGCTGCTGGCAC
Sequence ID NO 6:
   **> NR 024570.1 *Escherichia coli* strain U 5/41 16S ribosomal RNA,** partial sequence 
AGUUUGAUCAUGGCUCAGAUUGAACGCUGGCGGCAGGCCUAACACAUGCAAGU
CGAACGGUAACAGGAAGCAGCUUGCUGCUUUGCUGACGAGUGGCGGACGGGU
GAGUAAUGUCUGGGAAACUGCCUGAUGGAGGGGGAUAACUACUGGAAACGGUA
 
GCUAAUACCGCAUAACGUCGCAAGCACAAAGAGGGGGACCUUAGGGCCUCUUG
CCAUCGGAUGUGCCCAGAUGGGAUUAGCUAGUAGGUGGGGUAACGGCUCACC
UAGGCGACGAUCCCUAGCUGGUCUGAGAGGAUGACCAGCAACACUGGAACUGA
GACACGGUCCAGACUCCUACGGGAGGCAGCAGUGGGGAAUAUUGCACAAUGGG
CGCAAGCCUGAUGCAGCCAUGCNGCGUGUAUGAAGAAGGCCUUCGGGUUGUAA
AGUACUUUCAGCGGGGAGGAAGGGAGUAAAGUUAAUACCUUUGCUCAUUGACG
UUACCCGCAGAAGAAGCACCGGCUAACUCCGUGCCAGCAGCCGCGGUAAUACG
GAGGGUGCAAGCGUUAAUCGGAAUUACUGGGCGUAAAGCGCACGCAGGCGGU
UUGUUAAGUCAGAUGUGAAAUCCCCGGGCUCAACCUGGGAACUGCAUCUGAUA
CUGGCAAGCUUGAGUCUCGUAGAGGGGGGUAGAAUUCCAGGUGUAGCGGUGA
AAUGCGUAGAGAUCUGGAGGAAUACCGGUGGCGAAGGCGGCCCCCUGGACGAA
GACUGACGCUCAGGUGCGAAAGCGUGGGGAGCAAACAGGAUUAGAUACCCUGG
UAGUCCACGCCGUAAACGAUGUCGACUUGGAGGUUGUGCCCUUGAGGCGUGG
CUUCCGGANNUAACGCGUUAAGUCGACCGCCUGGGGAGUACGGCCGCAAGGU
UAAAACUCAAAUGAAUUGACGGGGGCCGCACAAGCGGUGGAGCAUGUGGUUUA
AUUCGAUGCAACGCGAAGAACCUUACCUGGUCUUGACAUCCACGGAAGUUUUC
AGAGAUGAGAAUGUGCCUUCGGGAACCGUGAGACAGGUGCUGCAUGGCUGUC
GUCAGCUCGUGUUGUGAAAUGUUGGGUUAAGUCCCGCAACGAGCGCAACCCUU
AUCCUUUGUUGCCAGCGGUCCGGCCGGGAACUCAAAGGAGACUGCCAGUGAUA
AACUGGAGGAAGGUGGGGAUGACGUCAAGUCAUCAUGGCCCUUACGACCAGGG
CUACACACGUGCUACAAUGGCGCAUACAAAGAGAAGCGACCUCGCGAGAGCAA
GCGGACCUCAUAAAGUGCGUCGUAGUCCGGAUUGGAGUCUGCAACUCGACUCC
AUGAAGUCGGAAUCGCUAGUAAUCGUGGAUCAGAAUGCCACGGUGAAUACGUU
CCCGGGCCUUGUACACACCGCCCGUCACACCAUGGGAGUGGGUUGCAAAAGAA
GUAGGUAGCUUAACUUCGGGAGGGCG
Sequence ID NO 7:
   *>* ***E.coli* 16S rRNA SMART-first probe** (29-nt hybridization)
   TGATGCAGCCATGCGGCGTGTATGAAGAA*TTCGAAAT*
Sequence ID NO 8:
   *>* ***E.coli* 16S rRNA SMART-Mango second probe** (29-nt hybridization)
   Mango Reverse Complement in lower-case letter, T7 promoter in **bold,** complementary sequence to first probe in *italics,* target-specific region underlined ggcacgtacgaatataccacataccaaaccttccttcgtacgtgccCCC**TATAGTGAGTCGTATTA***ATTT CGAA*/iSp18/GGCCTTCGGGTTGTAAAGTACTTTCAGCG/3AmMO/
Sequence ID NO 9:
   **> Reverse Complement RNA sequence of *E.coli* 16S, NASBA product** (191-nt)
   3' NASBA-primer used in *italics,* target-region recognized by the second-probe in lower-case letter, target-region recognized by the first-probe in **bold ,** RC of 5' NASBA-primer used underlined 
GGGAUUACCGCGGCUGCUGGCACGGAGUUAGCCGGUGCUUCUUCUGCGGGUA
ACGUCAAUGAGCAAAGGUAUUAACUUUACUCCCUUCCUCCCcgcugaaaguacuuuac
aacccgaaggccUUCUUCAUACACGCCGCAUGGCUGCAUCAGGCUUGCGCCCAUU
GUGCAAUAUUCCCCACUGCUGCCUCC
Sequence ID NO 10:
   ***> E.coli* 16S rRNA SMART-first probe, 2-complementary (3WJ) nucleotides**
   Target-specific region underlined, complementary sequence to second probe in *italics* TGATGCAGCCATGCGGCGTGTATGAAGAATT
Sequence ID NO 11:
   *>* ***E.coli* 16S rRNA SMART-first probe, 4-complementary (3WJ) nucleotides**
   Target-specific region underlined, complementary sequence to second probe in *italics* TGATGCAGCCATGCGGCGTGTATGAAGAA*TTCG*
Sequence ID NO 12:
   *>* ***E.coli* 16S rRNA SMART-first probe, 6-complementary (3WJ) nucleotides**
   Target-specific region underlined, complementary sequence to second probe in *italics* TGATGCAGCCATGCGGCGTGTATGAAGAA*TTCGAA*
Sequence ID NO 13:
   > **Synthetic RNA target of *E.coli* 16S ribosomal RNA** (75-nt, pos. 357-432, reverse complement)
   Target-region recognized by the second-probe in lower-case letter, target-region recognized by the first-probe in **bold** 
ugaaaguacuuuacaacccgaaggccUUCUUCAUACACGCGGCAUGGCUGCAUCAGGCU
UGCGCCCAUUGUGCAA
Sequence ID NO 14:
   *>* ***E.coli* 16S rRNA SMART-first probe, 10-complementary (3WJ) nucleotides**
   Target-specific region underlined, complementary sequence to second probe in *italics* TGATGCAGCCATGCGGCGTGTATGAAGAA*TTCGAAATTA*
Sequence ID NO 15:
   ***> E.coli* 16S rRNA SMART-first probe, 15-complementary (3WJ) nucleotides**
   Target-specific region underlined, complementary sequence to second probe in *italics* TGATGCAGCCATGCGGCGTGTATGAAGAA*TTCGAAATTAATACG*
Sequence ID NO 16:
   ***> E.coli* 16S rRNA SMART-first probe, 1-nt of separation from the second-probe hybridization region**
   Target-specific region underlined, complementary sequence to second probe in *italics* TGATGCAGCCATGCGGCGTGTATGAAGA*TTCGAAAT*
Sequence ID NO 17:
   *>* ***E.coli* 16S rRNA SMART-first probe, 2-nt of separation from the second-probe hybridization region**
   Target-specific region underlined, complementary sequence to second probe in *italics* TGATGCAGCCATGCGGCGTGTATGAAG *TTCGAAAT*
Sequence ID NO 18:
   *>* ***E.coli* 16S rRNA SMART-first probe, 4-nt of separation from the second-probe hybridization region**
   Target-specific region underlined, complementary sequence to second probe in *italics* TGATGCAGCCATGCGGCGTGTATGA*TTCGAAAT*
Sequence ID NO 19:
   *>* ***E.coli* 16S rRNA SMART-first probe, 6-nt of separation from the second-probe hybridization region**
   Target-specific region underlined, complementary sequence to second probe in *italics* TGATGCAGCCATGCGGCGTGTAT*TTCGAAAT*
Sequence ID NO 20:
   *>* ***E.coli* 16S rRNA SMART-first probe** (22-nt hybridization)
   Target-specific region underlined, complementary sequence to second probe in *italics* GCCATGCGGCGTGTATGAAGAA*TTCGAAAT*
Sequence ID NO 21:
   *>* ***E.coli* 16S rRNA SMART-first probe** (15-nt hybridization)
   Target-specific region underlined, complementary sequence to second probe in *italics* GGCGTGTATGAAGAA*TTCGAAAT*
Sequence ID NO 22:
   *>* ***E.coli* 16S rRNA SMART-first probe** (7-nt hybridization)
   Target-specific region underlined, complementary sequence to second probe in *italics* TGAAGAA*TTCGAAAT*
Sequence ID NO 23:
   > ***E.coli* 16S rRNA SMART-Mango second probe** (22-nt hybridization) Mango Reverse Complement in lower-case letter, T7 promoter in **bold**, complementary sequence to first probe in *italics,* target-specific region underlined 
ggcacgtacgaatataccacataccaaaccttccttcgtacgtgccCCCTATAGTGAGTCGTATTAATTT
CGAA/iSp18/GGCCTTCGGGTTGTAAAGTACT/3AmMO/
Sequence ID NO 24:
   > ***E.coli* 16S rRNA SMART-Mango second probe** (15-nt hybridization) Mango Reverse Complement in lower-case letter, T7 promoter in **bold**, complementary sequence to first probe in *italics,* target-specific region underlined 
ggcacgtacgaatataccacataccaaaccttccttcgtacgtgccCCCTATAGTGAGTCGTATTAATTT
CGAA/iSp18/GGCCTTCGGGTTGTA/3AmMO/
Sequence ID NO 25:
   > ***E.coli* 16S rRNA SMART-Mango second probe** (7-nt hybridization) Mango Reverse Complement in lower-case letter, T7 promoter in **bold**, complementary sequence to first probe in *italics,* target-specific region underlined 
ggcacgtacgaatataccacataccaaaccttccttcgtacgtgccCCCTATAGTGAGTCGTATTAATTT
CGAA/iSp18/GGCCTTC/3AmMO/
Sequence ID NO 26:
   > **SARS-CoV-2 T3 SMART-first probe**
   Target-specific region underlined, complementary sequence to second probe in *italics* TTGGTTCCATGCTATACATGTCTCTGGGAC*TTCGAAAT*
Sequence ID NO 27:
   > **SARS-CoV-2 T3 SMART-Mango second probe**
   Mango Reverse Complement in lower-case letter, T7 promoter in **bold**, complementary sequence to first probe in *italics,* target-specific region underlined 
ggcacgtacgaatataccacataccaaaccttccttcgtacgtgccCCCTATAGTGAGTCGTATTAATTT
CGAA/iSp18/CAATGGTACTAAGAGGTTTGATAACCCTGT/3AmMO/
Sequence ID NO 28:

   > **Synthetic RNA target of SARS-CoV-2 S *'spike'* RNA** (30-nt, pos. 21766-21795, reverse complement)
   Target-region recognized by the second-probe in lower-case letter, target-region recognized by the first-probe in **bold**
   cucuuaguaccauug**GUCCCAGAGACAUGU**
Sequence ID NO 29:
   > **Reverse Complement RNA sequence of SARS-CoV-2 product of NASBA** (189-nt, *S 'spike'* RNA)
   3' NASBA-primer used in *italics,* target-region recognized by the second-probe in lower-case letter, target-region recognized by the first-probe in **bold**, RC of 5' NASBA-primer used underlined 
CAGUGGAAGCAAAAUAAACACCAUCAUUAAAUGGUAGGACAGGGUUAUCAAACc
ucuuaguaccauugGUCCCAGAGACAUGUAUAGCAUGGAACCAAGUAACAUUGGAAA
AGAAAGGUAAGAACAAGUCCUGAGUUGAAUGUAAAACUGAGGAUCUGAAAACUU
UGUCAGGGUAAUAAACACCACGUG
Sequence ID NO 30:
   > **SARS-CoV-2 T3 SMART- second probe for Molecular Beacon detection** Molecular Beacon Reverse Complement in lower-case letter, T7 promoter in bold, complementary sequence to first probe in *italics,* target-specific region underlined 
tgaagaaggccttcgggttgtaaagCCCTATAGTGAGTCGTATTAATTTCGAA/iSp18/CAATGG
TACTAAGAGGTTTGATAACCCTGT/3AmMO/
Sequence ID NO 31:
   > **Molecular Beacon Oligonucleotide probe** (Grm neg 16S MB, Zhao *et al. J Clin Microbiol*, 2009)
   Complementary 5' and 3' ends underlined; 56-FAM = 5'-6-FAM; 3DAb = 3'-Dabcyl. /56-FAM/CGAGCTTGAAGAAGGCCTTCGGGTTGTAAAGAGCTCG/3DAb/
Sequence ID NO 32:

   > ***S 'spike'* surface glycoprotein RNA** (CDS); NC_045512.2:21563-25384 Severe acute respiratory syndrome coronavirus 2 isolate Wuhan-Hu-1 (SARS-CoV-2), complete genome 
AUGUUUGUUUUUCUUGUUUUAUUGCCACUAGUCUCUAGUCAGUGUGUUAAUCU
UACAACCAGAACUCAAUUACCCCCUGCAUACACUAAUUCUUUCACACGUGGUGU
UUAUUACCCUGACAAAGUUUUCAGAUCCUCAGUUUUACAUUCAACUCAGGACUU
GUUCUUACCUUUCUUUUCCAAUGUUACUUGGUUCCAUGCUAUACAUGUCUCUG
GGACCAAUGGUACUAAGAGGUUUGAUAACCCUGUCCUACCAUUUAAUGAUGGU
GUUUAUUUUGCUUCCACUGAGAAGUCUAACAUAAUAAGAGGCUGGAUUUUUGG
UACUACUUUAGAUUCGAAGACCCAGUCCCUACUUAUUGUUAAUAACGCUACUAA
UGUUGUUAUUAAAGUCUGUGAAUUUCAAUUUUGUAAUGAUCCAUUUUUGGGUG
UUUAUUACCACAAAAACAACAAAAGUUGGAUGGAAAGUGAGUUCAGAGUUUAUU
CUAGUGCGAAUAAUUGCACUUUUGAAUAUGUCUCUCAGCCUUUUCUUAUGGAC
CUUGAAGGAAAACAGGGUAAUUUCAAAAAUCUUAGGGAAUUUGUGUUUAAGAAU
AUUGAUGGUUAUUUUAAAAUAUAUUCUAAGCACACGCCUAUUAAUUUAGUGCGU
GAUCUCCCUCAGGGUUUUUCGGCUUUAGAACCAUUGGUAGAUUUGCCAAUAGG
UAUUAACAUCACUAGGUUUCAAACUUUACUUGCUUUACAUAGAAGUUAUUUGAC
UCCUGGUGAUUCUUCUUCAGGUUGGACAGCUGGUGCUGCAGCUUAUUAUGUG
GGUUAUCUUCAACCUAGGACUUUUCUAUUAAAAUAUAAUGAAAAUGGAACCAUU
ACAGAUGCUGUAGACUGUGCACUUGACCCUCUCUCAGAAACAAAGUGUACGUU
GAAAUCCUUCACUGUAGAAAAAGGAAUCUAUCAAACUUCUAACUUUAGAGUCCA
ACCAACAGAAUCUAUUGUUAGAUUUCCUAAUAUUACAAACUUGUGCCCUUUUGG
UGAAGUUUUUAACGCCACCAGAUUUGCAUCUGUUUAUGCUUGGAACAGGAAGA
GAAUCAGCAACUGUGUUGCUGAUUAUUCUGUCCUAUAUAAUUCCGCAUCAUUU
UCCACUUUUAAGUGUUAUGGAGUGUCUCCUACUAAAUUAAAUGAUCUCUGCUU
UACUAAUGUCUAUGCAGAUUCAUUUGUAAUUAGAGGUGAUGAAGUCAGACAAAU
CGCUCCAGGGCAAACUGGAAAGAUUGCUGAUUAUAAUUAUAAAUUACCAGAUGA
UUUUACAGGCUGCGUUAUAGCUUGGAAUUCUAACAAUCUUGAUUCUAAGGUUG
GUGGUAAUUAUAAUUACCUGUAUAGAUUGUUUAGGAAGUCUAAUCUCAAACCUU
UUGAGAGAGAUAUUUCAACUGAAAUCUAUCAGGCCGGUAGCACACCUUGUAAU
GGUGUUGAAGGUUUUAAUUGUUACUUUCCUUUACAAUCAUAUGGUUUCCAACC
CACUAAUGGUGUUGGUUACCAACCAUACAGAGUAGUAGUACUUUCUUUUGAAC
UUCUACAUGCACCAGCAACUGUUUGUGGACCUAAAAAGUCUACUAAUUUGGUUA
AAAACAAAUGUGUCAAUUUCAACUUCAAUGGUUUAACAGGCACAGGUGUUCUUA
CUGAGUCUAACAAAAAGUUUCUGCCUUUCCAACAAUUUGGCAGAGACAUUGCU
GACACUACUGAUGCUGUCCGUGAUCCACAGACACUUGAGAUUCUUGACAUUAC
ACCAUGUUCUUUUGGUGGUGUCAGUGUUAUAACACCAGGAACAAAUACUUCUA
 
ACCAGGUUGCUGUUCUUUAUCAGGAUGUUAACUGCACAGAAGUCCCUGUUGCU
AUUCAUGCAGAUCAACUUACUCCUACUUGGCGUGUUUAUUCUACAGGUUCUAA
UGUUUUUCAAACACGUGCAGGCUGUUUAAUAGGGGCUGAACAUGUCAACAACU
CAUAUGAGUGUGACAUACCCAUUGGUGCAGGUAUAUGCGCUAGUUAUCAGACU
CAGACUAAUUCUCCUCGGCGGGCACGUAGUGUAGCUAGUCAAUCCAUCAUUGC
CUACACUAUGUCACUUGGUGCAGAAAAUUCAGUUGCUUACUCUAAUAACUCUAU
UGCCAUACCCACAAAUUUUACUAUUAGUGUUACCACAGAAAUUCUACCAGUGUC
UAUGACCAAGACAUCAGUAGAUUGUACAAUGUACAUUUGUGGUGAUUCAACUGA
AUGCAGCAAUCUUUUGUUGCAAUAUGGCAGUUUUUGUACACAAUUAAACCGUG
CUUUAACUGGAAUAGCUGUUGAACAAGACAAAAACACCCAAGAAGUUUUUGCAC
AAGUCAAACAAAUUUACAAAACACCACCAAUUAAAGAUUUUGGUGGUUUUAAUU
UUUCACAAAUAUUACCAGAUCCAUCAAAACCAAGCAAGAGGUCAUUUAUUGAAG
AUCUACUUUUCAACAAAGUGACACUUGCAGAUGCUGGCUUCAUCAAACAAUAUG
GUGAUUGCCUUGGUGAUAUUGCUGCUAGAGACCUCAUUUGUGCACAAAAGUUU
AACGGCCUUACUGUUUUGCCACCUUUGCUCACAGAUGAAAUGAUUGCUCAAUA
CACUUCUGCACUGUUAGCGGGUACAAUCACUUCUGGUUGGACCUUUGGUGCAG
GUGCUGCAUUACAAAUACCAUUUGCUAUGCAAAUGGCUUAUAGGUUUAAUGGU
AUUGGAGUUACACAGAAUGUUCUCUAUGAGAACCAAAAAUUGAUUGCCAACCAA
UUUAAUAGUGCUAUUGGCAAAAUUCAAGACUCACUUUCUUCCACAGCAAGUGCA
CUUGGAAAACUUCAAGAUGUGGUCAACCAAAAUGCACAAGCUUUAAACACGCUU
GUUAAACAACUUAGCUCCAAUUUUGGUGCAAUUUCAAGUGUUUUAAAUGAUAUC
CUUUCACGUCUUGACAAAGUUGAGGCUGAAGUGCAAAUUGAUAGGUUGAUCAC
AGGCAGACUUCAAAGUUUGCAGACAUAUGUGACUCAACAAUUAAUUAGAGCUGC
AGAAAUCAGAGCUUCUGCUAAUCUUGCUGCUACUAAAAUGUCAGAGUGUGUAC
UUGGACAAUCAAAAAGAGUUGAUUUUUGUGGAAAGGGCUAUCAUCUUAUGUCC
UUCCCUCAGUCAGCACCUCAUGGUGUAGUCUUCUUGCAUGUGACUUAUGUCCC
UGCACAAGAAAAGAACUUCACAACUGCUCCUGCCAUUUGUCAUGAUGGAAAAGC
ACACUUUCCUCGUGAAGGUGUCUUUGUUUCAAAUGGCACACACUGGUUUGUAA
CACAAAGGAAUUUUUAUGAACCACAAAUCAUUACUACAGACAACACAUUUGUGU
CUGGUAACUGUGAUGUUGUAAUAGGAAUUGUCAACAACACAGUUUAUGAUCCU
UUGCAACCUGAAUUAGACUCAUUCAAGGAGGAGUUAGAUAAAUAUUUUAAGAAU
CAUACAUCACCAGAUGUUGAUUUAGGUGACAUCUCUGGCAUUAAUGCUUCAGU
UGUAAACAUUCAAAAAGAAAUUGACCGCCUCAAUGAGGUUGCCAAGAAUUUAAA
UGAAUCUCUCAUCGAUCUCCAAGAACUUGGAAAGUAUGAGCAGUAUAUAAAAUG
GCCAUGGUACAUUUGGCUAGGUUUUAUAGCUGGCUUGAUUGCCAUAGUAAUGG
UGACAAUUAUGCUUUGCUGUAUGACCAGUUGCUGUAGUUGUCUCAAGGGCUGU
 
UGUUCUUGUGGAUCCUGCUGCAAAUUUGAUGAAGACGACUCUGAGCCAGUGCU
CAAAGGAGUCAAAUUACAUUACACAUAA
Sequence ID NO 33:
   > **5' primer for SARS-CoV-2 NASBA amplification reaction**
   CACGTGGTGTTTATTACCCTGACA
Sequence ID NO 34:
   > **3' primer for SARS-CoV-2 NASBA amplification reaction**
   T7 promoter **in bold**
   AATTC**TAATACGACTCACTATA***GGGA*CAGTGGAAGCAAAATAAACACCA
Sequence ID NO 35:
   > ***E.coli* 23S rRNA SMART-first probe**
   Target-specific region underlined, complementary sequence to second probe in *italics* TCTGAATGGAAGGACCATCGCTCAACGGA*TTCGAAAT*
Sequence ID NO 36:
   > ***E.coli* 23S rRNA SMART-Mango second probe**
   Mango Reverse Complement in lower-case letter, T7 promoter in **bold,** complementary sequence to first probe in *italics,* target-specific region underlined 
ggcacgtacgaatataccacataccaaaccttccttcgtacgtgccCCCTATAGTGAGTCGTATTAATTT
CGAA/iSp18/TAAAAGGTACTCCGGGGATAACAGGCTGA/3AmMO/
Sequence ID NO 37:
   > **Reverse Complement RNA sequence of *E.coli* 23S rRNA, NASBA product** (256-nt)
   3' NASBA-primer used in *italics,* target-region recognized by the second-probe in lower-case letter, target-region recognized by the first-probe in **bold**, RC of 5' NASBA-primer used underlined 
GGGACAGCUCGCGUACCACUUUAAAUGGCGAACAGCCAUACCCUUGGGACCUA
CUUCAGCCCCAGGAUGUGAUGAGCCGACAUCGAGGUGCCAAACACCGCCGUCG
AUAUGAACUCUUGGGCGGUAucagccuguuauccccggaguaccuuuuaUCCGUUGAGCG
AUGGUCCUUCCAUUCAGAACCACCGGAUCACUAUGACCUGCUUUCGCACCUGC
UCGCGCCGUCACGCUCGCAGUCAAGCUGGCUUAUGCC
Sequence ID NO 38:
   **> NR_103073.1 *Escherichia coli* strain K-12 23S ribosomal RNA gene**, complete sequence 
GGUUAAGCGACUAAGCGUACACGGUGGAUGCCCUGGCAGUCAGAGGCGAUGAA
GGACGUGCUAAUCUGCGAUAAGCGUCGGUAAGGUGAUAUGAACCGUUAUAACC
GGCGAUUUCCGAAUGGGGAAACCCAGUGUGUUUCGACACACUAUCAUUAACUG
AAUCCAUAGGUUAAUGAGGCGAACCGGGGGAACUGAAACAUCUAAGUACCCCG
AGGAAAAGAAAUCAACCGAGAUUCCCCCAGUAGCGGCGAGCGAACGGGGAGGA
GCCCAGAGCCUGAAUCAGUGUGUGUGUUAGUGGAAGCGUCUGGAAAGGCGCG
CGAUACAGGGUGACAGCCCCGUACACAAAAAUGCACAUGCUGUGAGCUCGAUG
AGUAGGGCGGGACACGUGGUAUCCUGUCUGAAUAUGGGGGGACCAUCCUCCAA
GGCUAAAUACUCCUGACUGACCGAUAGUGAACCAGUACCGUGAGGGAAAGGCG
AAAAGAACCCCGGCGAGGGGAGUGAAAAAGAACCUGAAACCGUGUACGUACAA
GCAGUGGGAGCAUGCUUAGGCGUGUGACUGCGUACCUUUUGUAUAAUGGGUC
AGCGACUUAUAUUCUGUAGCAAGGUUAACCGAAUAGGGGAGCCGAAGGGAAAC
CGAGUCUUAACUGGGCGUUAAGUUGCAGGGUAUAGACCCGAAACCCGGUGAUC
UAGCCAUGGGCAGGUUGAAGGUUGGGUAACACUAACUGGAGGACCGAACCGAC
UAAUGUUGAAAAAUUAGCGGAUGACUUGUGGCUGGGGGUGAAAGGCCAAUCAA
ACCGGGAGAUAGCUGGUUCUCCCCGAAAGCUAUUUAGGUAGCGCCUCGUGAAC
UCAUCUCCGGGGGUAGAGCACUGUUUCGGCAAGGGGGUCAUCCCGACUUACCA
ACCCGAUGCAAACUGCGAAUACCGGAGAAUGUUAUCACGGGAGACACACGGCG
GGUGCUAACGUCCGUCGUGAAGAGGGAAACAACCCAGACCGCCAGCUAAGGUC
CCAAAGUCAUGGUUAAGUGGGAAACGAUGUGGGAAGGCCCAGACAGCCAGGAU
GUUGGCUUAGAAGCAGCCAUCAUUUAAAGAAAGCGUAAUAGCUCACUGGUCGA
GUCGGCCUGCGCGGAAGAUGUAACGGGGCUAAACCAUGCACCGAAGCUGCGG
CAGCGACGCUUAUGCGUUGUUGGGUAGGGGAGCGUUCUGUAAGCCUGUGAAG
GUGUACUGUGAGGUAUGCUGGAGGUAUCAGAAGUGCGAAUGCUGACAUAAGUA
ACGAUAAAGCGGGUGAAAAGCCCGCUCGCCGGAAGACCAAGGGUUCCUGUCCA
ACGUUAAUCGGGGCAGGGUGAGUCGACCCCUAAGGCGAGGCCGAAAGGCGUA
GUCGAUGGGAAACAGGUUAAUAUUCCUGUACUUGGUGUUACUGCGAAGGGGG
GACGGAGAAGGCUAUGUUGGCCGGGCGACGGUUGUCCCGGUUUAAGCGUGUA
GGCUGGUUUUCCAGGCAAAUCCGGAAAAUCAAGGCUGAGGCGUGAUGACGAGG
CACUACGGUGCUGAAGCAACAAAUGCCCUGCUUCCAGGAAAAGCCUCUAAGCA
UCAGGUAACAUCAAAUCGUACCCCAAACCGACACAGGUGGUCAGGUAGAGAAUA
CCAAGGCGCUUGAGAGAACUCGGGUGAAGGAACUAGGCAAAAUGGUGCCGUAA
CUUCGGGAGAAGGCACGCUGAUAUGUAGGUGAAGCGACUUGCUCGUGGAGCU
GAAAUCAGUCGAAGAUACCAGCUGGCUGCAACUGUUUAUUAAAAACACAGCACU
GUGCAAACACGAAAGUGGACGUAUACGGUGUGACGCCUGCCCGGUGCCGGAAG
 
GUUAAUUGAUGGGGUUAGCGCAAGCGAAGCUCUUGAUCGAAGCCCCGGUAAAC
GGCGGCCGUAACUAUAACGGUCCUAAGGUAGCGAAAUUCCUUGUCGGGUAAGU
UCCGACCUGCACGAAUGGCGUAAUGAUGGCCAGGCUGUCUCCACCCGAGACUC
AGUGAAAUUGAACUCGCUGUGAAGAUGCAGUGUACCCGCGGCAAGACGGAAAG
ACCCCGUGAACCUUUACUAUAGCUUGACACUGAACAUUGAGCCUUGAUGUGUA
GGAUAGGUGGGAGGCUUUGAAGUGUGGACGCCAGUCUGCAUGGAGCCGACCU
UGAAAUACCACCCUUUAAUGUUUGAUGUUCUAACGUUGACCCGUAAUCCGGGU
UGCGGACAGUGUCUGGUGGGUAGUUUGACUGGGGCGGUCUCCUCCUAAAGAG
UAACGGAGGAGCACGAAGGUUGGCUAAUCCUGGUCGGACAUCAGGAGGUUAGU
GCAAUGGCAUAAGCCAGCUUGACUGCGAGCGUGACGGCGCGAGCAGGUGCGA
AAGCAGGUCAUAGUGAUCCGGUGGUUCUGAAUGGAAGGGCCAUCGCUCAACGG
AUAAAAGGUACUCCGGGGAUAACAGGCUGAUACCGCCCAAGAGUUCAUAUCGA
CGGCGGUGUUUGGCACCUCGAUGUCGGCUCAUCACAUCCUGGGGCUGAAGUA
GGUCCCAAGGGUAUGGCUGUUCGCCAUUUAAAGUGGUACGCGAGCUGGGUUU
AGAACGUCGUGAGACAGUUCGGUCCCUAUCUGCCGUGGGCGCUGGAGAACUG
AGGGGGGCUGCUCCUAGUACGAGAGGACCGGAGUGGACGCAUCACUGGUGUU
CGGGUUGUCAUGCCAAUGGCACUGCCCGGUAGCUAAAUGCGGAAGAGAUAAGU
GCUGAAAGCAUCUAAGCACGAAACUUGCCCCGAGAUGAGUUCUCCCUGACUCC
UUGAGAGUCCUGAAGGAACGUUGAAGACGACGACGUUGAUAGGCCGGGUGUG
UAAGCGCAGCGAUGCGUUGAGCUAACCGGUACUAAUGAACCGUGAGGCUUAAC
CUU
Sequence ID NO 39:
   > **Synthetic RNA target of 23S ribosomal RNA** (79-nt, pos. 2393-2468, reverse complement)
   Target-region recognized by the second-probe in lower-case letter, target-region recognized by the first-probe in bold 
GGGAGGGCGGUAucagccuguuauccccggaguaccuuuuaUCCGUUGAGCGAUGGUCC
UUCCAUUCAGAACCACCGGA
Sequence ID NO 40:
   > ***S.aureus mecA* SMART-first probe**
   Target-specific region underlined, complementary sequence to second probe in *italics* AGGTGAAATACTGATTAACCCAGTACAGAT*TTCGAAAT*
Sequence ID NO 41:
   > ***S.aureus mecA* SMART-Mango second probe**
   Mango Reverse Complement in lower-case letter, T7 promoter in **bold**, complementary sequence to first probe in *italics,* target-specific region underlined 
ggcacgtacgaatataccacataccaaaccttccttcgtacgtgccCCCTATAGTGAGTCGTATTAATTT
CGAA/iSp18/CCTTTCAATCTATAGCGCATTAGAAAATAA/3AmMO/
Sequence ID NO 42:
   **> Reverse Complement RNA sequence of *S.aureus mecA* gene, NASBA product** (98-nt)
   3' NASBA-primer used in *italics,* target-region recognized by the second-probe in lower-case letter, target-region recognized by the first-probe in **bold**, RC of 5' NASBA-primer used underlined 
GGGAAGUGAGGUGCGUUAAUAUUGCCAuuauuuucuaaugcgcuauagauugaaaggAUC
UGUACUGGGUUAAUCAGUAUUUCACCUUGUCCGUAACC
Sequence ID NO 43:
   **>KX639010.1 *Staphylococcus aureus* strain SQ5 methicillin resistance protein subunit A (*mecA*) gene,** partial cds 
TGTAAAGGTTGGCAAAAAGATAAATCTTGGGGTGGTTACAACGTTACAAGATATGA
AGTGGTAAATGGTAATATCGACTTAAAACAAGGGATAGAATCATCAGATAACATTT
TCTTTGCTAGAGTAGCACTCGAATTAGGCAGTAAGAAATTTGAAAAAGGCATGAAA
AAACTAGGTGTTGGTGAAGATATACCAAGCCATTATCCATTTTATAGGGCTCAAAT
TTCAAACAAAAATTTAGATAATGAAATATTATTAGCTGATTCAGGTTACGGACAAGG
TGAAATACTGATTAACCCAGTACAGATCCTTTCAATCTATAGCGCATTAGAAGGTA
ATGGCAATATTAACGCACCTCACTTATTAAAAGACACGAAAAACAAAGTTTGGAAG
AAAAATATTATTTCCAAAGAAAATATCAATCTATTAACTGATGGTATGCAACAAGTC
GTAAATAAAACACATAAAGAAGATA
Sequence ID NO 44:
   **> Synthetic RNA target of *S.aureus mecA* RNA** (98-nt, pos. 268-362, reverse complement)
   Target-region recognized by the second-probe in lower-case letter, target-region recognized by the first-probe in **bold**, RC of 5' NASBA-primer used underlined 
GGGAAGUGAGGUGCGUUAAUAUUGCCAuuauuuucuaaugcgcuauagauugaaaggAUC
UGUACUGGGUUAAUCAGUAUUUCACCUUGUCCGUAACC
Sequence ID NO 45:
   > **Influenza A *PB1* gene SMART-first probe** (15-nt target hybridization region)
   Target-specific region underlined, complementary sequence to second probe in *italics* GGCTGTATGGAGGAT*TTCGAAAT*
Sequence ID 46:
   > **Influenza B *PA* gene SMART-first probe** (15-nt target hybridization region) Target-specific region underlined, complementary sequence to second probe in *italics* CCATCCATGCTATGG*TTCGAAAT*
Sequence ID NO 47:
   > **Influenza A *PB1* gene SMART-second probe** (15-nt target hybridization region) Mango Reverse Complement in lower-case letter, T7 promoter in **bold**, complementary sequence to first probe in *italics,* target-specific region underlined 
ggcacgtacgaatataccacataccaaaccttccttcgtacgtgccCCCTATAGTGAGTCGTATTAATTT
CGAA/iSp18/CTCCAGTATAAGGGA/3AmMO/
Sequence ID NO 48:
   > **Influenza B *PA* gene SMART-second probe** (15-nt target hybridization region) Mango Reverse Complement in lower-case letter, T7 promoter in **bold**, complementary sequence to first probe in *italics,* target-specific region underlined 
ggcacgtacgaatataccacataccaaaccttccttcgtacgtgccCCCTATAGTGAGTCGTATTAATTT
CGAA/iSp18/TTCTTGGCATTCCCT/3AmMO/
Sequence ID NO 49:
   **> Synthetic RNA target of Influenza A *PB1* gene** (30-nt)
   Target-region recognized by the second-probe in lower-case letter, target-region recognized by the first-probe in **bold**
   ucccuuauacuggaga**UCCUCCAUACAGCC**
Sequence ID NO 50:
   > **Synthetic RNA target of Influenza B *PA* gene** (30-nt)
   Target-region recognized by the second-probe in lower-case letter, target-region recognized by the first-probe in **bold**
   agggaaugccaagaac**CAUAGCAUGGAUGG**
Sequence ID NO 51:
   **>MK185298.1 Influenza A virus (A/swine/South Dakota/A02016893/2018(H1N1)) segment 2 polymerase PB1 (PB1) and PB1-F2 protein (PB1-F2) genes, complete cds** 
ATGGATGTCAATCCGACTCTACTTTTCCTAAAGGTTCCAGCGCAAAATGCCATAAG
CACCACATTCCCTTATACTGGAGATCCTCCATACAGCCATGGAACAGGAACAGGA
TACACTATGGACACAGTCAACAGAACACACCAATATTCAGAAAAAGGGAAATGGA
CAACAAACACAGAAACTGGGGCACCCCAGCTCAACCCGATTGATGGACCACTACC
TGAAGATAATGAACCAAGTGGATATGCACAAACAGACTGTGTCCTGGAGGCCATG
GCTTTCCTTGAGGAATCCCACCCAGGGATATTTGAGAATTCATGCCTTGAAACAAT
GGAAATTGTCCAACAAACAAGGGTGGATAAATTAACTCAAGGTCGTCAGACTTATG
ATTGGACATTGAACAGAAACCAGCCGGCAGCAACTGCATTGGCCAACACCATAGA
AGTTTTTAGATCAAACGGGCTAACGGCTAATGAGTCAGGAAGGCTAATAGATTTCC
TAAAAGATGTAATGGAATCAATGGATAAAGAGGAAATAGAGATAACAACACACTTT
CAAAGAAAAAGGAGAGTAAGAGACAACATGACCAAAAAGATGGTAACACAAAGAA
CAATAGGGAAGAAAAAACAAAGAATGAGTAAGAAAAGTTATCTAATAAGGGCACTA
ACATTAAATACGATGACCAAAGATGCAGAGAGAGGCAAGTTAAAAAGGAGGGCTA
TCGCAACACCTGGGATGCAAATTAGAGGGTTCGTGTACTTTGTTGAAACTTTAGCT
AGAAGCATTTGCGAAAAGCTTGAACAGTCCGGACTCCCAGTAGGGGGCAATGAAA
AGAAAGCCAAATTGGCAAATGTTGTGAGAAAGATGATGACTAATTCACAAGACACA
GAGCTTTCTTTCACAATCACTGGAGACAACACTAAGTGGAATGAGAACCAAAATCC
CCGAATGTTCCTGGCAATGATTACATACATCACCAGAAATCAACCCGAGTGGTTCA
GAAACATACTGAGCATGGCACCAATAATGTTCTCAAACAAAATGGCAAGACTAGGA
AAAGGGTACATGTTCGAGAGTAAAAGAATGAAGCTTCGAACACAAGTACCAGCAG
AAATGTTGGCAAGCATTGACCTGAAGTATTTTAATGAATCAACACGGAAGAAAATC
GAAAAAATAAGGCCTCTCCTAATAGATGGCACAGCATCATTGAGCCCTGGGATGA
TGATGGGCATGTTCAACATGCTAAGTACGGTTTTGGGAGTCTCAATATTGAATCTT
GGACAGAAGAAATACACCAGGACAACATATTGGTGGGATGGACTCCAATCCTCCG
ACGATTTTGCCCTCATAGTAAATGCACCAAACCATGAGGGAATACAAGCAGGAGT
GGATAGATTCTACAGGACCTGCAAGTTAGTAGGGATCAACATGAGCAAGAAGAAG
TCCTACATAAATAGGACTGGAACATTTGAATTCACAAGCTTTTTTTATCGCTATGGG
TTTGTAGCTAATTTTAGCATGGAGCTGCCCAGTTTTGGAGTGTCTGGAATAAACGA
ATCAGCTGACATGAGTATCGGGGTAACAGTTATAAAGAATAACATGATAAATAATG
ATCTTGGACCTGCAACAGCCCAGATGGCCCTTCAGCTATTCATCAAAGACTACAG
ATACACATATAGATGCCATAGAGGGGACACACTAATTCAGACGAGAAGATCATTC
GAGCTAAAGAGCCTGTGGGATCAAACCCAATCAAAGGCAGGGTTATTAGTATCTG
ATGGAGGACCGAATTTATACAACATCCGGAATCTTCACATTCCTGAAGTCTGCTTA
AAATGGGAGCTAATGGATGAGGATTATCGGGGAAGACTTTGCAATCCCCTAAATC
 
CTTTTGTCAGCCATAAAGAGATCGATTCTGTGAACAGTGCTGTTGTGATGCCAGCC
CATGGTCCAGCCAAAAGCATGGAATATGATGCCGTCGCAACTACACACTCCTGGA
TTCCCAAAAGGAACCGCTCTATTCTCAACACGAGCCAAAGGGGAATTCTTGAGGA
TGAACAGATGTACCAGAAGTGCTGCAACCTGTTCGAGAAATTTTTCCCTAGTAGTT
CATACAGGAGACCGGTTGGAATTTCTAGCATGGTGGAGGCCATGGTGTCTAGGG
CCCGGATTGATGCCAGGATTGACTTCGAATCTGGACGGATCAAAAAAGAAGAGTT
TTCTGAGATCATGAAGATCTGTTCCACCATTGAAGAACTCAGACGGCAAAAGTGA
Sequence ID NO 52:
   **>MT637912.1 Influenza B virus (B/Texas/9813/2019) segment 3 polymerase PA (PA) gene, complete cds** 
ATGGATACCTTTATTACAAGAAACTTCCAGACTACAATAATACAAAAGGCCAAAAA
CACAATGGCAGAATTTAGTGAAGATCCTGAATTGCAACCAGCAATGCTATTCAACA
TCTGTGTCCATCTAGAGGTTTGCTATGTAATAAGTGACATGAATTTTCTTGACGAA
GAAGGAAAAGCATATACAGCATTAGAAGGACAAGGAAAAGAACAAAATTTGAGAC
CACAATATGAAGTAATTGAGGGAATGCCAAGAACCATAGCATGGATGGTCCAAAG
ATCCTTAGCTCAAGAGCATGGGATAGAGACTCCCAAGTATCTGGCTGATTTGTTTG
ATTATAAGACCAAGAGATTTATAGAAGTTGGAATAACAAAAGGATTGGCTGATGAT
TACTTTTGGAAAAAGAAAGAGAAGCTGGGAAATAGCATGGAACTAATGATATTCAG
CTACAATCAAGACTATTCATTAAGTAATGAATCCTCATTGGATGAGGAAGGGAAAG
GGAGAGTGCTAAGCAGGCTCACAGAACTTCAAGCTGAATTAAGTCTGAAAAACTT
ATGGCAAGTTCTCATAGGAGAAGAAGATGTTGAAAAGGGAATTGACTTTAGACTTG
GACAAACAATATCTAGACTGAGGGATATATCTGTTCCTGCTGGTTTCTCCAATTTT
GAAGGAATGAGGAGCTACATAGACAATATAGACCCGAAAGGAGCAATAGAGAGAA
ACCTAGCAAGGATGTCTCCCTTAGTATCAGTCACACCTAAAAAGTTGAAATGGGAG
GATCTAAGACCAATAGGGCCTCACATTTACAACCATGAGCTACCAGAAGTTCCATA
CAATGCCTTTCTTTTAATGTCTGATGAACTGGGGCTGGCCAATATGACGGAGGGA
AAGTCCAAAAAACCAAAGACATTAGCCAAAGAATGTCTAGAAAAGTACTCAACACT
GCGGGATCAGACTGACCCAATATTAATAGTGAAAAGCGAAAAAGCTAACGAAAATT
TCCTATGGAAACTTTGGAGAGACTGTGTAAATACAATAAGCAATGAGGAAATGAGT
AACGAGTTACAGAAAACCAATTATGCCAAGTGGGCCACAGGGGATGGTTTAACAT
ACCAAAAAATAATGAAAGAAGTAGCAATAGATGACGAAACAATGTGCCAAGAAGA
GCCTAAAATCCCTAACAAGTGTAGAGTGGCTGCTTGGGTTCAAACAGAGATGAAC
CTATTGAGCACTCTGACAAGTAAAAGAGCTCTGGACCTACCAGAAATAGGGCCAG
ACGTGGCACCAGTGGAGCATGTTGGAAGTGAAAGAAGAAAATACTTTGTTAATGA
AATCAACTACTGTAAGGCCTCTACAGTTATGATGAAGTATGTGCTTTTTCACACTTC
ATTGCTGAATGAAAGCAATGCCAGCATGGGAAAATACAAAGTAATACCAATAACCA
 
ATAGAATAGTAAATGAAAAAGGAGAAAGTTTCGACATGCTTTATGGTCTGGCAGTT
AAAGGACAATCTCATCTGAGGGGGGACACTGATGTTGTAACAGTTGTAACTTTCG
AATTTAGTAGTACAGACCCCAGAGTGGACTCAGGAAAGTGGCCGAAATATACTGT
GTTTAGGATTGGCTCCCTATTTGTGGGTGGGAGGGAAAAATCTGTGTACCTATATT
GCAGAGTGAATGGCACAAATAAGATCCAAATGAAATGGGGAATGGAAGCTAGAAG
ATGTCTGCTTCAATCAATGCAACAAATGGAAGCAATTGTTGAACAAGAATCCTCGA
TACAAGGATACGACATGACCAAAGCTTGTTTCAAGGGGGACAGAATAAATAGCCC
CAAAACTTTCAGTATTGGAACTCAAGAAGGGAAACTAGTAAAAGGATCCTTTGGAA
AAGCACTAAGAGTAATATTTACCAAATGTTTGATGCACTATGTATTTGGAAATGCCC
AGTTGGAGGGATTCAGTGCCGAGTCTAGGAGACTTCTACTGTTGATTCAAGCATT
AAAGGACAGAAAGGGCCCTTGGGTGTTCGACTTAGAGGGAATGTACTCTGGAATA
GAAGAATGTATCAGTAACAACCCTTGGGTAATACAGAGTGCATACTGGTTCAATGA
ATGGTTGGGTTTTGAAAAGGAGGGAAGTAAAGTATTAGAATCAGTAGATGAAATAA
TGGATGAATAA
Sequence ID NO 53:
   > **5' primer for *E.coli* 23S rRNA NASBA amplification reaction** GGCATAAGCCAGCTTGACTG
Sequence ID NO 54:
   > **3' primer for *E.coli* 23S rRNA NASBA amplification reaction**
   T7 promoter **in bold**
   AATTC**TAATACGACTCACTATA**GGGACAGCTCGCGTACCACTTTAA
Sequence ID NO 55:
   > **5' primer for *S.aureus* mecA+ NASBA amplification reaction**
   GGTTACGGACAAGGTGAAAT
Sequence ID NO 56:
   > **3' primer for *S.aureus* mecA+ NASBA amplification reaction**
   T7 promoter **in bold**
   AATTC**TAATACGACTCACTATA**GGGAAGTGAGGTGCGTTAATATTG
Sequence ID NO 57:
   **> Broccoli RNA aptamer** (49-nt, Filonov *et al. JACS,* 2014)
   Variable region in lower-case letter, conservative bases participating in base pairing in **bold**, conservative bases in bulges underlined
   **GAGACG**G**UC**GGG**UCCA**gauauucguauc**UG**UC**GA**GUA**GA**G**UGU**GGG**CUC**
Sequence ID NO 58:
   > ***E.coli* 16S rRNA SMART-Broccoli second probe**
   Broccoli Reverse Complement in lower-case letter, T7 promoter in bold, complementary sequence to first probe in *italics,* target-specific region underlined 
gagcccacactctactcgacagatacgaatatctggacccgaccgtctcCCCTATAGTGAGTCGTATTAA
TTTCGAA/iSp18/GGCCTTCGGGTTGTAAAGTACTTTCAGCG/3AmMO/
Sequence ID NO 59:
   > **SARS-CoV-2 T3 SMART-Broccoli second probe**
   Broccoli Reverse Complement in lower-case letter, T7 promoter in **bold**, complementary sequence to first probe in *italics,* target-specific region underlined 
gagcccacactctactcgacagatacgaatatctggacccgaccgtctcCCCTATAGTGAGTCGTATTAA
TTTCGAA/iSp18/CAATGGTACTAAGAGGTTTGATAACCCTGT/3AmMO/
Sequence ID NO 60:
   > **Mango aptamer (Reverse Complement)**
   GGCACGTACGAATATACCACATACCAAACCTTCCTTCGTACGTGCC
Sequence ID NO 61:
   > **Broccoli aptamer (Reverse Complement)**
   GAGCCCACACTCTACTCGACAGATACGAATATCTGGACCCGACCGTCTC
Sequence ID NO 62:
   > **T7 Promoter (Reverse Complement)**
   TATAGTGAGTCGTATTA
Sequence ID NO 63 (similar to SEQ ID NO: 2, but substituting iSp18 for any spacer and optionally including any blocker molecule at the 3' end):
   > ***E.coli* 23S rRNA SMART-Mango second probe** (design based on Wharam *et al. Nucl Acids Res,* 2001)
   Mango Reverse Complement in lower-case letter, T7 promoter in **bold**, complementary sequence to first probe in *italics,* target-specific region underlined 
ggcacgtacgaatataccacataccaaaccttccttcgtacgtgccCCCTATAGTGAGTCGTATTAATTT
CGAA/Spacer/GGCATGACAACCCGAACACCAGTGAT/
 wherein, preferably, the 3' end of the molecule is blocked from extension by the DNA-directed DNA polymerase, preferably wherein the 3' end of the molecule is blocked by conjugating or coupling said 3' end to a 3' Amino Modifier (also referred herein as /3AmMO/).
Sequence ID NO 64 (similar to SEQ ID NO: 8, but substituting iSp18 for any spacer and optionally including any blocker molecule at the 3' end)
   > ***E.coli* 16S rRNA SMART-Mango second probe** (29-nt hybridization)
   Mango Reverse Complement in lower-case letter, T7 promoter in **bold**, complementary sequence to first probe in *italics,* target-specific region underlined 
ggcacgtacgaatataccacataccaaaccttccttcgtacgtgccCCCTATAGTGAGTCGTATTAATTT
CGAA/Spacer/GGCCTTCGGGTTGTAAAGTACTTTCAGCG/
 wherein, preferably, the 3' end of the molecule is blocked from extension by the DNA-directed DNA polymerase, preferably wherein the 3' end of the molecule is blocked by conjugating or coupling said 3' end to a 3' Amino Modifier (also referred herein as /3AmMO/).
Sequence ID NO 65 (similar to SEQ ID NO: 23, but substituting iSp18 for any spacer and optionally including any blocker molecule at the 3' end).
   *>* ***E.coli* 16S rRNA SMART-Mango second probe** (22-nt hybridization)
   Mango Reverse Complement in lower-case letter, T7 promoter in **bold,** complementary sequence to first probe in *italics,* target-specific region underlined 
ggcacgtacgaatataccacataccaaaccttccttcgtacgtgccCCCTATAGTGAGTCGTATTAATTT
CGAA/ Spacer /GGCCTTCGGGTTGTAAAGTACT
 wherein, preferably, the 3' end of the molecule is blocked from extension by the DNA-directed DNA polymerase, preferably wherein the 3' end of the molecule is blocked by conjugating or coupling said 3' end to a 3' Amino Modifier (also referred herein as /3AmMO/).
Sequence ID NO 66 (similar to SEQ ID NO: 24, but substituting iSp18 for any spacer and optionally including any blocker molecule at the 3' end).
   > ***E.coli* 16S rRNA SMART-Mango second probe** (15-nt hybridization)
   Mango Reverse Complement in lower-case letter, T7 promoter in **bold,** complementary sequence to first probe in *italics,* target-specific region underlined 
ggcacgtacgaatataccacataccaaaccttccttcgtacgtgccCCCTATAGTGAGTCGTATTAATTT
CGAA/ Spacer /GGCCTTCGGGTTGTA
 wherein, preferably, the 3' end of the molecule is blocked from extension by the DNA-directed DNA polymerase, preferably wherein the 3' end of the molecule is blocked by conjugating or coupling said 3' end to a 3' Amino Modifier (also referred herein as /3AmMO/).
Sequence ID NO 67 (similar to SEQ ID NO 25, but substituting iSp18 for any spacer and optionally including any blocker molecule at the 3' end)
   > ***E.coli* 16S rRNA SMART-Mango second probe** (7-nt hybridization)
   Mango Reverse Complement in lower-case letter, T7 promoter in **bold**, complementary sequence to first probe in *italics,* target-specific region underlined 
ggcacgtacgaatataccacataccaaaccttccttcgtacgtgccCCCTATAGTGAGTCGTATTAATTT
CGAA/ Spacer /GGCCTTC
 wherein, preferably, the 3' end of the molecule is blocked from extension by the DNA-directed DNA polymerase, preferably wherein the 3' end of the molecule is blocked by conjugating or coupling said 3' end to a 3' Amino Modifier (also referred herein as /3AmMO/).
Sequence ID NO 68 (similar to SEQ ID NO 27, but substituting iSp18 for any spacer and optionally including any blocker molecule at the 3' end).
   > **SARS-CoV-2 T3 SMART-Mango second probe**
   Mango Reverse Complement in lower-case letter, T7 promoter in **bold**, complementary sequence to first probe in *italics,* target-specific region underlined 
ggcacgtacgaatataccacataccaaaccttccttcgtacgtgccCCCTATAGTGAGTCGTATTAATTT
CGAA/ Spacer /CAATGGTACTAAGAGGTTTGATAACCCTGT
 wherein, preferably, the 3' end of the molecule is blocked from extension by the DNA-directed DNA polymerase, preferably wherein the 3' end of the molecule is blocked by conjugating or coupling said 3' end to a 3' Amino Modifier (also referred herein as /3AmMO/).
Sequence ID NO 69 (similar to SEQ ID NO: 36, but substituting iSp18 for any spacer and optionally including any blocker molecule at the 3' end).
   > ***E.coli* 23S rRNA SMART-Mango second probe**
   Mango Reverse Complement in lower-case letter, T7 promoter in **bold,** complementary sequence to first probe in *italics,* target-specific region underlined 
ggcacgtacgaatataccacataccaaaccttccttcgtacgtgccCCCTATAGTGAGTCGTATTAATTT
CGAA/Spacer/TAAAAGGTACTCCGGGGATAACAGGCTGA
 wherein, preferably, the 3' end of the molecule is blocked from extension by the DNA-directed DNA polymerase, preferably wherein the 3' end of the molecule is blocked by conjugating or coupling said 3' end to a 3' Amino Modifier (also referred herein as /3AmMO/).
Sequence ID NO 70 (similar to SEQ ID NO: 41, but substituting iSp18 for any spacer and optionally including any blocker molecule at the 3' end).
   > ***S.aureus mecA* SMART-Mango second probe**
   Mango Reverse Complement in lower-case letter, T7 promoter in **bold**, complementary sequence to first probe in *italics,* target-specific region underlined 
ggcacgtacgaatataccacataccaaaccttccttcgtacgtgccCCCTATAGTGAGTCGTATTAATTT
CGAA/ Spacer /CCTTTCAATCTATAGCGCATTAGAAAATAA
 wherein, preferably, the 3' end of the molecule is blocked from extension by the DNA-directed DNA polymerase, preferably wherein the 3' end of the molecule is blocked by conjugating or coupling said 3' end to a 3' Amino Modifier (also referred herein as /3AmMO/).
Sequence ID NO 71 (similar to SEQ ID NO: 47, but substituting iSp18 for any spacer and optionally including any blocker molecule at the 3' end).
   > **Influenza A *PB1* gene SMART-second probe** (15-nt target hybridization region) Mango Reverse Complement in lower-case letter, T7 promoter in **bold,** complementary sequence to first probe in *italics,* target-specific region underlined 
ggcacgtacgaatataccacataccaaaccttccttcgtacgtgccCCCTATAGTGAGTCGTATTAATTT
CGAA/ Spacer /CTCCAGTATAAGGGA
 wherein, preferably, the 3' end of the molecule is blocked from extension by the DNA-directed DNA polymerase, preferably wherein the 3' end of the molecule is blocked by conjugating or coupling said 3' end to a 3' Amino Modifier (also referred herein as /3AmMO/).
Sequence ID NO 72 (similar to SEQ ID NO: 48, but substituting iSp18 for any spacer and optionally including any blocker molecule at the 3' end).
   > **Influenza B *PA* gene SMART-second probe** (15-nt target hybridization region) Mango Reverse Complement in lower-case letter, T7 promoter in **bold,** complementary sequence to first probe in *italics,* target-specific region underlined 
ggcacgtacgaatataccacataccaaaccttccttcgtacgtgccCCCTATAGTGAGTCGTATTAATTT
CGAA/Spacer/TTCTTGGCATTCCCT
 wherein, preferably, the 3' end of the molecule is blocked from extension by the DNA-directed DNA polymerase, preferably wherein the 3' end of the molecule is blocked by conjugating or coupling said 3' end to a 3' Amino Modifier (also referred herein as /3AmMO/).
Sequence ID NO 73 (similar to SEQ ID NO: 58, but substituting iSp18 for any spacer and optionally including any blocker molecule at the 3' end).
   > ***E.coli* 16S rRNA SMART-Broccoli second probe**
   Broccoli Reverse Complement in lower-case letter, T7 promoter in **bold**, complementary sequence to first probe in *italics,* target-specific region underlined 
gagcccacactctactcgacagatacgaatatctggacccgaccgtctcCCCTATAGTGAGTCGTATTAA
TTTCGAA/ Spacer /GGCCTTCGGGTTGTAAAGTACTTTCAGCG
 wherein, preferably, the 3' end of the molecule is blocked from extension by the DNA-directed DNA polymerase, preferably wherein the 3' end of the molecule is blocked by conjugating or coupling said 3' end to a 3' Amino Modifier (also referred herein as /3AmMO/).
Sequence ID NO 74 (similar to SEQ ID NO: 59, but substituting iSp18 for any spacer and optionally including any blocker molecule at the 3' end).
   > **SARS-CoV-2 T3 SMART-Broccoli second probe**
   Broccoli Reverse Complement in lower-case letter, T7 promoter in **bold**, complementary sequence to first probe in *italics,* target-specific region underlined 
gagcccacactctactcgacagatacgaatatctggacccgaccgtctcCCCTATAGTGAGTCGTATTAA
TTTCGAA/ Spacer /CAATGGTACTAAGAGGTTTGATAACCCTGT
 wherein, preferably, the 3' end of the molecule is blocked from extension by the DNA-directed DNA polymerase, preferably wherein the 3' end of the molecule is blocked by conjugating or coupling said 3' end to a 3' Amino Modifier (also referred herein as /3AmMO/).

The invention is described below by the following examples, which must be considered as merely illustrative and in no case limiting the scope of the present invention.

### EXAMPLES

### Example 1: Apta-SMART probes design

The aptamer-SMART ('apta-SMART') assay is a detection method that depends on the hybridization of two DNA probes of which regions are complementary to a target nucleic acid (either DNA or RNA). These two probes (first and second probes) are capable of hybridizing to adjacent regions of the sequence of interest, and only in that case enable the annealing of complementary arm specific sequences in both probes forming a three-way junction (3WJ) structure. Following 3WJ formation, *Bst* DNA polymerase extends the first probe sequence from 5' to 3' and creates a functional double-stranded RNA polymerase promoter that in the presence of the specific RNA polymerase (either T7, T3 or SP6), allows the transcription of the aptamer sequence using the second probe as template (i.e. Mango, Broccoli, etc.) producing multiple copies of the RNA aptamer to be detected by a fluorescent readout after the addition of the fluorogen substrate (e.g. T01-biotin or TO3-biotin for Mango).

Based on this concept, here we show how to design a pair of apta-SMART probes to detect *Escherichia coli -E.coli-* 23S rRNA (**Figure 4**). The design of the probes was based on previously published SMART probes for the detection of an *E.coli* K12 23S DNA region (Wharam et al. Nucl Acids Res, 2001). Each probe includes one region that can hybridize to the target and another region, much shorter, that hybridizes to the other probe. These probes are designed such that they can only anneal to each other in the presence of the specific target, forming the 3WJ structure. The probes to detect the different rRNA target sequences were all designed in the same way, by changing the target-hybridizing regions only.

The *E.coli* 23S rRNA first probe (**SEQ ID NO: 1**) comprises a target-specific region of 30 nucleotides which hybridizes specifically to the target sequence and a non-target-complementary region (contiguous with the target-specific region) of 8 fixed nucleotides (TTCGAAAT) that is complementary to the second probe forming the 3WJ.

The *E.coli* 23S rRNA second probe (**SEQ ID NO: 2**) also comprises a target-specific region of 30 nucleotides which hybridizes to the target sequence (in a position adjacent to the target region recognized by the first probe) and the 8 nucleotide-fixed sequence (reverse complemented) for the annealing with the first probe. The second probe also contains the non-functional (reverse complement) single-stranded RNA polymerase promoter sequence followed by the reverse complement RNA aptamer sequence, i.e. Mango-III (A10U) aptamer **(SEQ ID NO: 3,** Trachman et al. Nat Chem Biol, 2019). The 3' end of the second probe is blocked by an amino group, to avoid RNA polymerase-mediated first. In addition, to reduce the nonspecific background signal, a non-nucleosidic linker (in this case, an 18-atom hexa-ethyleneglycol spacer) is included just prior the 3WJ (indicated as iSp18 in **Figure 4**).

### Example 2: Region of hybridization between probes forming the 3WJ (non-target complementary region).

Based on previously published primers (OV2 and OV3, **SEQ ID NO: 4** and **5,** respectively) for NASBA detection of the *E.coli* 16S rRNA (**SEQ ID NO: 6**) (Kao et al. Anal Letters, 2010), we designed the *E.coli* 16S rRNA first probe (**SEQ ID NO: 7**) and a modified second probe containing a T7 promoter and a Mango aptamer (**SEQ ID NO: 8**) to detect the previously reported reverse complement (RC) region of the *E.coli* 16S rRNA (**SEQ ID NO: 9**) (Kao et al. Anal Letters, 2010).

The preferred size of the non-target-complementary region is of 8 nucleotides (8-nt, TTCGAAAT) that are complementary to the arm of the second probe. In this example, we tested the 8-nt hybridizing and forming the 3WJ in the first and second probes (**SEQ ID NO: 7** and **8**) as our standard and whether using less (2-, 4-, 6-nt; **SEQ ID NO: 10**, **11, 12**, respectively) hybridizing nucleotides in the first probe are sufficient for SMART detection with a high signal-to-noise ratio using a synthetic RNA target (sRNA, **SEQ ID NO: 13**) of *E.coli* 16S rRNA. To test this, we used the sequences TT, TTCG and TTCGAA as 3WJ forming between probes (as shown in **SEQ ID NO: 10, 11, 12**). The complementary sequences in the second probes were (in 5' to 3' direction): ATTTCGAA for the standard 8-nt and AA, CGAA and TTCGAA for the 2-, 4- and 6-nt ones. Furthermore, we use more than 8-nt (specifically, 10- and 15-nt; **SEQ ID NO: 14** and **15**) to decipher whether the hybridization between probes becomes nonspecific resulting in a reaction that can occur in the absence of the sRNA. For this, we use the sequences TTCGAAATTA and TTCGAAATTAATACG (as shown in **SEQ ID NO: 14** and **15**). The complementary sequences in the second probes were (in 5' to 3' direction): TAATTTCGAA and CGTATTAATTTCGAA for the 10- and 15-nt ones, respectively.

Using the *E.coli* 16S rRNA standard first probe (**SEQ ID NO: 7**) and the first ones with 2-, 4-, 6-, 10- and 15-nt (**SEQ ID NO: 10, 11, 12, 14** and **15**, respectively) and a standard second probe (**SEQ ID NO: 8**) containing a Mango aptamer for the detection of a reverse complement RNA sequence (synthetic RNA, sRNA) (**SEQ ID NO: 13**) of *E.coli* 16S rRNA (**SEQ ID NO: 6**), we ran the apta-SMART reaction as detailed: denaturing at 65°C for 5 minutes followed by a pre-incubation step at 41°C for 60 minutes of a reaction mixture with both first and second probes (840 and 700 fmols, respectively) with 17.5 nM of sRNA (**SEQ ID NO: 13**) in 1X transcription buffer (40 mM Tris-HCI pH 7.9, 6 mM MgCl₂, 2 mM spermidine and 10 mM NaCl) (from T7 RNA pol, Promega) plus 1 mM spermidine (Sigma). After that, 3 µL of dNTPs/ NTPs and enzyme mix (containing 5 µM dNTPs, 2 mM NTPs, 2 U Bst DNA pol and 25 U T7 RNA pol) were added to a final volume of 10 µL and incubated for another 60 minutes in the same buffer. Finally, 1 µL of 4.8 µM T01-biotin fluorogen (BioCat, Cat#: G955-ABM) was added (480 nM final concentration) right before fluorescence was read at 510 nm (excitation) and 535 nm (emission) wavelengths using a BioTek plate reader (Synergy H1 Hybrid Multi-Mode Reader) in black bottom 384-well plates (Nunc).

As shown in **Figure 5**, the apta-SMART detection of 17.5 nM sRNA (**SEQ ID NO: 13**) is optimal when the region of hybridization (3WJ) contains 8-nt and 6-nt (**SEQ ID NO: 7** and **SEQ ID NO: 12**) with a fold-change of sRNA detection over no sRNA of 4.6 and 3.6, respectively (**Figure 5B**) and abrogated when containing 4-nt or less nucleotides (**SEQ ID NO: 10** and **SEQ ID NO: 11**) forming the 3WJ. When 10- and 15-nt of hybridization were tested, high nonspecific signal coming from the no sRNA control was found, suggesting an interaction that can occur in the absence of the RNA target. The lowest amount of sRNA tested, 1.75 nM, is specifically detected with the optimal 8 hybridizing nucleotides construct reaching 1.4-fold-change over no sRNA. Of note, 2 or 5 residues of the 10- and 15-nt probes, respectively, may be interacting with the T7 promoter sequence in the second probe region and forming a dsDNA that could be resulting in T7 RNA polymerase 'promiscuous' activity. To discard that, we performed an assay without the *Bst* polymerase. As **Figure 5C** and **5D** show, the apta-SMART signal is null without *Bst* polymerase activity. These results confirm that 8-nt hybridization between the first and second probes is the optimal size for target specific as 10 complementary nucleotides are already hybridizing non-specifically in the absence of an RNA target (sRNA, in the example).

### Example 3: Separation between hybridization zones (target-probes)

As detailed, both the first and the second probes have a target-specific region of 30 nucleotides each which hybridize to a 60 nucleotides region target sequence. These regions of 30-nt are adjacent in the target one to each other, thus, after target hybridization they are contiguous.

Here, we tested whether the first probe can anneal to the RNA target region (**SEQ ID NO: 13**) with a separation of 1-, 2-, 4- or 6-nucleotides (**SEQ ID NO: 16, 17, 18** and **19**) from the hybridization of the second probe region and still form the 3WJ and give an optimal SMART reaction and signal.

Using the *E.coli* 16S rRNA second probe (**SEQ ID NO: 8**) and the first probes with 0-(standard, **SEQ ID NO: 7**), 1-, 2-, 4- and 6-nucleotides separation (**SEQ ID NO: 16, 17, 18** and **19**), we ran the apta-SMART reaction as detailed: denaturing at 65°C for 5 minutes followed by a pre-incubation step at 41°C for 60 minutes of a reaction mixture with both first and second probes (840 and 700 fmols, respectively) with 17.5 nM of sRNA (**SEQ ID NO: 13**) in 1X transcription buffer (40 mM Tris-HCI pH 7.9, 6 mM MgCl₂, 2 mM spermidine and 10 mM NaCl) (from T7 RNA pol, Promega) plus 1 mM spermidine (Sigma). After that, 3 µL of dNTPs/ NTPs and enzyme mix (containing 5 µM dNTPs, 2 mM NTPs, 2 U Bst DNA pol and 25 U T7 RNA pol) were added to a final volume of 10 µL and incubated for another 60 minutes in the same buffer. Finally, 1 µL of 4.8 µM T01-biotin fluorogen (BioCat, Cat#: G955-ABM) was added (480 nM final concentration) right before fluorescence was read at 510 nm (excitation) and 535 nm (emission) wavelengths using a BioTek plate reader (Synergy H1 Hybrid Multi-Mode Reader) in black bottom 384-well plates (Nunc).

**Figure 6** shows the detection of sRNA with Mango-SMART using 'on-target' contiguous probes and probes with a gap between the target hybridization region of 1-to 6-nt. All conditions optimally detect 17.5 nM of sRNA showing a fold-change of sRNA over no sRNA > 4.0 (**Figure 6B**).

### Example 4: Minimal hybridization region length of probes to the target.

To decipher whether an hybridization region of probes with the target shorter than the standard of 29-nt (**SEQ ID NO: 7** and **8**) would still be optimal for apta-SMART reaction, we performed experiments using 22-nt, 15-nt and 7-nt hybridization regions in the first (**SEQ ID NO: 20, 21** and **22**) and second probes (**SEQ ID NO: 23, 24** and **25**) using (1) the *E.coli* 16S sRNA (**SEQ ID NO: 13**) as target and (2) NASBA product of *E.coli* 16S rRNA region. All the probes contained the standard 8-nt 3WJ region and the detection was through Mango aptamer as per the two previous examples.

### 4.1. Apta-SMART detection of E.coli 16S synthetic RNA target with probes of different length

Using the 29 nucleotides (-nt) *E.coli* 16S rRNA standard first and second probes (**SEQ ID NO: 7** and **8**) and 22-, 15- and 7-nt first (**SEQ ID NO: 20, 21** and **22**) and second (**SEQ ID NO: 23, 24** and **25**) probes containing a Mango aptamer for the detection of a reverse complement RNA sequence (synthetic target) (**SEQ ID NO: 13**) of *E.coli* 16S rRNA (**SEQ ID NO: 6**), we ran the apta-SMART reaction as detailed: denaturing at 65°C for 5 minutes followed by a pre-incubation step at 41°C for 60 minutes of a reaction mixture with both first and second probes (840 and 700 fmols, respectively) with 17.5 nM of sRNA (**SEQ ID NO: 13**) in 1X transcription buffer (40 mM Tris-HCI pH 7.9, 6 mM MgCl₂, 2 mM spermidine and 10 mM NaCl) (from T7 RNA pol, Promega) plus 1 mM spermidine (Sigma). After that, 3 µL of dNTPs/ NTPs and enzyme mix (containing 5 µM dNTPs, 2 mM NTPs, 2 U Bst DNA pol and 25 U T7 RNA pol) were added to a final volume of 10 µL and incubated for another 60 minutes in the same buffer. Finally, 1 µL of 4.8 µM T01-biotin fluorogen (BioCat, Cat#: G955-ABM) was added (480 nM final concentration) right before fluorescence was read at 510 nm (excitation) and 535 nm (emission) wavelengths using a BioTek plate reader (Synergy H1 Hybrid Multi-Mode Reader) in black bottom 384-well plates (Nunc).

### 4.2. Apta-SMART detection of E.coli 16S RNA region after NASBA reaction with probes of different length

Based on previously published primers (OV2 and OV3, **SEQ ID NO:** and **5**, respectively) for NASBA detection of the *E.coli* 16S rRNA (**SEQ ID NO: 6**) (Kao et al. Anal Letters, 2010), we designed the *E.coli* 16S rRNA first (**SEQ ID NO: 7**) and a modified second probe containing a T7 promoter and a Mango aptamer (**SEQ ID NO: 8**) to detect the previously reported reverse complement (RC) region of the *E.coli* 16S rRNA (**SEQ ID NO: 9**) (Kao et al. Anal Letters, 2010).

### Step 1: NASBA.

We used the previously published 5' primer (**SEQ ID NO: 4**) and the 3' primer containing a T7 promoter (**SEQ ID NO: 5**) that isothermally amplify a region (**SEQ ID NO: 9**) of *E.coli* 16S rRNA (**SEQ ID NO: 6**) (Kao et al. Anal Letters, 2010). The NASBA consisted of a reaction containing 2 µL of an *E.coli* culture and 500 nM of NASBA primers in 15 µL final volume in NASBA buffer (40 mM Tris-HCl, pH 8.0, 13.2 mM MgCl₂, 75 mM KCI, 10 mM DTT, 1 mM dNTPs, 2 mM ATP, 2 mM UTP, 2 mM CTP, 1.5 mM GTP, 0.5 mM ITP and 15% uL DMSO -corresponding to 3 µL of the reaction mix-) in a initial denaturation step of 2 minutes at 95°C and 2 minutes at 37°C. Then, 5 µL of enzyme mix (6.8 U AMV-RT, 0.08 U RNAse H, 32 U T7 RNA polymerase and 120 µg/mL BSA in water) was added and incubated at 37°C for 90 minutes. The resulting amplified fragment is a reverse complement (RC) region of a 191-nt region (**SEQ ID NO: 9**) of *E.coli* 16S rRNA (**SEQ ID NO: 6**) used as template for Mango-SMART reaction (*Step 2*).

### Step 2: Mango-SMART detection.

Using the 29-, 22-, 15- and 7-nt *E.coli* 16S rRNA first (**SEQ ID NO: 7, 20, 21** and **22**) and second (**SEQ ID NO: 8, 23, 24** and **25**) probes containing a Mango aptamer for the detection of a reverse complement RNA sequence of *E.coli* 16S RNA (**SEQ ID NO: 6**), we ran the Mango-SMART reaction as detailed: denaturing at 65°C for 5 minutes followed by a pre-incubation step at 41°C for 60 minutes of a reaction mixture with both first and second probes (840 and 700 fmols, respectively) with 1 µL of NASBA reaction in 1X transcription buffer (*see composition above*; from T7 RNA pol, Promega) plus 1 mM spermidine (Sigma). After that, 3 µL of dNTPs/ NTPs and enzyme mix (containing 5 µM dNTPs, 2 mM NTPs, 2 U Bst DNA pol and 25 U T7 RNA pol) were added to a final volume of 10 µL and incubated for another 60 minutes in the same buffer. Finally, 1 µL of 4.8 µM T01-biotin fluorogen (BioCat, Cat#: G955-ABM) was added (480 nM final concentration) right before fluorescence was read at 510 nm (excitation) and 535 nm (emission) wavelengths using a BioTek plate reader (Synergy H1 Hybrid Multi-Mode Reader) in black bottom 384-well plates (Nunc).

As shown in **Figure 7****,** and when using sRNA as target, Mango-SMART signal was optimal down to 15-nt hybridization region per probe but the reaction was completely abolished when using probes with only a 7-nt hybridization region (**Figure 7A**). Thus, no signal (compared to no RNA) was found for 7-nt hybridization whereas 15-nt, 22-nt and 29-nt probes showed increases of 6.1- to 7.7-fold change over no RNA (**Figure 7B**). When using a NASBA amplicon as target, as for sRNA, the reaction showed a positive outcome for Mango-TO1 signal for standard 29-nt hybridization region length down to 15-nt (per probe) but no signal was detected for 7-nt hybridization region (**Figure 7C**). When normalizing data per NASBA no RNA data point (**Figure 7D**) 29-, 22-, 15-nt probes showed fold-changes >1.5 indicating positivity.

### Example 5: Comparison of Molecular Beacon (MB) to apta-SMART detection

In this example, we compare the use of a molecular beacon for the detection of the newly-synthesized RNA with our apta-SMART (using a Mango aptamer) detection method. For this comparison, SARS-CoV-2 first and second probes (**SEQ ID NO: 26** and **27**, respectively) were used for the detection of a reverse complement (RC) of a region of SARS-CoV-2 *S* 'spike' ssRNA that encodes for spike -S glycoprotein-; *S 'spike'* RNA from now on in the text), sequence identical to the result of NASBA reaction. We tested the SMART reaction using: (1) a synthetic RNA consisting of the 30-nucleotide region of interaction (**SEQ ID NO: 28**) and, (2) a RC RNA sequence result of the NASBA reaction (**SEQ ID NO: 29**).

For Mango-SMART detection we use the SARS-CoV-2 first (**SEQ ID NO: 26**) and the second probe containing Mango aptamer (**SEQ ID NO: 27**).

For Molecular Beacon (MB) detection (see diagram on **Figure 8**) of the same region of SARS-CoV-2, we designed a new SARS-CoV-2 second probe (**SEQ ID NO: 30**) containing a 25-nt RNA sequence identical to the internal loop of the Molecular Beacon Oligonucleotide probe (instead of the Mango-aptamer sequence). The Molecular Beacon Oligonucleotide probe used for the detection (**SEQ ID NO: 31**) (Zhao et al. J Clin Microbiol, 2009) comprises a sequence derived from *E. coli*, with complimentary 5' and 3' ends.

### 4.1. Apta-SMART detection of synthetic target

Using the SARS-CoV-2 first (**SEQ ID NO: 26**) and SARS-CoV-2 second probes containing a Mango aptamer (**SEQ ID NO: 27**) for the detection of a reverse complement RNA sequence (synthetic target) (**SEQ ID NO: 28**) of SARS-CoV-2 S *'spike'* RNA (**SEQ ID NO: 32**), we ran the apta-SMART reaction (using Mango as fluorogenic aptamer) as detailed: denaturing at 65°C for 5 minutes followed by a pre-incubation step at 41°C for 60 minutes of a reaction mixture with both first and second probes (840 and 700 fmols, respectively) with 1.75, 17.5 or 175 nM of synthetic RNA in 1X transcription buffer (40 mM Tris-HCI pH 7.9, 6 mM MgCl₂, 2 mM spermidine and 10 mM NaCl) (from T7 RNA pol, Promega) plus 1 mM spermidine (Sigma). After that, 3 µL of dNTPs/ NTPs and enzyme mix (containing 5 µM dNTPs, 2 mM NTPs, 2 U Bst DNA pol and 25 U T7 RNA pol) were added to a final volume of 10 µL and incubated for another 60 minutes in the same buffer. Finally, 1 µL of 4.8 µM T01-biotin fluorogen (BioCat, Cat#: G955-ABM) was added (480 nM final concentration) right before fluorescence was read at 510 nm (excitation) and 535 nm (emission) wavelengths using a BioTek plate reader (Synergy H1 Hybrid Multi-Mode Reader) in black bottom 384-well plates (Nunc).

### 4.2. Apta-SMART detection of genomic region after NASBA

### Step 1: NASBA.

We designed a 5' primer (**SEQ ID NO: 33**) and a 3' primer containing a T7 promoter (**SEQ ID NO: 34**) that isothermally amplify a 189 nucleotide region (**SEQ ID NO: 29**) of SARS-CoV-2 *S 'spike'* RNA (**SEQ ID NO: 32**). The NASBA reaction consisted of a reaction containing 2 µL of previously isolated SARS-CoV-2 RNA (with known Ct values as assessed by qPCR) and 500 nM of NASBA primers in 15 µL final volume in NASBA buffer (40 mM Tris-HCl, pH 8.0, 13.2 mM MgCl₂, 75 mM KCI, 10 mM DTT, 1 mM dNTPs, 2 mM ATP, 2 mM UTP, 2 mM CTP, 1.5 mM GTP, 0.5 mM ITP and 15% uL DMSO -corresponding to 3 µL of the reaction mix-) in a initial denaturation step of 2 minutes at 95°C and 2 minutes at 37°C. Then, 5 µL of enzyme mix (6.8 U AMV-RT, 0.08 U RNAse H, 32 U T7 RNA polymerase and 120 µg/mL BSA in water) was added and incubated at 37°C for 90 minutes. The resulting amplified fragment (**SEQ ID NO: 29**) is a reverse complement (RC) region of the *S 'spike'* RNA (**SEQ ID NO: 32**) used as template for Mango-SMART reaction (*Step 2*).

### Step 2a: Mango-SMART detection.

Using the SARS-CoV-2 first (**SEQ ID NO: 26**) and SARS-CoV-2 second probes containing a Mango aptamer (**SEQ ID NO: 27**) for the detection of a RC RNA sequence (result of NASBA reaction) (**SEQ ID NO: 29**) of SARS-CoV-2 *S 'spike'* RNA (**SEQ ID NO: 32**), we ran the Mango-SMART reaction as detailed: denaturing at 65°C for 5 minutes followed by a pre-incubation step at 41°C for 60 minutes of a reaction mixture with both first and second probes (840 and 700 fmols, respectively) with 1 µL of NASBA reaction or 1.75, 17.5 or 175 nM of synthetic RNA in 1X transcription buffer (*as above;* from T7 RNA pol, Promega) plus 1 mM spermidine (Sigma). After that, 3 µL of dNTPs/ NTPs and enzyme mix (containing 5 µM dNTPs, 2 mM NTPs, 2 U Bst DNA pol and 25 U T7 RNA pol) were added to a final volume of 10 µL and incubated for another 60 minutes in the same buffer. Finally, 1 µL of 4.8 µM T01-biotin fluorogen (BioCat, Cat#: G955-ABM) was added (480 nM final concentration) right before fluorescence was read at 510 nm (excitation) and 535 nm (emission) wavelengths using a BioTek plate reader (Synergy H1 Hybrid Multi-Mode Reader) in black bottom 384-well plates (Nunc).

### 4.3. Molecular Beacon (MB) detection assay

### 4.3.1. MB detection of synthetic RNA

Using the SARS-CoV-2 first (**SEQ ID NO: 26**) and SARS-CoV-2 second (**SEQ ID NO: 30**) probes containing a 25-nt RNA sequence identical to the internal loop of the MB Oligonucleotide probe (**SEQ ID NO: 31**, Zhao et al. J Clin Microbiol. 2009) for the detection of a RC RNA sequence (synthetic target) (**SEQ ID NO: 28**) of a region of SARS-CoV-2 *S 'spike'* RNA (**SEQ ID NO: 32**), we ran the SMART reaction as detailed: denaturing at 65°C for 5 minutes followed by a pre-incubation step at 41°C for 60 minutes of a reaction mixture with both first and second probes (840 and 700 fmols, respectively) with 1 µL of NASBA reaction or 1.75, 17.5 or 175 nM of synthetic RNA in 1X transcription buffer (Promega) plus 1 mM spermidine (Sigma). After that, 3 µL of dNTPs/ NTPs and enzyme mix (containing 5 µM dNTPs, 2 mM NTPs, 2 U Bst DNA pol and 25 U T7 RNA pol) were added to a final volume of 10 µL and incubated for another 60 minutes in the same buffer. Finally, 1 µL of MB Oligonucleotide probe containing a 5'-6-FAM (reporter) and a 3'-Dabcyl (quencher) (**SEQ ID NO: 31**, Zhao et al. J Clin Microbiol. 2009) (at 3 different final concentrations: 300 nM, 100 nM and 10 nM) was added and incubated in the dark at room temperature for 60 minutes. Fluorescence signal from the hybridized beacon/target was read at 495 nm (excitation) and 520 nm (emission) wavelengths using a BioTek plate reader (Synergy H1 Hybrid Multi-Mode Reader) in black bottom 384-well plates (Nunc).

### 4.3.2. MB detection of a genomic region after NASBA

Using the SARS-CoV-2 first (**SEQ ID NO: 26**) and SARS-CoV-2 second (**SEQ ID NO: 30**) probes containing a 25-nt RNA sequence identical to the internal loop of the MB Oligonucleotide probe (**SEQ ID NO: 31**, Zhao et al. J Clin Microbiol. 2009) for the detection of a RC RNA sequence (result of NASBA reaction) (**SEQ ID NO: 29**) of a region of SARS-CoV-2 *S 'spike'* RNA (**SEQ ID NO: 32**), we ran the SMART reaction as detailed: denaturing at 65°C for 5 minutes followed by a pre-incubation step at 41°C for 60 minutes of a reaction mixture with both first and second probes (840 and 700 fmols, respectively) with 1 µL of NASBA reaction or 1.75, 17.5 or 175 nM of synthetic RNA in 1X transcription buffer (Promega) plus 1 mM spermidine (Sigma). After that, 3 µL of dNTPs/ NTPs and enzyme mix (containing 5 µM dNTPs, 2 mM NTPs, 2 U Bst DNA pol and 25 U T7 RNA pol) were added to a final volume of 10 µL and incubated for another 60 minutes in the same buffer. Finally, 1 µL of MB Oligonucleotide probe (**SEQ ID NO: 31**) (at 3 different final concentrations: 300 nM, 100 nM and 10 nM) was added and incubated in the dark at room temperature for 60 minutes. Fluorescence signal from the hybridized beacon/target was read at 495 nm (excitation) and 520 nm (emission) wavelengths using a BioTek plate reader (Synergy H1 Hybrid Multi-Mode Reader) in black bottom 384-well plates (Nunc).

### 4.4. Results of the comparison of MB versus apta-SMART using sRNA and NASBA products

As we can see in **Figure 9**, the Mango-TO1 detection of 175 and 17.5 nM sRNA concentration was optimal, despite some variability between experiments with a fold-change over no RNA control of nearly 4-fold. 1.75 nM sRNA was also detected with a lower sensitivity (fold-change not reaching 2-fold). However, when using MB for detection, the sensitivity was completely lost even at higher sRNA and MB concentrations (175 nM and 300 nM, respectively). None of the conditions were capable of detecting the sRNA concentrations tested. These results show a clear advantage of Mango-SMART over MB detection.

**Figure 10** shows the fluorescence detection comparison of SARS-CoV-2 *'spike' S* RNA region NASBA products. As for sRNA, the Mango-SMART method was sensitive to detect the NASBA products from three SARS-CoV-2 RNA samples tested (with Ct values ranging from 13.23 to 22.66) with changes of ≥1.5-fold over NASBA no RNA (control). On the contrary, MB were incapable of detecting any of the samples, confirming a low range of sensitivity for the detection of SARS-CoV-2 RNA samples.

### Example 6: Apta-SMART detection of synthetic RNA target of an E.coli 16S rRNA

Based on previously published primers (OV2 and OV3, **SEQ ID NO:** and **5**, respectively) for NASBA detection of an *E.coli* 16S rRNA region (**SEQ ID NO: 6**) (Kao et al. Anal Letters, 2010), we designed the *E.coli* 16S rRNA first (**SEQ ID NO: 7**) and a modified second probe containing a Mango aptamer (**SEQ ID NO: 8**) to detect the previously reported reverse complement (RC) region of the *E.coli* 16S rRNA (**SEQ ID NO: 9**) (Kao et al. Anal Letters, 2010). Our assay for each bacterial species tested consisted in the SMART reaction using a sRNA of a 75-nucleotide region (**SEQ ID NO: 13**) that contains the hybridization regions of *E.coli* 16S rRNA first (**SEQ ID NO: 7**) and second (**SEQ ID NO: 8**) probes. The SMART reaction started with a denaturing step at 65°C for 5 minutes followed by a pre-incubation step at 41°C for 60 minutes of a reaction mixture with both first and second probes (840 and 700 fmols, respectively) and 17.5 nM of synthetic RNA target in 1X transcription buffer (Promega) plus 1 mM spermidine (Sigma), up to 7 µL. After that, 3 µL of dNTPs/ NTPs and enzyme mix (containing 5 µM dNTPs, 2 mM NTPs, 2 U Bst DNA pol and 25 U T7 RNA pol) were added and incubated for another 60 minutes in the same buffer. Finally, 1 µL of 4.8 µM TO1-biotin fluorogen (BioCat, Cat#: G955-ABM) was added (480 nM final concentration) right before fluorescence was read at 510 nm (excitation) and 535 nm (emission) wavelengths using a BioTek plate reader (Synergy H1 Hybrid Multi-Mode Reader) in black bottom 384-well plates (Nunc).

As shown in **Figure 11****,** the Mango-SMART detection of *E.coli* 16S rRNA was optimal at 17.5 nM sRNA showing a fold-change over no sRNA control of 6.0. No sRNA control was used for background fluorescence showing an average of ~10.500 RFU.

### Example 7: Apta-SMART detection of synthetic RNA target of an E.coli 23S rRNA

We designed an *E*. *coli* 23S rRNA first (**SEQ ID NO: 35**) and a second probe containing a Mango aptamer (**SEQ ID NO: 36**) for the detection of a reverse complement (RC) of an RNA region (**SEQ ID NO: 37**) of *E.coli* 23S rRNA (**SEQ ID NO: 38**). We tested the SMART reaction using a sRNA containing the 58-nucleotide region of interaction (**SEQ ID NO: 39**) with *E.coli* 23S first and second probes. The SMART reaction started with a denaturing step at 65°C for 5 minutes followed by a pre-incubation step at 41°C for 60 minutes of a reaction mixture with both first and second probes (420 and 350 fmols, respectively) and 17.5 nM of synthetic RNA target in 1X transcription buffer (Promega) plus 1 mM spermidine (Sigma), up to 7 µL. After that, 3 µL of dNTPs/ NTPs and enzyme mix (containing 5 µM dNTPs, 2 mM NTPs, 2 U Bst DNA pol and 25 U T7 RNA pol) were added and incubated for another 60 minutes in the same buffer. Finally, 1 µL of 4.8 µM T01-biotin fluorogen (BioCat, Cat#: G955-ABM) was added (480 nM final concentration) right before fluorescence was read at 510 nm (excitation) and 535 nm (emission) wavelengths using a BioTek plate reader (Synergy H1 Hybrid Multi-Mode Reader) in black bottom 384-well plates (Nunc).

As shown in **Figure 12**, the Mango-SMART detection of *E.coli* 23S rRNA was optimal using 17.5 nM sRNA showing an increase of signal over no sRNA control of 8.7-fold (~98.500 RFU) with sRNA control showing an average of fluorescence of ~11.750 RFU.

### Example 8: Apta-SMART detection of synthetic RNA target of methicillin resistance protein subunit A gene (mecA) of Staphylococcus aureus (S. aureus).

We designed a *mecA* first (**SEQ ID NO: 40**) and a *mecA* second probe containing a Mango aptamer (**SEQ ID NO: 41**) for the detection of a reverse complement (RC) of an RNA region (**SEQ ID NO: 42**) of *S.aureus mecA* gene (**SEQ ID NO: 43**). We tested the SMART reaction using a synthetic RNA consisting of the 98-nucleotide region containing 60-nt of target-probe interaction (**SEQ ID NO: 44**) with mecA first and second probes. The SMART reaction started with a denaturing step at 65°C for 5 minutes followed by a pre-incubation step at 41°C for 60 minutes of a reaction mixture with both first and second probes (420 and 350 fmols, respectively) and 17.5 nM of synthetic RNA target in 1X transcription buffer (Promega) plus 1 mM spermidine (Sigma), up to 7 µL. After that, 3 µL of dNTPs/ NTPs and enzyme mix (containing 5 µM dNTPs, 2 mM NTPs, 2 U Bst DNA pol and 25 U T7 RNA pol) were added and incubated for another 60 minutes in the same buffer. Finally, 1 µL of 4.8 µM T01-biotin fluorogen (BioCat, Cat#: G955-ABM) was added (480 nM final concentration) right before fluorescence was read at 510 nm (excitation) and 535 nm (emission) wavelengths using a BioTek plate reader (Synergy H1 Hybrid Multi-Mode Reader) in black bottom 384-well plates (Nunc).

**Figure 13** shows Mango-SMART detection of *S*. *aureus mecA* gene using 17.5 nM sRNA. Results showed an optimal detection change of sRNA over no sRNA control of 9.1-fold (~79500 RFU). No sRNA control was used for background fluorescence showing an average of ~8900 RFU.

### Example 9: Apta-SMART detection of synthetic RNA target of SARS-CoV-2 S 'spike' RNA region

We designed a SARS-CoV-2 first (**SEQ ID NO: 26**) and a second probe containing a Mango aptamer (**SEQ ID NO: 27**) for the detection of a reverse complement (RC) of a 189-nt RNA region (**SEQ ID NO: 29**) of SARS-CoV-2 *S 'spike'* RNA (**SEQ ID NO: 32**). We tested the SMART reaction using a synthetic RNA consisting of the 30-nucleotide region of interaction (**SEQ ID NO: 28**) with SARS-CoV-2 first and second probes. The SMART reaction started with a denaturing step at 65°C for 5 minutes followed by a pre-incubation step at 41°C for 60 minutes of a reaction mixture with both first and second probes 840 and 700 fmols, respectively) and 17.5 nM of synthetic RNA target in 1X transcription buffer (Promega) plus 1 mM spermidine (Sigma), up to 7 µL. After that, 3 µL of dNTPs/ NTPs and enzyme mix (containing 5 µM dNTPs, 2 mM NTPs, 2 U Bst DNA pol and 25 U T7 RNA pol) were added and incubated for another 60 minutes in the same buffer. Finally, 1 µL of 4.8 µM T01-biotin fluorogen (BioCat, Cat#: G955-ABM) was added (480 nM final concentration) right before fluorescence was read at 510 nm (excitation) and 535 nm (emission) wavelengths using a BioTek plate reader (Synergy H1 Hybrid Multi-Mode Reader) in black bottom 384-well plates (Nunc).

As seen in **Figure 14****,** Mango-SMART detection of SARS-CoV-2 *S 'spike'* RNA using 17.5 nM was optimal, despite showing a lower fold-change (~4.1) than previous examples. No sRNA control was used as background fluorescence showing a bit higher average RFU than previous examples (~16400).

### Example 10: Mango-SMART detection of synthetic RNA target of Influenza A & B virus

We designed specific first (**SEQ ID NO: 45** and **46**) and second probes containing a Mango aptamer (**SEQ ID NO: 47** and **48**) for the detection of a highly specific RNA region of Influenza A and Influenza B viruses, respectively (**SEQ ID NO: 49** and **50**). For Influenza A, the region corresponds to the segment 2 of polymerase PB1 (*PB1*) gene (**SEQ ID NO: 51**) and for influenza B, it corresponds to the segment 3 of polymerase PA (*PA*) gene (**SEQ ID NO: 52**). We tested the SMART reaction using 30-nt long synthetic RNAs corresponding to 15-nt hybridization nucleotides per probe (first and second) for both Influenza A and B (**SEQ ID NO: 49** and **50**, respectively). The SMART reaction started with a denaturing step at 65°C for 5 minutes followed by a pre-incubation step at 41°C for 60 minutes of a reaction mixture with both first and second probes 840 and 700 fmols, respectively) and 17.5 nM of synthetic RNA target in 1X transcription buffer (Promega) plus 1 mM spermidine (Sigma), up to 7 µL. After that, 3 µL of dNTPs/ NTPs and enzyme mix (containing 5 µM dNTPs, 2 mM NTPs, 2 U Bst DNA pol and 25 U T7 RNA pol) were added and incubated for another 60 minutes in the same buffer. Finally, 1 µL of 4.8 µM T01-biotin fluorogen (BioCat, Cat#: G955-ABM) was added (480 nM final concentration) right before fluorescence was read at 510 nm (excitation) and 535 nm (emission) wavelengths using a BioTek plate reader (Synergy H1 Hybrid Multi-Mode Reader) in black bottom 384-well plates (Nunc).

As seen in **Figure 15**, Mango-SMART detection of Influenza A RNA region (corresponding to *PB1* gene) and Influenza B RNA region (corresponding to *PA* gene) using 17.5 nM was optimal, with RFUs over 30000 units and fold-changes of ~5.0 and ~2.4 for Influenza A and B, respectively. No sRNA control (background fluorescence) reads were ~6700 RFUs for Influenza A and ~13200 RFUs for Influenza B.

### Example 11: Apta-SMART detection of E.coli 16S rRNA after NASBA amplification

### Step 1: NASBA

We used the 5' primer (**SEQ ID NO: 4**) and the 3' primer, containing a T7 promoter (**SEQ ID NO: 5**), previously reported by Kao et al. (Anal Letters, 2010) that isothermally amplify a 191 nucleotide region (**SEQ ID NO: 9**) of *E.coli* 16S rRNA (**SEQ ID NO: 6**). The NASBA reaction consisted of a mixture containing 2 µL of previously isolated *E.coli* RNA and 1 µM of NASBA primers in 15 µL final volume in NASBA buffer (40 mM Tris-HCl, pH 8.0, 13.2 mM MgCl₂, 75 mM KCI, 10 mM DTT, 1 mM dNTPs, 2 mM ATP, 2 mM UTP, 2 mM CTP, 1.5 mM GTP, 0.5 mM ITP and 15% uL DMSO -corresponding to 3 µL of the reaction mix-) in a initial denaturation step of 2 minutes at 95°C and 2 minutes at 37°C. Then, 5 µL of enzyme mix (6.8 U AMV-RT, 0.08 U RNAse H, 32 U T7 RNA polymerase and 120 µg/mL BSA in water) was added and incubated at 37°C for 90 minutes. The resulting amplified region (**SEQ ID NO: 9**) is a reverse complement (RC) of the *E.coli* 16S rRNA fragment used as template for Mango-SMART reaction (Step 2).

### Step 1.1. ExoCIP treatment (optional)

In the assays where ExoCIP is used (panels **B** and **D** in **Figure 16** and scheme in **Figure 3**), right after NASBA amplification, 0.5 µL of ExoCIP solution A and 0.5 µL of ExoCIP solution B (New England Biolabs) is added to 2.5 uL of NASBA product and incubated at 37°C for 4 minutes. To inactivate the enzyme, the mixture is heated at 80°C for 1 minute and apta-SMART is performed right after.

### Step 2: Mango-SMART.

Using the *E.coli* 16S first (**SEQ ID NO: 7**) and second probe containing a Mango aptamer (**SEQ ID NO: 8**) from previous example for the detection of a RC RNA sequence (product of NASBA reaction) (**SEQ ID NO: 9**) of *E.coli* 16S rRNA (**SEQ ID NO: 6**), we ran the Mango-SMART reaction as detailed: denaturing at 65°C for 5 minutes followed by a pre-incubation step at 41°C for 60 minutes of a reaction mixture with both first and second probes (840 and 700 fmols, respectively) with 1 µL of NASBA reaction or 1.4 µL of ExoCIP-treated NASBA product in 1X transcription buffer (Promega) plus 1 mM spermidine (Sigma) up to 7 µL volume. After that, 3 µL of dNTPs/ NTPs and enzyme mix (containing 5 µM dNTPs, 2 mM NTPs, 2 U Bst DNA pol and 25 U T7 RNA pol) were added to obtain a final volume of 10 µL and incubated for another 60 minutes in the same buffer (shown as '+dNTPs' in **Figure 16**). Moreover, the same reaction is performed without dNTPs (shown as '-dNTPs', **Figure 16**), thus, adding just the enzyme mix (2 mM NTPs, 2 U Bst DNA pol and 25 U T7 RNA pol) to a final volume of 10 µL. Finally, 1 µL of 4.8 µM T01-biotin fluorogen (BioCat, Cat#: G955-ABM) was added (480 nM final concentration) right before fluorescence was read at 510 nm (excitation) and 535 nm (emission) wavelengths using a BioTek plate reader (Synergy H1 Hybrid Multi-Mode Reader) in black bottom 384-well plates (Nunc).

**Figure 16** shows the results of Mango-SMART detection of *E.coli* 16S rRNA detection in the different conditions tested. As shown in panel A (RFU) and C (fold-change), all cultured *E.coli* samples without ExoCIP treatment after NASBA amplification were detected over NASBA no RNA control with fluorescence fold-changes >2.0. The differences between adding or not adding dNTPs in the SMART detection step were not significant. Despite some variability between the three (-dNTPs) or five (+dNTPs) experiments performed, the detection was optimal even for the lowest concentration of bacteria tested (10² CFU/mL). Panels B (RFU) and D (fold-change) show the same samples tested with ExoCIP clean-up step after NASBA amplification showing a bit of an improvement in detection: lower variability giving tighter fold-changes for both 10⁶ (from 3.9 to 4.2) and 10² CFU/mL (~2.2) samples tested. As for non ExoCIP-treated samples, the differences between minus or plus dNTPs in the SMART detection did not make a difference on the sensibility of detection.

### Example 12: Apta-SMART detection of E.coli 23S rRNA after NASBA amplification

### Step 1: NASBA.

We designed a 5' primer (**SEQ ID NO: 53**) and a 3' primer containing a T7 promoter (**SEQ ID NO: 54**) that isothermally amplifies a 256-nucleotide region (**SEQ ID NO: 37**) of *E.coli* 23S rRNA (**SEQ ID NO: 38**). The NASBA reaction consisted of mixture containing 2 µL of previously isolated *E.coli* RNA and 1 µM of NASBA primers in 15 µL final volume in NASBA buffer (40 mM Tris-HCl, pH 8.0, 13.2 mM MgCl₂, 75 mM KCI, 10 mM DTT, 1 mM dNTPs, 2 mM ATP, 2 mM UTP, 2 mM CTP, 1.5 mM GTP, 0.5 mM ITP and 15% uL DMSO -corresponding to 3 µL of the reaction mix-) in a initial denaturation step of 2 minutes at 95°C and 2 minutes at 37°C. Then, 5 µL of enzyme mix (6.8 U AMV-RT, 0.08 U RNAse H, 32 U T7 RNA polymerase and 120 µg/mL BSA in water) was added and incubated at 37°C for 90 minutes. The resulting amplified RNA region (**SEQ ID NO: 37**) is a reverse complement (RC) of the *E.coli* 23S rRNA fragment used as template for Mango-SMART reaction (Step 2).

### Step 2: Mango-SMART.

Using the *E.coli* 23S rRNA first (**SEQ ID NO: 35**) and second probe containing a Mango aptamer (**SEQ ID NO: 36**) from previous example for the detection of a RC RNA sequence (product of NASBA reaction) (**SEQ ID NO: 37**) of *E.coli* 23S rRNA (**SEQ ID NO: 38**), we ran the Mango-SMART reaction as detailed: denaturing at 65°C for 5 minutes followed by a pre-incubation step at 41°C for 60 minutes of a reaction mixture with both first and second probes (840 and 700 fmols, respectively) with 1 µL of NASBA reaction in 1X transcription buffer (Promega) plus 1 mM spermidine (Sigma) up to 7 µL volume. After that, 3 µL of dNTPs/ NTPs and enzyme mix (containing 5 µM dNTPs, 2 mM NTPs, 2 U Bst DNA pol and 25 U T7 RNA pol) were added to obtain a final volume of 10 µL and incubated for another 60 minutes in the same buffer. Finally, 1 µL of 4.8 µM T01-biotin fluorogen (BioCat, Cat#: G955-ABM) was added (480 nM final concentration) right before fluorescence was read at 510 nm (excitation) and 535 nm (emission) wavelengths using a BioTek plate reader (Synergy H1 Hybrid Multi-Mode Reader) in black bottom 384-well plates (Nunc).

As we can see in **Figure 17**, the detection of an *E.coli* 23S rRNA region of 10⁴ and 10⁶ CFU/mL *E.coli* was optimal for both samples cultured, showing a fold-change over NASBA no RNA control of 3.60 (31150 RFU) and 6.70 (64360 RFU), respectively. Data was normalized over a NASBA no RNA control with an average RFU of 9825.

### Example 13: NASBA and apta-SMART detection of mecA S. aureus.

### Step 1: NASBA.

We designed a 5' primer (**SEQ ID NO: 55**) and a 3' primer containing a T7 promoter (**SEQ ID NO: 56**) that isothermally amplify a 98-nucleotide region (**SEQ ID NO: 42**) of *mecA* gene of *S*. *aureus* (**SEQ ID NO: 43**). NASBA reaction consisted of mixture containing 2 µL of previously isolated *mecA S. aureus* RNA and 1 µM of NASBA primers in 15 µL final volume in NASBA buffer (40 mM Tris-HCl, pH 8.0, 13.2 mM MgCl₂, 75 mM KCI, 10 mM DTT, 1 mM dNTPs, 2 mM ATP, 2 mM UTP, 2 mM CTP, 1.5 mM GTP, 0.5 mM ITP and 15% uL DMSO -corresponding to 3 µL of the reaction mix-) in a initial denaturation step of 2 minutes at 95°C and 2 minutes at 37°C. Then, 5 µL of enzyme mix (6.8 U AMV-RT, 0.08 U RNAse H, 32 U T7 RNA polymerase and 120 µg/mL BSA in water) was added and incubated at 37°C for 90 minutes. The resulting amplified RNA region (**SEQ ID NO: 42**) is a reverse complement (RC) of the *mecA S. aureus* RNA fragment used as template for Mango-SMART reaction (Step 2).

### Step 2: Mango-SMART.

Using the *mecA* first (**SEQ ID NO: 40**) *mecA* second probe containing a Mango aptamer (**SEQ ID NO: 41**) for the detection of a reverse complement (RC) of an RNA region (**SEQ ID NO: 42**) of *mecA S. aureus* (**SEQ ID NO: 43**), we ran the Mango-SMART reaction as detailed: denaturing at 65°C for 5 minutes followed by a pre-incubation step at 41°C for 60 minutes of a reaction mixture with both first and second probes (420 and 350 fmols, respectively) with 1 µL of NASBA reaction (instead of synthetic RNA) in 1X transcription buffer (Promega) plus 1 mM spermidine (Sigma). After that, 3 µL of dNTPs/ NTPs and enzyme mix (containing 5 µM dNTPs, 2 mM NTPs, 2 U Bst DNA pol and 25 U T7 RNA pol) were added to a final volume of 10 µL and incubated for another 60 minutes in the same buffer. Finally, 1 µL of 4.8 µM TO1-biotin fluorogen (BioCat, Cat#: G955-ABM) was added (480 nM final concentration) right before fluorescence was read at 510 nm (excitation) and 535 nm (emission) wavelengths using a BioTek plate reader (Synergy H1 Hybrid Multi-Mode Reader) in black bottom 384-well plates (Nunc).

**Figure 18** shows the optimal Mango-SMART detection of a region of *mecA* gene of a culture of 10⁶ CFU/mL *S*. *aureus.* The fold-change of the NASBA amplified sample over no RNA NASBA control was 5.17 (64930 RFU over 13087 RFU) after six repeated and independent experiments.

### Example 14: NASBA and apta-SMART detection of SARS-CoV-2 S 'spike' RNA

### Step 1: NASBA.

We designed a 5' primer (**SEQ ID NO: 33**) and a 3' primer containing a T7 promoter (**SEQ ID NO: 34**) that isothermally amplify a 189 nucleotide region (**SEQ ID NO: 29**) of *S 'spike'* RNA of SARS-CoV-2 (**SEQ ID NO: 32**). The NASBA reaction consisted of a reaction containing 2 uL of previously isolated SARS-CoV-2 RNA (with known Ct values as assessed by qPCR) and 500 nM of NASBA primers in 15 µL final volume in NASBA buffer (40 mM Tris-HCl, pH 8.0, 13.2 mM MgCl₂, 75 mM KCI, 10 mM DTT, 1 mM dNTPs, 2 mM ATP, 2 mM UTP, 2 mM CTP, 1.5 mM GTP, 0.5 mM ITP and 15% uL DMSO -corresponding to 3 µL of the reaction mix-) in a initial denaturation step of 2 minutes at 95°C and 2 minutes at 37°C. Then, 5 µL of enzyme mix (6.8 U AMV-RT, 0.08 U RNAse H, 32 U T7 RNA polymerase and 120 µg/mL BSA in water) was added and incubated at 37°C for 90 minutes. The resulting amplified region (**SEQ ID NO: 29**) is a reverse complement (RC) of a *S 'spike'* RNA fragment used as template for Mango-SMART reaction (*Step 2*).

### Step 2: Mango-SMART.

Using the SARS-CoV-2 first (**SEQ ID NO: 26**) and SARS-CoV-2 second probe containing a Mango aptamer (**SEQ ID NO: 27**) from previous example for the detection of a RC RNA sequence (result of NASBA reaction) (**SEQ ID NO: 29**) of SARS-CoV-2 *S 'spike'* RNA (**SEQ ID NO: 32**), we ran the Mango-SMART reaction as detailed: denaturing at 65°C for 5 minutes followed by a pre-incubation step at 41°C for 60 minutes of a reaction mixture with both first and second probes (840 and 700 fmols, respectively) with 1 µL of NASBA reaction (instead of synthetic RNA) in 1X transcription buffer (Promega) plus 1 mM spermidine (Sigma). After that, 3 µL of dNTPs/ NTPs and enzyme mix (containing 5 µM dNTPs, 2 mM NTPs, 2 U Bst DNA pol and 25 U T7 RNA pol) were added to a final volume of 10 µL and incubated for another 60 minutes in the same buffer. Finally, 1 µL of 4.8 µM T01-biotin fluorogen (BioCat, Cat#: G955-ABM) was added (480 nM final concentration) right before fluorescence was read at 510 nm (excitation) and 535 nm (emission) wavelengths using a BioTek plate reader (Synergy H1 Hybrid Multi-Mode Reader) in black bottom 384-well plates (Nunc).

As we can see in **Figure 19****,** Mango-SMART detection of SARS-CoV-2 *S 'spike'* RNA was optimal for the three samples tested, with higher fold-change values for NASBA samples #3 and #7 (3.44 and 3.26 *versus* 2.04 for sample #1 of an average of six independent experiments). The Mango-TO1 RFU background of no RNA or NASBA no RNA samples was between 15375 and 18000 RFUs in this example.

### Example 15: Broccoli-SMART detection of synthetic RNA target of an E.coli 16S rRNA.

Based on the *E.coli* 16S rRNA first (**SEQ ID NO: 7**) and second probes containing a Mango aptamer (**SEQ ID NO: 8**) used in the Examples 2 and 6, we designed a modified second probe for the detection of the same reverse complement (RC) region of *E.coli* 16S *rRNA* (Kao et al. Anal Letters, 2010) but using a Broccoli RNA aptamer (**SEQ ID NO: 57**, Filonov et al. JACS, 2014) instead of Mango-III (A10U) (**SEQ ID NO: 3**, Trachman et al. Nat Chem Biol, 2019).

Our test consisted in the SMART reaction using a synthetic RNA of the 75-nucleotide region of interaction (**SEQ ID NO: 13**) with *E.coli* 16S rRNA first (**SEQ ID NO: 7**) and Broccoli-second probes (**SEQ ID NO: 58**). The SMART reaction started with a denaturing step at 65°C for 5 minutes followed by a pre-incubation step at 41°C for 60 minutes of a reaction mixture with both first and second probes (840 and 700 fmols, respectively) and synthetic RNA target (17.5 nM final concentration) in 1X transcription buffer (Promega) plus 1 mM spermidine (Sigma), up to 7 µL. After that, 3 µL of dNTPs/ NTPs and enzyme mix (containing 5 µM dNTPs, 2 mM NTPs, 2 U Bst DNA pol and 25 U T7 RNA pol) were added and incubated for another 60 minutes in the same buffer. Finally, 1 µL of 10 µM DFHBI or DFHBI-1T fluorogen (MedChemExpress, #HY-110250 and #HY-110251, respectively) was added (1 µM final concentration) right before fluorescence was read at 472 nm (excitation) and 507 nm (emission) wavelengths using a BioTek plate reader (Synergy H1 Hybrid Multi-Mode Reader) in black bottom 384-well plates (Nunc).

**Figure 20** shows the comparison of Mango *versus* Broccoli aptamers detection of a 75-nt fragment of an *E.coli* 16S rRNA synthetic RNA using T01-biotin ('TO1' in the graph) fluorogen for Mango and both DFHBI and DFHBI-1T fluorogen substrates for Broccoli. Results show a clear advantage of Mango-TO1 over Broccoli (either plus DFHBI or DFHBI-1T) for the detection of this *E.coli* RNA fragment: whereas Mango-TO1 shows a fold-change of sRNA over no RNA of ~3.0, neither Broccoli-DFHBI nor Broccoli-DFHBI-1T reached 2.2-fold.

### Example 16: SMART-Broccoli detection of synthetic RNA target of SARS-CoV-2 S 'spike' RNA.

Based on the SARS-CoV-2 first (**SEQ ID NO: 26**) and second probe containing a Mango aptamer (**SEQ ID NO: 27**), we designed a modified second probe (**SEQ ID NO: 59**) for the detection of the same reverse complement (RC) of a region (**SEQ ID NO: 29**) of SARS-CoV-2 *S 'spike'* RNA (**SEQ ID NO: 32**) using a Broccoli RNA aptamer (**SEQ ID NO: 57**) instead of Mango-III (A10U) (**SEQ ID NO: 3**).

We tested the SMART reaction using a synthetic RNA consisting of the 30-nucleotide region of interaction (**SEQ ID NO: 28**) with SARS-CoV-2 first (**SEQ ID NO: 26**) and Broccoli-second probes (**SEQ ID NO: 59**). The SMART reaction started with a denaturing step at 65°C for 5 minutes followed by a pre-incubation step at 41°C for 60 minutes of a reaction mixture with both first and second probes (840 and 700 fmols, respectively) and synthetic RNA target (17.5 nM final concentration) in 1X transcription buffer (Promega) plus 1 mM spermidine (Sigma), up to 7 µL. After that, 3 µL of dNTPs/ NTPs and enzyme mix (containing 5 µM dNTPs, 2 mM NTPs, 2 U Bst DNA pol and 25 U T7 RNA pol) were added and incubated for another 60 minutes in the same buffer. Finally, 1 µL of 10 µM DFHBI or DFHBI-1T fluorogen (MedChemExpress, #HY-110250 and #HY-110251, respectively) was added (1 µM final concentration) right before fluorescence was read at 472 nm (excitation) and 507 nm (emission) wavelengths using a BioTek plate reader (Synergy H1 Hybrid Multi-Mode Reader) in black bottom 384-well plates (Nunc).

As we can see in **Figure 21****,** a comparison of Mango *versus* Broccoli aptamers detection was also performed for a 30-nucleotide synthetic RNA fragment of *S 'spike'* RNA of SARS-CoV-2, as shown for Example 14, using T01-biotin for Mango and DFHBI and DFHBI-1T for Broccoli. Results again show a clear advantage of Mango-TO1 over Broccoli (either using DFHBI or DFHBI-1T) for the detection of SARS-CoV-2 RNA. Mango-TO1 shows a fold-change of sRNA over no RNA of ~5.0 whereas Broccoli-DFHBI and Broccoli-DFHBI-1T only reach 2.5- and 3.3-fold, respectively. Nevertheless, these are optimal fold-changes (>2.0) that confirm Broccoli and its fluorogen substrates as alternative aptamers to be used instead of Mango (if necessary) for apta-SMART detection.

### REFERENCES

- Filonov et al. Broccoli: Rapid Selection of an RNA Mimic of Green Fluorescent Protein by Fluorescence-Based Selection and Directed Evolution. Journal of the American Chemical Society, 136 (46), 16299-16308 (2014).
- Kao et al. Detection of Escherichia coli Using Nucleic Acid Sequence-Based Amplification and Oligonucleotide Probes for 16S Ribosomal RNA. Analytical Letters, 43, 1756-1769 (2010).
- Trachman et al. Structure and functional reselection of the Mango-III fluorogenic RNA aptamer. Nature Chemical Biology, 15, 472-479 (2019).
- Wharam et al. Specific detection of DNA and RNA targets using a novel isothermal nucleic acid amplification assay based on the formation of a three-way junction structure. Nucleic Acids Research, 29 (11), e54 (2001).
- Zhao et al. Rapid Real-Time Nucleic Acid Sequence-Based Amplification-Molecular Beacon Platform to Detect Fungal and Bacterial Bloodstream Infections. Journal of Clinical Microbiology, 47 (7), 2067-2078 (2009).

## Claims

1. A method for detecting the presence of a target nucleic acid in a sample, the method comprising the steps of:
a) adding a first and a second nucleic acid probes to a sample comprising the target nucleic acid so as to form a three-way junction structure by hybridization between the target nucleic acid molecule, the first and the second nucleic acid probe, wherein:
i) the first probe comprises a foot region located at the 5' region of the probe which is complementary to a first portion of the target nucleic acid and hybridizes thereto, and an arm region located at the 3' region of the probe;
ii) the second probe comprises:
(1) a foot region located at the 3' region of the probe which is complementary and hybridizes thereto to a second portion of the target nucleic acid, and
(2) an arm region located at the 5' region of the probe, comprising, in the 5' to 3' direction:
- a full length reverse complement sequence of at least one fluorogenic aptamer,
- a full length reverse complement sequence of an DNA-directed RNA polymerase promoter, wherein the reverse complement sequence of the at least one fluorogenic aptamer is operably linked to said DNA-directed RNA polymerase promoter,
- a region of between 5-9 nucleotides that is complementary and hybridizes thereto to the arm region of the first probe, optionally wherein said region of between 5-9 nucleotides is fully or partially included in the full length reverse complement sequence of the DNA-directed RNA polymerase promoter, and
iii) the target nucleic acid comprises a first portion located at the 3' region of the target nucleic acid which is complementary to the foot region of the first probe, and a second portion located at the 5' region of the target nucleic acid which is complementary to the foot region of the second probe, wherein the first portion and the second portion are adjacent or substantially adjacent, preferably wherein the first portion and the second portions are separated by 0 to 6 nucleotides, and wherein the three-way junction structure is formed between the first probe, the second probe and the target nucleic acid when the target nucleic acid is present in the sample, and
b) adding a DNA-directed DNA polymerase which extends the arm of the first probe until the end of the arm of the second probe, creating a double-stranded structure comprising a functional RNA polymerase promoter and at least one fluorogenic aptamer, wherein one strand of the double-stranded structure is provided by the extended arm of the first probe, and the other strand of the double-stranded structure is provided by the arm of second probe;
c) adding a DNA-directed RNA polymerase which recognises the double-stranded promoter formed in step b), so as to cause the *de novo* synthesis of a single-stranded nucleic acid comprising the at least one fluorogenic aptamer; and
d) adding at least one fluorophore ligand of said at least one fluorogenic aptamer thereby directly detecting the *de novo* synthezised nucleic acid comprising the at least one fluorogenic aptamer, wherein the detection of said nucleic acid comprising the at least one fluorogenic aptamer indicates the presence of the target nucleic acid in the sample,
wherein, optionally, the method further comprises an amplification step previous to step a), wherein the target nucleic acid is amplified.

2. The method according to claim 1, wherein the double-stranded RNA promoter formed in step b) is a T7 RNA polymerase promoter, and wherein the DNA-directed RNA polymerase added in step c) is a T7 RNA polymerase.

3. The method according to any of claims 1 or 2, wherein the DNA-directed DNA polymerase added in step b) is the *Bacillus stearothermophilus* DNA Polymerase I.

4. The method according to any of claims 1 to 3, wherein the foot region of the first probe and/or of the second probe is at least 15 nucleotides in length.

5. The method according to any of claims 1 to 4, wherein the at least one fluorescent aptamer is a Mango aptamer or a Broccoli aptamer.

6. The method according to claim 5, wherein the at least one fluorophore ligand added in step d) is TO1 biotin fluorogen if the at least one fluorescent aptamer comprised in the arm of the second probe is a Mango aptamer or wherein the at least one fluorophore ligand added in step d) is DFHBI or DFHBI-1T fluorogen if the at least one fluorogenic aptamer comprised in the arm of the second probe is a Broccoli aptamer.

7. The method according to any of claims 1 to 6, wherein the method further comprises an amplification step previous to step a) comprising amplifying the target nucleic acid to provide a plurality of molecules identical to the target nucleic acid or the reverse complement thereof, wherein said plurality of molecules represent the target nucleic acid in the subsequent steps a) to d), and wherein said amplification step is optionally followed by a step of degrading residual amplification primers and dephosphorylating excess dNTPs after amplification.

8. The method according to claim 7, wherein the amplification step is carried out by nucleic acid sequence-based amplification (NASBA).

9. The method according to any of claims 1 to 8, wherein the target nucleic acid is an RNA molecule, preferably derived from the genome of an infectious agent such as a bacterium or a virus.

10. A **computer-implemented method** for designing at least a pair of probes suitable for implementing the method as defined in any of claims 1 to 9, the method comprising the steps of:
i) reading a target nucleic acid,
ii) obtaining or generating the sequence of at least a pair of probes, wherein each of the probes comprise a foot region and an arm region, wherein:
the foot region of the first probe is located at the 5' region of said probe and is complementary to a first portion of the target nucleic acid and hybridizes thereto, and the arm region of the first probe is located at the 3' region of said probe and is non-complementary to the target nucleic acid, wherein
the foot region of the second probe is located at the 3' region of said probe and is complementary and hybridizes thereto to a second portion of the target nucleic acid, and the arm region of the second probe is located at the 5' region of said probe and comprises, in the 5' to 3' direction:
- a full length reverse complement sequence of at least one fluorogenic aptamer,
- a full length reverse complement sequence of an DNA-directed RNA polymerase promoter, wherein the reverse complement sequence of the at least one fluorogenic aptamer is operably linked to said DNA-directed RNA polymerase promoter,
- a region of between 5-9 nucleotides that is complementary and hybridizes thereto to the arm region of the first probe, optionally wherein said region of between 5-9 nucleotides is fully or partially included in the full length reverse complement sequence of the DNA-directed RNA polymerase promoter,
wherein the first portion and the second portion of the target nucleic acid are separated by 0 to 6 nucleotides,
wherein the first and the second probes are capable of forming a three-way junction structure by hybridization with the target nucleic acid molecule, and
iii) optionally, providing the sequences obtained or generated in step ii) as output.

11. The computer implementer method of claim 10, wherein step ii) comprises the steps of:
a) obtaining the foot region of the first probe by selecting a first portion of nucleotides comprised in the target nucleic acid and providing its complementary sequence,
b) obtaining the foot region of the second probe by selecting a second portion of nucleotides comprised in the target nucleic acid and providing its complementary sequence, wherein the first portion and the second portion are separated by 0 to 6 nucleotides,
c) obtaining the arm region of the second probe by selecting a nucleotide sequence comprising the full length sequence of an DNA-directed RNA polymerase promoter and the full length reverse complement sequence of at least one fluorogenic aptamer, wherein the reverse complement sequence of at least one fluorogenic aptamer is operably linked to said DNA-directed RNA polymerase promoter,
d) obtaining the arm region of the first probe by selecting between 5-9 nucleotides that are complementary to a region located at the 3' region of the arm region of the second probe obtained in step c), and
e) optionally, synthesizing the designed first and a second probe.

12. A **kit of parts** for detecting the presence of a target nucleic acid in a sample, comprising the following elements:
a) at least one DNA-directed DNA polymerase, preferably Bst polymerase,
b) at least one DNA-directed RNA polymerase, preferably T7 RNA polymerase,
c) at least a suitable fluorophore ligand of at least one fluorogenic aptamer,
d) spermidine,
e) deoxynucleoside triphosphates,
f) suitable buffers,
g) optionally, a retrotranscriptase, an RNAse, at least an exonuclease and a phosphatase,
wherein elements a) to g) are comprised in different containers or grouped in one or more containers.

13. A **system** for detecting the presence of a target nucleic acid in a sample comprising a first probe and a second probe, wherein the first probe comprises a foot region located at the 5' region of the probe which is complementary to a first portion of the target nucleic acid and hybridizes thereto, and an arm region located at the 3' region of the probe; and wherein the second probe comprises:
(1) a foot region located at the 3' region of the probe which is complementary and hybridizes thereto to a second portion of the target nucleic acid, and
(2) an arm region comprising, in the 5' to 3' direction:
i. a full length reverse complement sequence of at least one fluorogenic aptamer,
ii. a full length reverse complement sequence of an DNA-directed RNA polymerase promoter, wherein the reverse complement sequence of the at least one fluorogenic aptamer is operably linked to said RNA polymerase promoter,
iii. a region comprising between 5-9 nucleotides that is complementary and hybridizes thereto to the arm region of the first probe, wherein said region between 5-9 nucleotides may be a region included in the full length reverse complement sequence of an DNA-directed RNA polymerase promoter.
